(19) Europäisches Patentamt
European Patent Office
Office européen des brevets

(11) EP 2 934 508 B1

(12) **EUROPÄISCHE PATENTSCHRIFT**

(45) Veröffentlichungstag und Bekanntmachung des Hinweises auf die Patenterteilung:
**06.12.2017 Patentblatt 2017/49**

(21) Anmeldenummer: **13814901.8**

(22) Anmeldetag: **19.12.2013**

(51) Int Cl.:
***A61K 31/295*** (2006.01) ***A61K 31/555*** (2006.01)
***A61P 7/06*** (2006.01) ***A61K 45/06*** (2006.01)

(86) Internationale Anmeldenummer:
**PCT/EP2013/077383**

(87) Internationale Veröffentlichungsnummer:
**WO 2014/096193 (26.06.2014 Gazette 2014/26)**

(54) **FE(III)-KOMPLEXVERBINDUNGEN ZUR BEHANDLUNG UND PROPHYLAXE VON EISENMANGELERSCHEINUNGEN UND EISENMANGELANÄMIEN**

FE(III)-2-OXO-BUTANEDIAMIDE COMPLEXES FOR TREATMENT AND PROPHYLAXIS OF IRON DEFICIENCIES AND ANEMIA

LIAISONS DE COMPLEXES DE FE(III)-2-OXO-BUTANEDIAMIDE POUR LE TRAITEMENT ET LA PROPHYLAXIE DES CARENCES EN FER ET DES ANÉMIES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.12.2012 EP 12199075**

(43) Veröffentlichungstag der Anmeldung:
**28.10.2015 Patentblatt 2015/44**

(73) Patentinhaber: **Vifor (International) AG**
**9001 St. Gallen (CH)**

(72) Erfinder:
- **BARK, Thomas**
  **CH-8050 Zürich (CH)**
- **BUHR, Wilm**
  **78465 Konstanz (DE)**
- **BURCKHARDT, Susanna**
  **CH-8049 Zürich (CH)**
- **BURGERT, Michael**
  **D-88045 Friedrichshafen (DE)**
- **CANCLINI, Camillo**
  **CH-9000 St. Gallen (CH)**
- **DÜRRENBERGER, Franz**
  **CH-4143 Dornach (CH)**
- **FUNK, Felix**
  **CH-8404 Winterthur (CH)**
- **GEISSER, Peter Otto**
  **CH-9014 St. Gallen (CH)**
- **KALOGERAKIS, Aris**
  **CH-8400 Winterthur (CH)**
- **MAYER, Simona**
  **CH-9055 Bühler (CH)**
- **PHILIPP, Erik**
  **CH-9320 Arbon (CH)**
- **REIM, Stefan**
  **CH-8404 Stadel Winterthur (CH)**
- **SIEBER, Diana**
  **CH-9030 Abtwil (CH)**
- **SCHMITT, Jörg**
  **78343 Gaienhofen (DE)**
- **SCHWARZ, Katrin**
  **CH-9000 St. Gallen (CH)**

(74) Vertreter: **Gille Hrabal**
**Brucknerstrasse 20**
**40593 Düsseldorf (DE)**

(56) Entgegenhaltungen:
**WO-A1-2011/117225 WO-A1-2012/163938**

- **R. THOMAS-MAMERT: "Sur l'aminobutènediamide et la butanonediamide", BULLETIN DE LA SOCIETE CHIMIQUE DE PARIS, 1894, Seiten 96-98, XP002719207,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **BANVILLE J ET AL: "(Z)-2,2-Dimethyl-5-carboxymethylene-1,3-d ioxolan-4-one: a new synthon for the synthesis of alpha,gamma-diketoacid derivatives", TETRAHEDRON LETTERS, Bd. 51, Nr. 24, 16. Juni 2010 (2010-06-16) , Seiten 3170-3173, XP027044281, PERGAMON, GB ISSN: 0040-4039 [gefunden am 2010-04-21] in der Anmeldung erwähnt**


Bemerkungen:

Die Akte enthält technische Angaben, die nach dem Eingang der Anmeldung eingereicht wurden und die nicht in dieser Patentschrift enthalten sind.

## Beschreibung

### Einleitung:

Die Erfindung betrifft Eisen(III)-2-oxo-butandiamid-Komplexverbindungen und deren sie umfassende pharmazeutischen Zusammensetzungen zur Verwendung als Arzneimittel, insbesondere zur Behandlung und/oder zur Prophylaxe von Eisenmangelerscheinungen und Eisenmangelanämien.

### Hintergrund:

Eisen ist ein essentielles Spurenelement für fast alle Lebewesen und ist dabei insbesondere für das Wachstum und die Blutbildung relevant. Die Balance des Eisenhaushaltes wird dabei primär auf der Ebene der Eisenwiedergewinnung aus Hämoglobin von alternden Erythrozyten und der duodenalen Absorption von nahrungsgebundenem Eisen reguliert. Das freigesetzte Eisen wird über den Darm insbesondere durch spezifische Transportsysteme (DMT-1, Ferroportin, Transferrin, Transferrin Rezeptoren) aufgenommen, in den Blutkreislauf transportiert und hierüber in die entsprechenden Gewebe und Organe weitergeleitet.
Das Element Eisen ist im menschlichen Körper unter anderem für den Sauerstofftransport, die Sauerstoffaufnahme, Zellfunktionen, wie den mitochondrialen Elektronentransport und letztlich für den gesamten Energiestoffwechsel von großer Bedeutung.
Der Körper eines Menschen enthält im Durchschnitt 4 bis 5 g Eisen, wobei dies in Enzymen, in Hämoglobin und Myoglobin, sowie als Depot- oder Reserve-Eisen in Form von Ferritin und Hämosiderin vorliegt.
Etwa die Hälfte dieses Eisens ca. 2 g liegt als Häm Eisen gebunden im Hämoglobin der roten Blutkörperchen vor. Da Erythrozyten nur eine begrenzte Lebensdauer aufweisen (75-150 Tage), müssen ständig neue gebildet und alte eliminiert werden (über 2 Mio. Erythrozyten werden pro Sekunde neu gebildet). Diese hohe Neubildungskapazität wird durch Makrophagen erreicht, indem diese die alternden Erythrozyten phagozytotisch aufnehmen, lysieren und so das enthaltene Eisen für den Eisenhaushalt rezyklieren können. Somit wird die täglich für die Erythropoese benötige Eisenmenge von ca. 25 mg grösstenteils bereit gestellt.

Der tägliche Eisenbedarf eines erwachsenen Menschen beträgt zwischen 0,5 und 1,5 mg pro Tag, bei Kleinkindern sowie bei Frauen in der Schwangerschaft liegt der Eisenbedarf bei 2 bis 5 mg pro Tag. Die täglichen Eisenverluste, z.B. durch Abschilferung von Haut- und Epithelzellen ist gering, erhöhte Eisenverluste treten beispielsweise bei der Menstruationsblutung bei Frauen auf. Generell können Blutverluste den Eisenhaushalt beträchtlich verringern, da pro 2 ml Blut etwa 1 mg Eisen verloren gehen. Der normale tägliche Eisenverlust von ca. 1 mg wird üblicherweise bei einem erwachsenen, gesunden Menschen über die tägliche Nahrungsaufnahme wieder ersetzt. Der Eisenhaushalt wird über Resorption reguliert, wobei die Resorptionsquote des in der Nahrung vorhandenen Eisens zwischen 6 und 12 % beträgt, bei Eisenmangel beträgt die Resorptionsquote bis zu 25 %. Die Resorptionsquote wird vom Organismus in Abhängigkeit vom Eisenbedarf und der Eisenspeichergröße reguliert. Dabei nutzt der menschliche Organismus sowohl zweiwertige als auch dreiwertige Eisenionen. Üblicherweise werden Eisen(III)-Verbindungen bei ausreichend saurem pH-Wert im Magen gelöst und damit für die Resorption verfügbar gemacht. Die Resorption des Eisens findet im oberen Dünndarm durch Mukosazellen statt. Dabei wird dreiwertiges nicht Häm Eisen für die Resorption z.B. durch Ferrireduktase (membranständiges, duodenales Cytochrom b) in der Darmzellmembran zunächst zu Fe(II) reduziert, um dann durch das Transportprotein DMT1 (divalent metal transporter 1) in die Darmzellen transportiert werden zu können. Dagegen gelangt Häm Eisen unverändert über die Zellmembran in die Enterozyten. In den Enterozyten wird Eisen entweder als Depot-Eisen in Ferritin gespeichert oder durch das Transportprotein Ferroportin ins Blut abgegeben. In diesem Vorgang spielt Hepcidin eine zentrale Rolle, da es den wesentlichen Regulationsfaktor der Eisenaufnahme darstellt. Das durch das Ferroportin ins Blut transportierte zweiwertige Eisen wird durch Oxidasen (Ceruloplasmin, Hephaestin) in dreiwertiges Eisen überführt, welches dann mittels Transferrin an die relevanten Stellen im Organismus transportiert wird (s. zum Beispiel: "Balancing acts: molecular control of mammalian iron metabolism". M.W. Hentze, Cell 117,2004,285-297.)

Der Organismus von Säugetieren kann Eisen nicht aktiv ausscheiden. Der Eisenmetabolismus wird im Wesentlichen über die zelluläre Freisetzung von Eisen aus Makrophagen, Hepatocyten und Enterozyten durch das Hepcidin gesteuert.

Ein verringerter Serum-Eisenspiegel führt in krankhaften Fällen zu einem verringerten Gehalt an Hämoglobin, verringerter Erythrozytenproduktion und damit zu einer Anämie.

Äussere Symptome von Anämien beinhalten Müdigkeit, Blässe sowie verringerte Aufmerksamkeitskapazitäten. Die klinischen Symptome einer Anämie beinhalten niedrige Serum-Eisengehalte (Hypoferrämie), geringe Hämoglobingehalte, geringe Hämatokritlevel sowie eine reduzierte Anzahl an roten Blutkörperchen, reduzierte Retikulozyten und erhöhte Werte an löslichen Transferrinrezeptoren.

Eisenmangelerscheinungen oder Eisenanämien werden durch Eisenzufuhr behandelt. Dabei erfolgt die Substitution mit Eisen entweder auf dem oralen Weg oder durch intravenöse Eisengabe. Außerdem können zur Ankurbelung

der Bildung von roten Blutkörperchen auch Erythropoetin und andere Erythropoese-stimulierende Substanzen bei der Behandlung von Anämien eingesetzt werden.

Anämie kann oft auf Mangelernährung bzw. eisenarme Diäten oder unausgewogene, eisenarme Ernährungsgewohnheiten zurück geführt werden. Außerdem treten Anämien durch verringerte bzw. schlechte Eisenabsorption beispielsweise aufgrund von Gastrektomien oder von Erkrankungen wie Crohn's-Disease auf. Auch kann ein Eisenmangel infolge eines erhöhten Blutverlustes z.B. durch eine Verletzung, starke Menstruationsblutung oder Blutspende auftreten. Auch ist ein erhöhter Eisenbedarf in der Wachstumsphase Heranwachsender und Kinder sowie von Schwangeren bekannt. Da ein Eisenmangel nicht nur zu einer verringerten Bildung von roten Blutkörperchen, sondern damit auch zu einer schlechten Versorgung des Organismus mit Sauerstoff führt, was zu den oben genannten Symptomen wie Müdigkeit, Blässe und Konzentrationsschwäche auch gerade bei Heranwachsenden zu langfristigen negativen Auswirkungen auf die kognitive Entwicklung führen kann, ist eine gut wirksame und gut verträgliche Therapie von besonderem Interesse.

Durch die Verwendung der erfindungsgemäßen Fe(III)-Komplexverbindungen besteht die Möglichkeit, Eisenmangelerscheinungen und Eisenmangelanämien durch orale Applikation effektiv zu behandeln, ohne das grosse Nebenwirkungspotential der klassischen Präparate, der Fe(II)-Eisensalze, wie $FeSO_4$, hervorgerufen durch Oxidativen Stress, in Kauf zu nehmen. Somit wird eine schlechte Compliance vermieden, die oft Grund für die mangelnde Beseitigung des Eisenmangelzustands darstellt.

**Stand der Technik:**

Aus dem Stand der Technik ist eine Vielzahl von Eisenkomplexen für die Behandlung von Eisenmangelzuständen bekannt.

Ein sehr grosser Anteil dieser Komplex-Verbindungen besteht aus polymeren Strukturen. Hierbei handelt es sich bei den meisten Komplex-Verbindungen um Eisen-Polysaccharid-Komplexverbindungen (WO20081455586, WO2007062546, WO20040437865, US2003236224, EP150085). Gerade aus diesem Bereich sind schon Medikamente auf dem Markt verfügbar (wie Maltofer, Venofer, Ferinject, Dexferrum, Ferumoxytol).

Ein anderer grosser Bestandteil der Gruppe der polymeren Komplexverbindungen sind die Eisen-Peptid-Komplexverbindungen (CN101481404, EP939083, JP02083400).

In der Literatur sind auch Fe-Komplexverbindungen beschrieben, die sich von Makromolekülen, wie Hämoglobin, Chlorophyll, Curcumin und Heparin strukturell herleiten (US474670, CN1687089, Biometals, 2009,22,701-710).

Ferner sind in der Literatur auch niedermolekulare Fe-Komplexverbindungen beschrieben. Eine Vielzahl dieser Fe-Komplexverbindungen umfasst Carbonsäuren und Aminosäuren als Liganden. Besonders stehen hier Aspartat (US2009035385) und Citrat (EP308362) als Liganden im Vordergrund. In diesem Zusammenhang werden auch Fe-Komplexverbindungen, die derivatisierte Phenylalanin-Reste als Liganden beinhalten beschrieben (ES2044777).

Im weiteren sind in der Literatur Fe-Komplexverbindungen beschrieben, die aus monomeren Zuckereinheiten oder aus einer Kombination von monomeren und polymeren Einheiten aufgebaut sind (FR19671016).

US 2005/0192315 offenbart pharmazeutische Zusammensetzungen, die Chinolin-Verbindungen enthalten, die formal ein 2-(Hydroxymethylen)propandiamid-, also ein Propandiamid-Strukturelement, aufweisen. Sie weisen demzufolge kein 2-Oxo-butandiamid-Strukturelement auf.

In der Literatur sind auch Hydroxypyron- und Hydroxypyridon-Fe-Komplexverbindungen beschrieben (EP159194, EP138420, EP107458). Analog hierzu sind auch die entsprechenden 5-Ringsysteme, die Hydroxyfuranon-Fe-Komplexverbindungen beschrieben (WO2006037449).

Ausserdem sind in der Literatur auch Fe-Komplexverbindungen mit Pyrimidin-2-ol-1-oxid Liganden beschrieben, die für die Behandlung von Eisenmangelanämien eingesetzt werden sollen (WO2012130882). Die WO2012163938 beschreibt Eisen(III)-2,4-dioxo-1-carbonyl-Komplexverbindungen, die ebenfalls für die Behandlung von Eisenmangelanämien eingesetzt werden sollen.

Im weiteren sind in der Literatur auch Fe-Komplexe mit β-Ketoamid-Liganden beschrieben, deren Verwendung für die Behandlung von Eisenmangelzuständen vorgeschlagen werden (WO2011117225). Einen Hinweis auf Fe(III)-Komplexverbindungen mit 2-Oxo-butandiamid-Liganden findet sich in dieser Anmeldung nicht. Des weiteren sind die Fe-Komplexe mit β-Ketoamid-Liganden insbesondere im Hinblick auf ihre Wasserlöslichkeit sowie ihre Eisenutilisation noch verbesserungswürdig.

Ein andere wichtiger Bestandteil für die Behandlung von Eisenmangelerscheinungen und Eisenmangelanämien stellen die Eisensalze (z.B. Eisen(II)sulfat, Eisen(II)fumarat, Eisen(III)chlorid, Eisen(II)aspartat, Eisen(II)succinat) dar.

Ein grosses Problem dieser Eisensalze stellt ihre teilweise hohe Unverträglichkeit (bis zu 50%) in Form von Übelkeit, Erbrechen, Durchfall aber auch Obstipation und Krämpfe dar. Ausserdem kommt es bei der Verwendung von diesen Eisen(II)-salzen zum Auftreten freier Fe(II)Ionen, die die Bildung (u.a. Fenton Reaktion) von reaktiven Sauerstoff Spezies (ROS) katalysieren. Durch diese ROS kommt es zur Beschädigung von DNA, Lipiden, Proteinen und Kohlenhydraten, was weitreichende Auswirkungen auf Zellen, Gewebe und Organe hat. Diese Problematik ist bekannt und

wird in der Literatur weitgehend als Ursache für die hohe Unverträglichkeit angesehen und als Oxidativer Stress bezeichnet.

**Aufgabenstellung:**

Die Aufgabe der vorliegenden Erfindung bestand darin, neue therapeutisch wirksame Verbindungen mit guter Aktivität bzw. Eisenutilisation, Komplexstabilität und Löslichkeit, insbesondere guter Stabilität und guter Löslichkeit im pH-Wert neutraler wässriger Medien zu entwickeln, die für eine oralen Behandlung von Eisenmangelerscheinungen und Eisenmangelanämien eine effektive Therapie darstellen. Gerade eine gute Stabilität und eine gute Löslichkeit sind sehr wichtig für eine effektive orale Eisentherapie. Ausserdem sollten diese Eisenkomplexe gleichzeitig deutlich weniger Nebenwirkungen bzw. eine geringe Toxizität aufzeigen, insbesondere im Vergleich zu den klassisch verwendeten Fe(II)-Salzen. Im Vergleich zu den bekannten polymeren Eisenkomplexverbindungen sollten diese neuen Eisenkomplexe eine definierte Struktur (Stöchiometrie) aufweisen und durch einfache Synthesewege darstellbar sein. Diese Ziele wurden durch die Entwicklung neuartiger Fe(III)-Komplexverbindungen erreicht.

Weiterhin sollten die neuen Eisenkomplexe so beschaffen sein, das sie direkt über die Membran in die Darmzellen aufgenommen werden, um somit ihr komplexgebundenes Eisen direkt an das Ferritin oder an das Transferrin abzugeben oder direkt als intakter Komplex in die Blutbahn zu gelangen. Diese neuen Komplexe sollten aufgrund ihrer Eigenschaften praktisch nicht zum Auftreten von hohen Konzentrationen an freien Eisenionen führen. Denn gerade durch freie Eisenionen kommt es zum Auftreten von ROS, die letztlich für die auftretenden Nebenwirkungen verantwortlich sind.

Um diese Anforderungen erreichen zu können, entwickelten die Erfinder neue potente Fe(III)-Komplexverbindungen mit nicht zu grossem Molekulargewicht, mittlerer Lipophilie, sehr guter Aktivität bzw. Eisenutilisation, hoher Wasserlöslichkeit und optimaler pH-Wert abhängiger Komplexstabilität.
Bei der Entwicklung der neuen Komplexe sollte die Stabilitätverbesserung gerade im neutralen wässrigen Medium nicht auf Kosten der Löslichkeit gehen, da die Löslichkeit für orale Anwendungen ein sehr wichtiges Kriterium darstellt. Dieses kombinierte Ziel wird mit den erfindungsgemäßen Fe-Komplexen erreicht. Diese zeigen eine gute Stabilität im wässrigen Medium bei neutralem pH-Wert und verfügen gleichzeitig über eine sehr gute Wasserlöslichkeit. Somit ermöglichen es die erfindungsgemäßen EisenKomplexverbindungen einen deutlich schnelleren Behandlungserfolg zu erreichen.

**Beschreibung der Erfindung:**

Die Erfinder fanden, dass neue Fe(III)-Komplexverbindungen mit 2-Oxo-butandiamid-Liganden sich für die oben beschriebenen Anforderungen besonders eignen. Es konnte gezeigt werden, dass diese Fe-Komplexverbindungen eine hohe Eisenaufnahme zeigen, wodurch ein schneller Therapieerfolg bei der Behandlung der Eisenmangelanämie erreicht werden kann. Gerade im Vergleich mit Eisensalzen zeigen die erfindungsgemäßen Komplexverbindungen eine schnellere und höhere Utilisation. Ausserdem weisen diese neuen Systeme deutlich geringere Nebenwirkungen als die klassisch verwendeten Eisensalze auf, da es hier nicht in nennenswertem Ausmaß zum Auftreten von freien Eisenionen kommt. Die erfindungsgemäßen Komplexverbindungen zeigen nahezu keinen Oxidativen Stress, da es nicht zur Bildung von freien Radikalen kommt. Somit treten bei diesen Komplexverbindungen deutlich weniger Nebenwirkungen als bei den aus dem Stand der Technik bekannten Fe Salzen auf. Die Komplexverbindungen zeigen sowohl im sauren als auch im neutralen pH-Wert-Bereich gute Stabilitäten was gerade für orale Anwendungen sehr vorteilhaft ist. Die Komplexverbindungen lassen sich gut herstellen und eignen sich optimal zur Formulierung von Arzneimitteln, insbesondere zur oralen Verabreichung.

Gegenstand der Erfindung sind somit Eisen(III)-2-oxo-butandiamid-Komplexverbindungen oder deren Salze, insbesondere pharmazeutisch verträgliche Salze zur Verwendung als Arzneimittel. Gegenstand der Erfindung sind demnach auch Eisen(III)-2-oxo-butandiamid-Komplexverbindungen oder deren pharmazeutisch verträglichen Salze zur Anwendung in einem Verfahren zur therapeutischen Behandlung des menschlichen oder tierischen Körpers.

Die erfindungsgemäß verwendeten Eisen(III)-2-oxo-butandiamid-Komplexverbindungen schließen solche Verbindungen ein, die mindestens einen Liganden der Formel (I) enthalten:

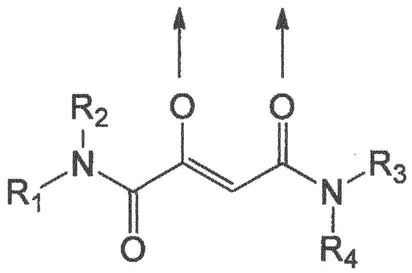

(I)

worin
die Pfeile jeweils eine koordinative Bindung zu einem Eisenatom darstellen,

$R_1$ und $R_2$ gleich oder verschieden sind, und jeweils ausgewählt werden aus der Gruppe, die besteht aus Wasserstoff und gegebenenfalls substituiertem Alkyl, oder
$R_1$ und $R_2$ gemeinsam mit dem Stickstoffatom, an welches sie gebunden sind, einen gegebenenfalls substituierten 3- bis 6-gliedrigen Ring bilden, der gegebenenfalls ein weiteres Heteroatom aufweisen kann,
$R_3$ und $R_4$ gleich oder verschieden sind, und jeweils ausgewählt werden aus der Gruppe, die besteht aus Wasserstoff und gegebenenfalls substituiertem Alkyl, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein weiteres Heteroatom aufweisen kann
oder pharmazeutisch verträgliche Salze davon.

Bevorzugt sind insbesondere Eisen(III)-2-oxo-butandiamid-Komplexverbindungen, die mindestens einen Liganden der Formel (I) enthalten:

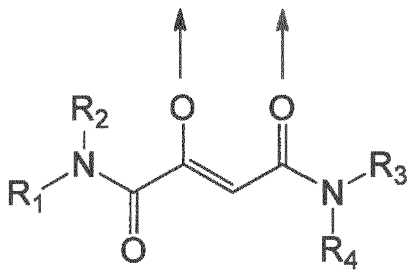

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, i-Propyl, n-Butyl, sek-Butyl und tert-Butyl, und worin die genannten Alkylgruppen substituiert sein können mit einem Substituenten, der ausgewählt wird aus der Gruppe, die besteht aus: Alkoxy, Alkoxycarbonyl, Aminocarbonyl ($H_2NCO$-), Monoalkylaminocarbonyl und Dialkylaminocarbonyl, oder
$R_1$ und $R_2$ zusammen mit dem Stickstoffatom, an das sie gebunden sind, einen gegebenenfalls substituierten 5- bis 6-gliedrigen Ring bilden, der gegebenenfalls ein weiteres Heteroatom aufweisen kann,
$R_3$ und $R_4$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein weiteres Heteroatom aufweisen kann.

Weiterhin sind insbesondere Eisen(III)-2-oxo-butandiamid-Komplexverbindungen bevorzugt, die mindestens einen Liganden der Formel (I) enthalten:

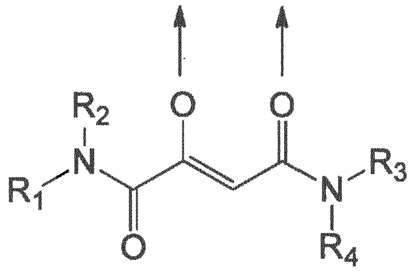

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, i-Propyl, n-Butyl, sek-Butyl und tert-Butyl, und worin die Alkylgruppen substituiert sein können mit einem Substituenten, der ausgewählt wird aus der Gruppe, die besteht aus: Alkoxy, Alkoxycarbonyl, Aminocarbonyl ($H_2NCO$-), Monoalkylaminocarbonyl und Dialkylaminocarbonyl, oder
$R_1$ und $R_2$ zusammen mit dem Stickstoffatom, an das sie gebunden sind, Pyrrolidinyl bilden,
$R_3$ und $R_4$ gleich oder verschieden sind, und jeweils ausgewählt werden aus der Gruppe, die besteht aus Wasserstoff, Alkyl und Cycloalkyl, worin Alkyl und Cycloalkyl substituiert sein können mit einem Substituenten, der ausgewählt wird aus der Gruppe, die besteht aus: Hydroxy, Alkoxy, Alkoxycarbonyl, Aminocarbonyl ($H_2NCO$-), Monoalkylaminocarbonyl und Dialkylaminocarbonyl,
$R_3$ und $R_4$ gemeinsam mit dem Stickstoffatom, an das sie gebunden sind, Morpholinyl oder Pyrrolidinyl bilden, worin die genannten Ringgruppen mit Hydroxy substituiert sein können.

Bevorzugt sind weiterhin insbesondere Eisen(III)-2-oxo-butandiamid-Komplexverbindungen, die mindestens einen Liganden der Formel (I) enthalten:

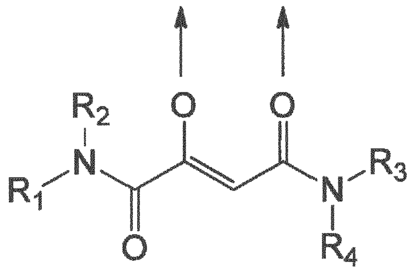

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, Isopropyl, n-Butyl, sek-Butyl und Isobutyl,
$R_3$ und $R_4$ gleich oder verschieden sind, und jeweils ausgewählt werden aus der Gruppe, die besteht aus Wasserstoff und gegebenenfalls substituiertem Alkyl, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein weiteres Heteroatom aufweisen kann.

Besonders bevorzugte Eisen(III)-Komplexverbindungen enthalten mindestens einen Liganden der Formel (I):

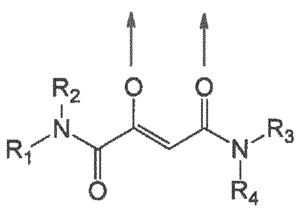

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff und Methyl,
$R_3$ und $R_4$ gleich oder verschieden sind, und jeweils ausgewählt werden aus der Gruppe, die besteht aus Wasserstoff und gegebenenfalls substituiertem Alkyl.

Besonders bevorzugt sind Eisen(III)-Komplexverbindungen nach der Formel (II):

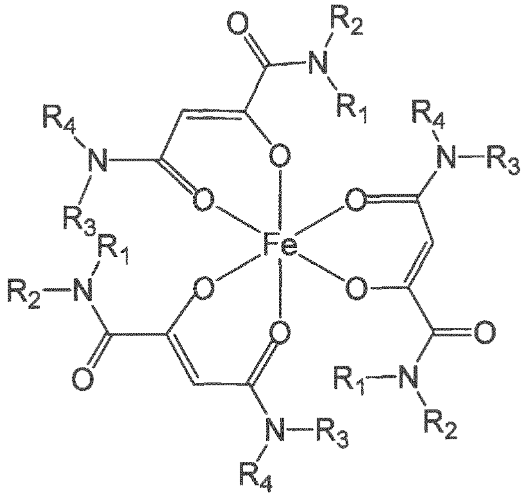

(II)

worin $R_1$, $R_2$, $R_3$ und $R_4$ wie oben definiert sind.

Bevorzugt liegt das Molekulargewicht der erfindungsgemäßen Eisen(III)-2-oxo-butandiamid-Komplexverbindungen bei weniger als 1000 g/mol, noch bevorzugter bei weniger als 800 g/mol (jeweils bestimmt aus der Strukturformel).

Im Rahmen der gesamten Erfindung schließt gegebenenfalls substituiertes Alkyl insbesondere für die Substituenten $R_1$ bis $R_4$ bevorzugt ein:

Geradkettiges oder verzweigtes Alkyl mit 1 bis 6, Kohlenstoffatomen, Cycloalkyl mit 3 bis 6, bevorzugt 5 oder 6 Kohlenstoffatomen, oder Alkyl mit 1 bis 4 Kohlenstoffatomen, welches mit Cycloalkyl substituiert ist, wobei diese Alkylgruppen jeweils gegebenenfalls substituiert sein können.

Die genannten Alkylgruppen können gegebenenfalls jeweils bevorzugt 1 bis 3 Substituenten tragen. Bevorzugter tragen sie keine Substituenten.

Die Substituenten von Alkyl werden bevorzugt aus der Gruppe ausgewählt, die besteht aus: Hydroxy, Alkoxy, Alkoxycarbonyl, Aminocarbonyl ($H_2NCO$-), Monoalkylaminocarbonyl und Dialkylaminocarbonyl, insbesondere wie nachstehend definiert. Bezüglich der Alkylgruppen in den genannten Substituentengruppen Alkoxy, Alkoxycarbonyl, Monoalkylaminocarbonyl und Dialkylaminocarbonyl kann auf die vorstehende und nachstehende Definition von Alkyl verwiesen werden.

Die Substituenten von Alkyl werden bevorzugt aus der Gruppe ausgewählt, die besteht aus: Hydroxy und gegebenenfalls substituiertem Alkoxy insbesondere wie nachstehend definiert.

In den vorstehend definierten Alkylgruppen können des Weiteren gegebenenfalls ein oder zwei Kohlenstoffatome durch Sauerstoff ersetzt sein. Dies bedeutet insbesondere, dass eine oder zwei Methylengruppen ($-CH_2-$) in den Alkylresten durch -O- ersetzt sein können.

Beispiele von Alkylresten mit 1 bis 6 Kohlenstoffatomen schließen ein: eine Methylgruppe, eine Ethylgruppe, eine n-Propylgruppe, eine i-Propylgruppe, eine n-Butylgruppe, eine i-Butylgruppe, eine sec-Butylgruppe, eine t-Butylgruppe, eine n-Pentylgruppe, eine i-Pentylgruppe, eine sec-Pentylgruppe, eine t-Pentylgruppe, eine 2-Methylbutylgruppe, eine n-Hexylgruppe, eine 1-Methylpentylgruppe, eine 2-Methylpentylgruppe, eine 3-Methylpentylgruppe, eine 4-Methylpentylgruppe, eine 1-Ethylbutylgruppe, eine 2-Ethylbutylgruppe, eine 3-Ethylbutylgruppe, eine 1,1-Dimethylbutylgruppe, eine 2,2-Dimethylbutylgruppe, eine 3,3-Dimethylbutylgruppe, eine 1-Ethyl-1-methylpropylgruppe usw. Bevorzugt sind solche mit 1 bis 4 Kohlenstoffatomen. Am meisten bevorzugt sind Methyl, Ethyl, n-Propyl und n-Butyl.

Cycloalkylreste mit 3 bis 6 Kohlenstoffatomen schließen eine Cyclopropylgruppe, eine Cyclobutylgruppe, eine

Cyclopentylgruppe und eine Cyclohexylgruppe ein. Die Cycloalkylreste können gegebenenfalls substituiert sein, bevorzugt mit einem Substituenten, wie Hydroxy, Methyl oder Methoxy.

Die Definition der gegebenenfalls substituierten Alkylgruppen schließt auch Alkylgruppen ein, welche durch vorstehend definierte Cycloalkylgruppen substituiert sind, wie beispielsweise Cyclopropylmethyl, Cyclobutylmethyl, Cyclopentylmethyl oder Cyclohexylmethyl.

Beispiele eines mit Hydroxy substituierten Alkylrestes schließen die oben genannten Alkylreste ein, die 1 bis 2 Hydroxylreste aufweisen, wie zum Beispiel Hydroxymethyl, 2-Hydroxyethyl, 3-Hydroxypropyl, 4-Hydroxybutyl, etc.

Gegebenenfalls substituiertes Alkoxy (RO-) schließt erfindungsgemäß beispielsweise lineare oder verzweigte Alkoxyreste bevorzugt mit bis zu 4 Kohlenstoffatomen ein, wie eine Methoxygruppe, eine Ethoxygruppe, eine n-Propyloxygruppe und eine i-Propyloxygruppe ein. Die Alkoxygruppen können gegebenenfalls substituiert sein, wie beispielsweise mit den für Alkyl genannten möglichen Substituenten, insbesondere mit 1 bis 3, bevorzugter 1 Substituenten.

Bevorzugtes Alkoxy ist Methoxy und Ethoxy.

Alkoxycarbonyl schließt im Rahmen der Erfindung vorstehend beschriebenes Alkoxy ein, an welches eine Carbonylgruppe gebunden ist (schematisch: RO(C=O)-, worin R Alkyl wie vorstehend erwähnt ist). Beispiele schließen ein Methoxycarbonyl und Ethoxycarbonyl.

Monoalkylaminocarbonyl und Dialkylaminocarbonyl schließen im Rahmen der Erfindung schematisch Reste der Formeln HRN-C(=O)- und $R_2$N-C(=O)- ein, wobei bezüglich der Alkylgruppen (R) auf die vorstehende Definition von Alkyl verwiesen werden kann. Beispiele schließen ein: Methylaminocarbonyl und Dimethylaminocarbonyl.

Der 2-Oxo-butandiamid-Komplexligand schließt erfindungsgemäß den entsprechenden Grundkörper:

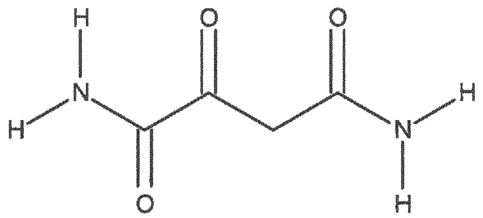

2-oxobutanediamide

sowie sämtliche Verbindungen ein, in denen eines oder mehrere der vorhandenen Wasserstoffatome durch andere Atome bzw. Atomgruppen substituiert sind, wobei zur Ausbildung der nachfolgend beschriebenen Keto-Enol-Tautomerie mindestens ein Wasserstoffatom zwischen den beiden koordinierenden C=O-Gruppen vorhanden sein muß.

Es ist für den Fachmann klar, dass die erfindungsgemäßen 2-Oxo-butandiamid-Komplexliganden, insbesondere die Liganden der Formel (I)

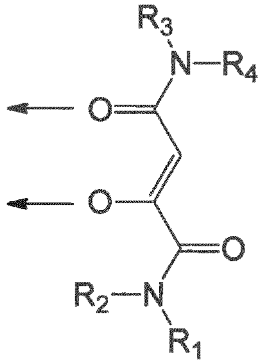

(I)

aus den entsprechenden 2-Oxo-butandiamid -Verbindungen (III) hervorgehen:

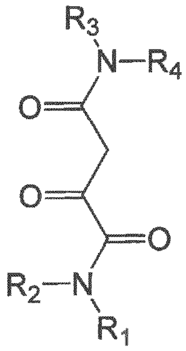

(III) ,

in denen bekanntermaßen eine Keto-Enol-Tautomerie vorliegt:

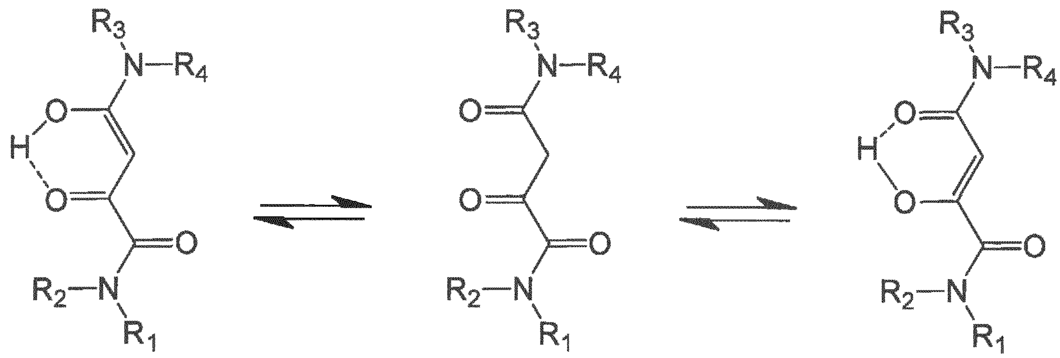

A B C .

Die mesomeren Grenzformen A und C sind analytisch nicht unterscheidbar. Im Rahmen der vorliegenden Erfindung sind in jedem Fall sämtliche Formen eingeschlossen, wobei jedoch im Rahmen der vorliegenden Erfindung bei den Liganden im Allgemeinen nur die Ketoform gezeichnet ist.

Der Ligand geht formal durch Abspalten eines Protons aus den entsprechenden Enolformen A oder C hervor:

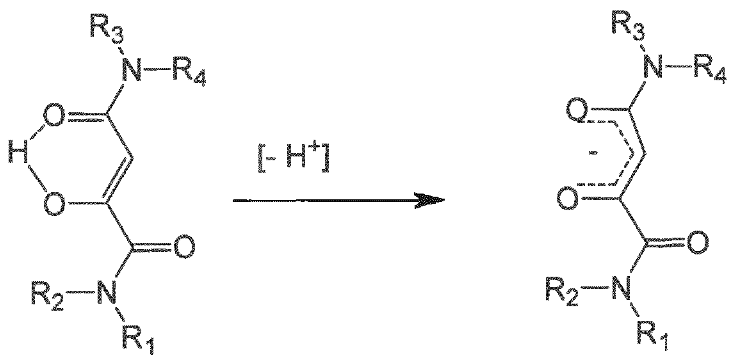


,

trägt also formal eine einfach negative Ladung. Auch bei den Eisen-Komplex-verbindungen ist im Rahmen der vorliegenden Erfindung stets lediglich eine der beiden lokalisierten Grenzformeln gezeigt:

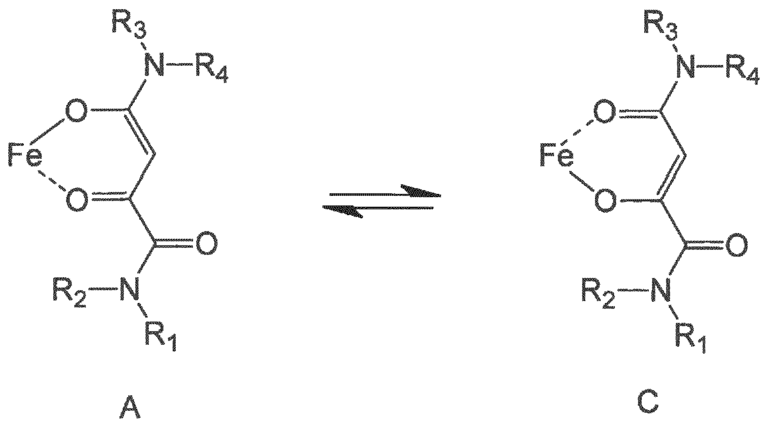

wobei aufgrund der geringeren Elektronendichte am amidischen Sauerstoffatom der Amidgruppe in 1,3 Position zur Ketogruppe, zu erwarten ist, dass die Grenzformel C vorherrschen sein sollte. Eine analytische Unterscheidung der Grenzformeln A und C ist jedoch wie gesagt nicht möglich. Die andere Amidgruppe, die in 1,2 Position zur Ketogruppe steht, scheint aufgrund der analytischen Daten nicht am Eisenbindungsmodus beteiligt zu sein. IR Messungen am Komplex konnten zeigen, dass hier nur eine sehr geringe Verschiebung der IR Banden zwischen freiem Ligand und Komplex für diese Amidgruppe beobachtet werden konnte, was gegen eine Bindungsbeteidigung dieser Carbonylgruppe am Eisenkomplex spricht.

Beispiele von erfindungsgemäß verwendeten 2-Oxo-butandiamid -Liganden werden nachfolgend dargestellt:

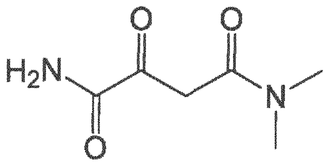

Die Erfindung betrifft weiterhin ein Verfahren zur Herstellung der erfindungsgemäßen Eisen(III) -Komplexverbindungen, welches die Umsetzung eines 2-Oxo-butandiamids (III) mit einem Eisen(III)-salz (IV) umfasst.
2-Oxo-butandiamide schließen insbesondere diejenigen der Formel (III) ein:

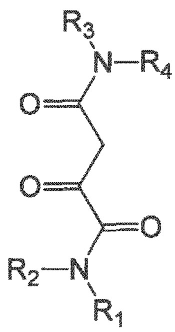

(III)

worin $R_1$ bis $R_4$ wie oben definiert sind.

Beispiele geeigneter Eisen(III)-salze schließen ein: Eisen(III)-chlorid, Eisen(III)-acetat, Eisen(III)-sulfat, Eisen(III)-nitrat und Eisen(III)-acetylacetonat, worunter Eisen(III)-chlorid bevorzugt ist.

Ein bevorzugtes Verfahren ist im folgenden Schema gezeigt:

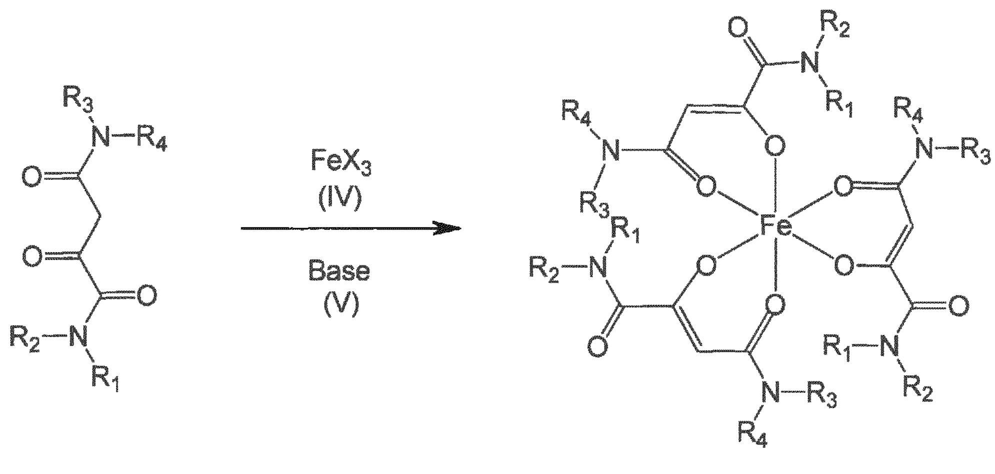


(III) (II) ,

worin $R_1$ bis $R_4$ wie oben definiert sind, X ein Anion wie Halogenid, wie Chlorid, ein Carboxylat, wie Acetat, Sulfat, Nitrat und Acetylacetonat ist und Base eine übliche organische oder anorganische Base darstellt.

Beim erfindungsgemäßen Verfahren werden bevorzugt 3-5 eq Ligand (III) unter Verwendung von geeigneten Eisen(III)-salzen (IV) (besonders eignen sich hier Fe(III)-chlorid, Fe(III)-acetat, Fe(III)-sulfat und Fe(III)-acetylacetonat) zu den entsprechenden Komplexen, der allgemeinen Formel (II), unter Standardbedingungen umgesetzt. Hierbei wird die Synthese unter den für die Komplexbildung optimalen pH-Bedingungen durchgeführt. Der optimale pH-Wert wird gegebenenfalls durch Zugabe von Base (V), besonders eignet sich hier die Verwendung von Natrium-Acetat, Triethylamin, NatriumCarbonat, Natrium-Hydrogencarbonat, Natrium-Methanolat, Natrium-Ethanolat, Kaliumcarbonat, Kalium-Hydrogencarbonat oder Kalium-Methanolat, eingestellt.

Die für die Darstellung der Komplexe benötigten Liganden (III) sind entweder käuflich erhältlich oder wurden nach folgender Synthesemethode dargestellt. Hierzu wurde nachfolgender Syntheseweg beschritten.
Im Falle der teilsubstituierten Amide (III) ($R_1$=H; ,$R_2$=H od. $R_2 \neq H$; $R_3,R_4 \neq H$) wird durch basische Kondensationsreaktion aus einem Oxalsäureester, der allgemeinen Formel **4** und einem Dialkylacetamid, der allgemeinen Formel **5** zunächst Ester **6** hergestellt. (R. J. Gobeil et al, Journal of the American Chemical Society, 1945, 67, 511). Als Basen kommen verschiedene Kondensationsbasen wie beispilesweise Buthyllithium, Lithiumdiisopropylamin, Natrium, Natriumhydrid, Natriumamid, Natriumalkoholate, Kalium, Kaliumhydrid, Kaliumamid und Kaliumalkoholate in Frage, wobei Kalium-tert-butylat bevorzugt ist.

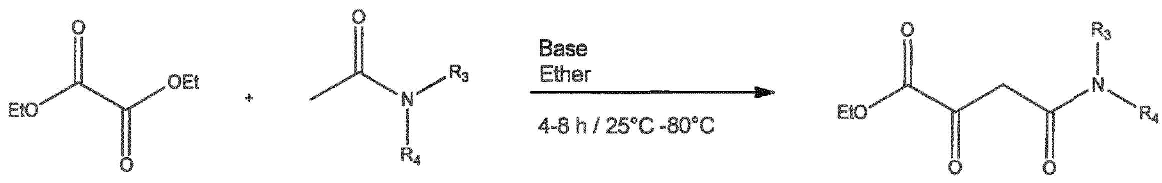


4 5 6

Zur Herstellung von Amid (III) wird Ester **6** in einen Metallkomplex 7 überführt. Als Metall ist Kupfer bevorzugt, obwohl auch andere Übergangsmetalle in Frage kommen. Anschliessend wird der Metallkomplex **7** mit dem entsprechenden Amin zum einem Metallkomplex von Amid (III) umgesetzt. Amid (III) wird abschliessend mit verdünnter Mineralsäure aus dem entsprechenden Metallkomplex freigesetzt (A. Ichiba et al, Journal of the Scientific Research Institute, Tokyo, 1948, 23, 23-29), wobei im Falle von Kupfer verdünnte Schwefelsäure bevorzugt ist. Sollte das Amid (III) zu gut wasserlöslich sein, kann die Freisetzung auch mit Schwefelwasserstoff in einem organischen Lösungsmittel erfolgen, wobei Methanol bevorzugt ist.

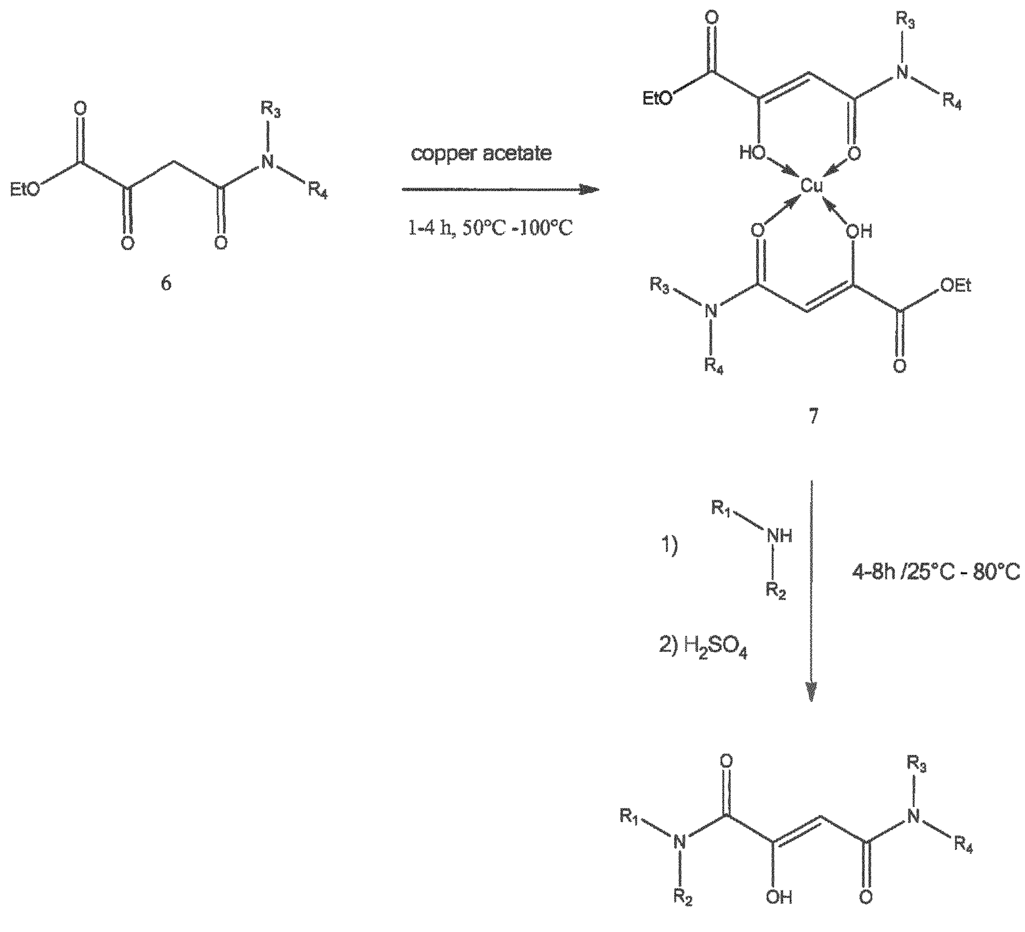


6

7

(III)

Alternativ hierzu lässt sich Ester **6** auch mit ortho-Ameisensäurealkylestern zum Ketal **8** umsetzen. (J. Perronnet et al, Journal of Heterocyclic Chemistry, 1980, 17, 727-731). Dieses wird dann jeweils mit den entsprechenden Aminen zum jeweiligen Diamidkomplex umgesetzt, welcher bei wässrig saurer Aufarbeitung zu Amid (III) hydrolisiert wird. (W. Kantlehner et al, Liebigs Annalen der Chemie, 1980, 9, 1448-1454).

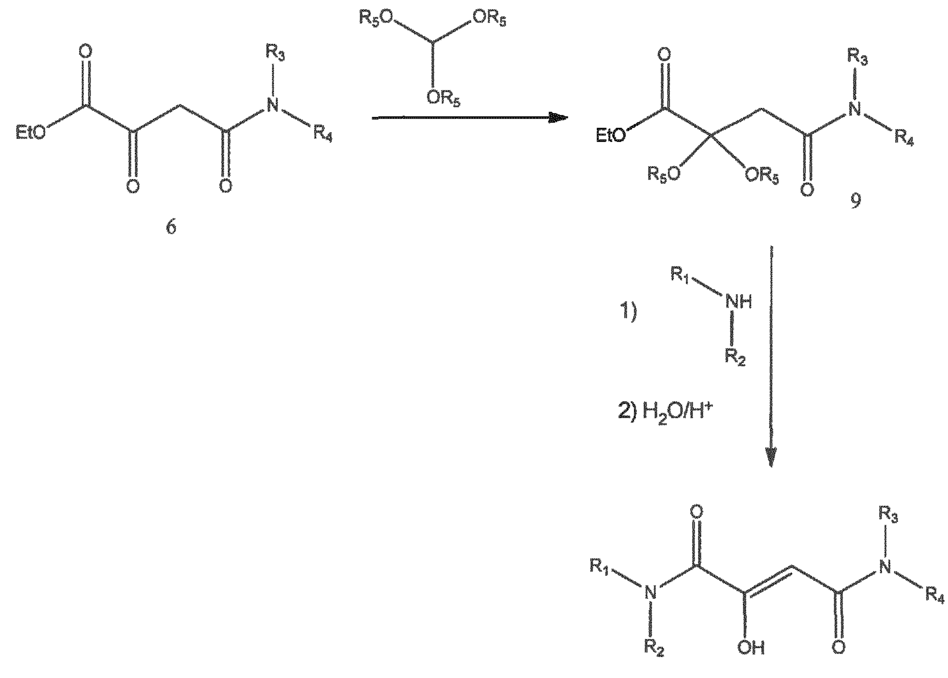


6

9

(III)

Als weiteres Verfahren zur Herstellung von Amid (III) mit beliebigem Substitutionsmuster ($R_1$=H od. $R_1$≠H; $R_2$=H od. $R_2$≠H; $R_3$=H od. $R_3$≠H, $R_4$=H od. $R_4$≠H) wird die Synthese ausgehend von (2Z)-(2,2-dimethyl-5-oxo-1,3-dioxolan-4-yliden)acetyl chlorid durchgeführt. Hierbei wird das Säurechlorid 10 mit dem entsprechenden Amin zu Acetonid 11 umgesetzt, welches in einem zweiten Reaktionsschritt zu Amid (III) geöffnet wird. (J. Banville et al, Tetrahedron Letters, 2010, 51, 3170 - 3173).

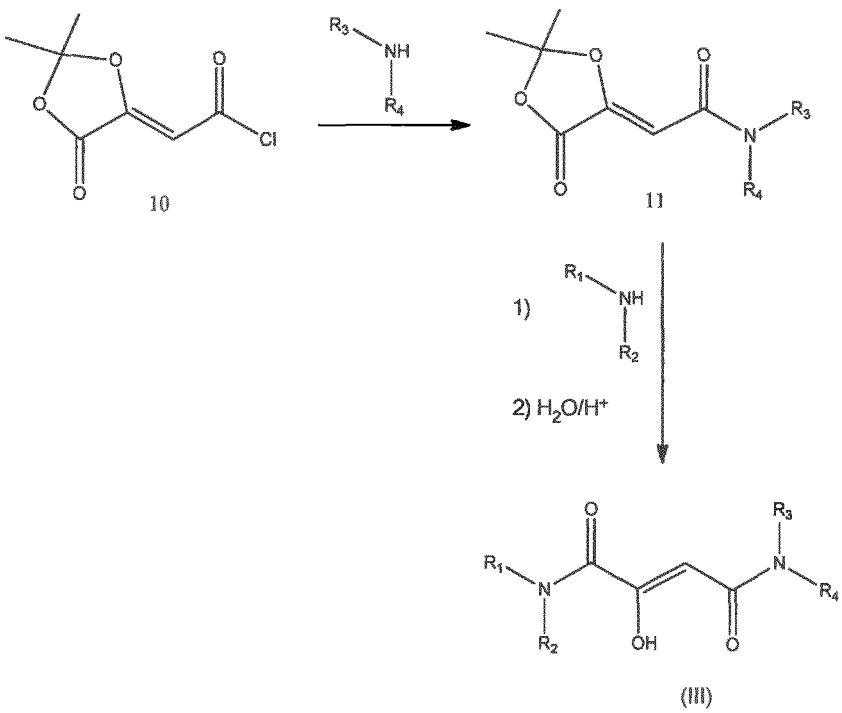


10

11

(III)

Um eine Verbesserung der Ausbeute in der letzten Stufe zu erzielen, kann Acetonid 11 zuerst zu einem Metallkomplex **7a** (mit R3=H od. R3≠H, R4=H od. $R_4$≠H) umgesetzt werden. Als Metall ist Kupfer bevorzugt, obwohl auch andere Übergangsmetalle in Frage kommen. Anschliessend wird der Metallkomplex **7a** mit dem entsprechenden Amin zum einem Metallkomplex von Amid (III) umgesetzt. Amid (III) wird abschliessend mit verdünnter Mineralsäure aus dem

entsprechenden Metallkomplex freigesetzt (A. Ichiba et al, Journal ofthe Scientific Research Institute, Tokyo, 1948, 23, 23-29), wobei im Falle von Kupfer verdünnte Schwefelsäure bevorzugt ist. Sollte Amid III zu gut wasserlöslich sein, kann die Freisetzung auch mit Schwefelwasserstoff in einem organischen Lösungsmittel erfolgen, wobei Methanol bevorzugt ist.

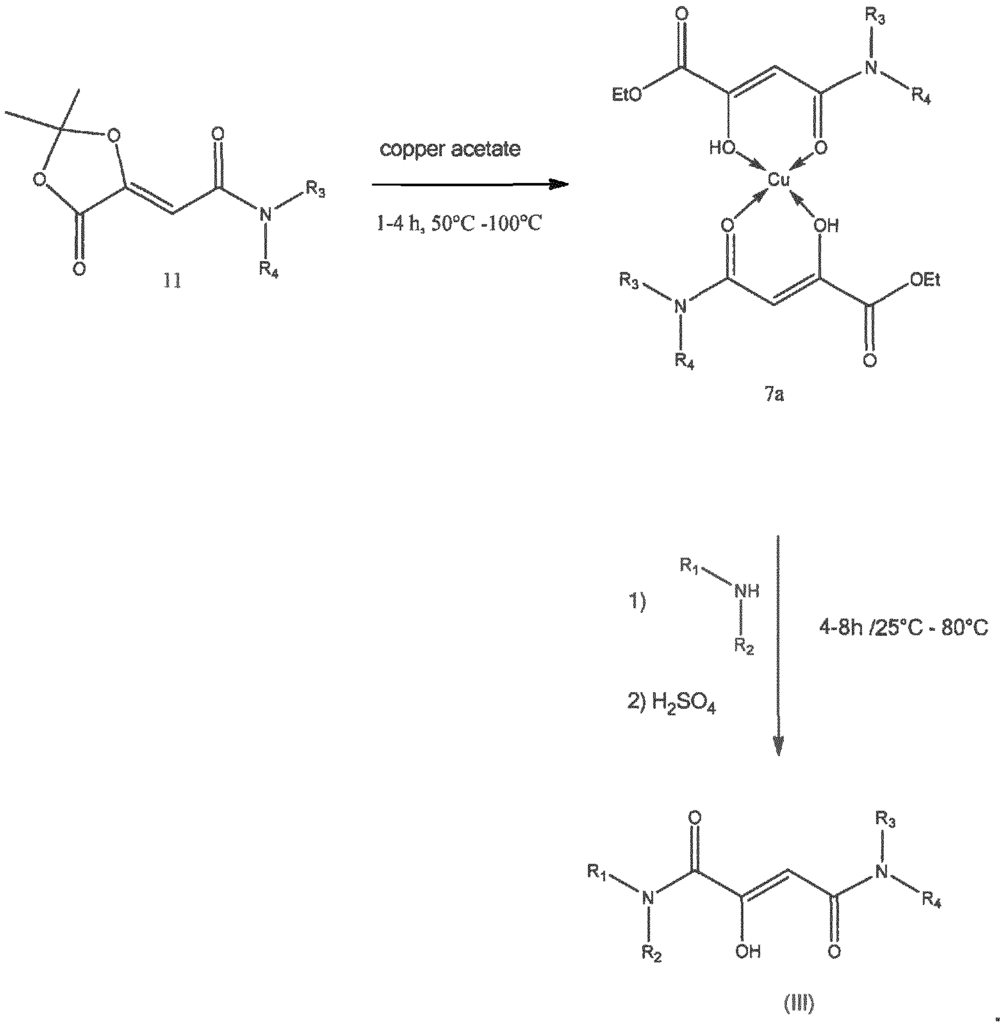

11
7a
(III)

Für den vollsubstituierten Fall ($R_1$, $R_2$, $R_3$, $R_4 \neq H$) vereinfacht sich die Synthese auf eine klassische Kondensationsreaktion. Hierbei wird käuflich verfügbares Alkyl N,N-dialkyloxamat 9 und Dialkylacetamid 5 mit einer geeigneten Kondensationsbase direkt zu Amid (III) umgesetzt. Als Basen kommen verschiedene Kondensationsbasen wie Buthyllithium, Lithiumdiisopropylamin, Natrium, Natriumhydrid, Natriumamid, Natriumalkoholate, Kalium, Kaliumhydrid, Kaliumamid und Kaliumalkoholate in Frage, wobei Kalium-tert-butylat bevorzugt ist.

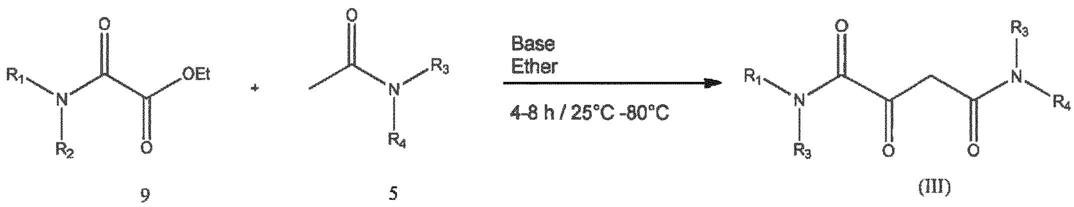

9
5
(III)

Hierbei sind die Reste $R_1$ bis $R_4$ wie beschrieben und wie jeweils oben definiert.
Pharmazeutisch annehmbare Salze der erfindungsgemäßen Verbindungen, in denen der Eisen(III)-Komplex formal eine positive Ladung trägt, schließen beispielsweise Salze mit geeigneten Anionen ein, wie Carboxylate, Sulfonate, Sulfate, Chloride, Bromide, Iodide, Phosphate, Tartrate, Methansulfonate, Hydroxyethansulfonate, Glycinate, Maleate, Propionate, Fumarate, Toluolsulfonate, Benzolsulfonate, Trifluoracetate, Naphthalindisulfonate-1,5, Salicylate, Benzoate, Lactate, Salze der Äpfelsäure, Salze der 3-Hydroxy-2-naphthoesäure-2, Citrate und Acetate ein.
Pharmazeutisch annehmbare Salze der erfindungsgemäßen Verbindungen, in denen der Eisen(III)-Komplex formal eine negative Ladung trägt, schließen beispielsweise Salze mit geeigneten pharmazeutisch annehmbaren Basen ein, wie z.B. Salze mit Alkali- oder Erdalkalihydroxiden, wie NaOH, KOH, $Ca(OH)_2$, $Mg(OH)_2$ etc., Aminverbindungen, wie Ethylamin, Diethylamin, Triethylamin, Ethyldiisopropylamin, Ethanolamin, Diethanolamin, Triethanolamin, Methylglucamin, Dicyclohexylamin, Dimethylaminoethanol, Prokain, Dibenzylamin, N-Methylmorpholin, Arginin, Lysin, Ethylendiamin, N-Methylpiperidin 2-Amino-2-methyl-propanol-(1), 2-Amino-2-methyl-propandiol-(1,3) , 2-Amino-2-hydroxyl-methyl-propandiol-(1,3) (TRIS). Die Wasserlöslichkeit bzw. die Löslichkeit in physiologischer Kochsalzlösung und damit gegebenenfalls auch die Wirksamkeit der erfindungsgemäßen Verbindungen kann durch Salzbildung generell, speziell auch durch die Wahl des Gegenions signifikant beeinflusst werden.
Bevorzugt stellen die erfindungsgemäßen Verbindungen neutrale Komplex-Verbindungen dar.

**Vorteilhafte pharmakologische Effekte:**

Die Erfinder fanden überraschend, dass die Eisen(III)-2-oxo-butandiamid-Komplexverbindungen, die Gegenstand der vorliegenden Erfindung sind, und die insbesondere durch die allgemeine Strukturformel (II) dargestellt werden, stabile bioverfügbare Eisenkomplexe sind und zur Verwendung als Arzneimittel zur Behandlung und Prophylaxe von Eisenmangelerscheinungen und Eisenmangelanämien sowie der damit einhergehenden Symptome geeignet sind.

Die erfindungsgemäßen Verbindungen enthaltende Arzneimittel eignen sich zum Einsatz in der Human- und der Veterinärmedizin.

Die erfindungsgemäßen Verbindungen eignen sich somit auch zur Herstellung eines Medikamentes zur Behandlung von Patienten, die unter Symptomen einer Eisenmangelanämie wie zum Beispiel: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit oder einer depressive Verstimmungen leiden.

Die erfindungsgemäßen Eisen(III)-Komplexverbindungen eignen sich weiterhin zur Behandlung der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3 -5 (CDK 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus) und Eisenmangelanämien bei inflammatorischen Darmerkrankungen (IBD; inflammatory bowel diseases). Die erfindungsgemäßen Eisen(III)-Komplexverbindungen eignen sich auch zur Behandlung von Eisenmangel, wenn der Haemoglobin-Wert im Normbereich liegt. Eisenmangel kann nämlich trotz Haemoglobin-Werten im Normbereich auftreten. Als Normbereich wird im allgemeinen der Bereich bezeichnet, in dem die Hb-Werte von 96 Prozent aller gesunden Menschen liegen. Der Normbereich des Hämoglobins beim Menschen ist wie folgt (s. Wikipedia):

| | **g/dl** | **mmol/l** |
|---|---|---|
| Männer | 13,5-17,5 | 8,4-10,9 |
| Frauen | 12-16 | 7,4-9,9 |
| Neugeborene | 19 | 11,8 |

(Die Bestimmung des Hb-Wertes kann beispielsweise nach DIN 58931 erfolgen).

Die Verabreichung kann über einen Zeitraum von mehreren Monaten bis zur Verbesserung des Eisenstatus, reflektiert beispielsweise durch den Hämoglobin-Wert, die Transferrin-Sättigung und den Serum Ferritin-Wert der Patienten, oder zur gewünschten Verbesserung einer durch Eisenmangelanämie hervorgerufenen Beeinträchtigung des

Gesundheitszustandes erfolgen.

Das erfindungsgemäße Präparat kann von Kindern, Jugendlichen und Erwachsenen eingenommen werden.

Die erfindungsgemäßen Verbindungen angewendeten Verbindungen können dabei sowohl oral als auch parenteral verabreicht werden. Bevorzugt ist die orale Verabreichung.

Die erfindungsgemäßen Verbindungen sowie die vorgenannten Kombinationen der erfindungsgemäßen Verbindungen mit weiteren Wirkstoffen oder Arzneimitteln können somit insbesondere zur Herstellung von Medikamenten zur Behandlung der Eisenmangelanämie verwendet werden, wie Eisenmangelanämien bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen (z.B. infolge von Geschwüren, Karzinomen, Hämorriden, entzündlichen Störungen, Einnahme von Acetylsalicylsäure), Menstruation, Verletzungen, Eisenmangelanämie infolge von Psilosis (sprue), Eisenmangelanämie infolge verminderter Eisenaufnahme bei der Ernährung, insbesondere bei selektiv essenden Kindern und Heranwachsenden, Immunschwäche hervorgerufen durch Eisenmangelanämie, Beeinträchtigung der Hirnleistung hervorgerufen durch Eisenmangelanämie, Restless Leg Syndrom.

Die erfindungsgemäße Anwendung führt zu einer Verbesserung der Eisen-, Hämoglobin-, Ferritin- und Transferrinwerte, welche insbesondere bei Heranwachsenden und Kindern aber auch bei Erwachsenen mit einer Verbesserung im Kurzzeitgedächtnistests (STM), im Langzeitgedächtnistest (LTM), im Test der progressiven Matrices nach Raven, in der Welschers Erwachsenenintelligenzskala (WAIS) und/oder im emotionalen Koeffizienten (Baron EQ-i, YV-Test; Jugendversion), oder zu einer Verbesserung der Neutrophilen-Niveaus, der Antikörperniveaus und/oder der Lymphozytenfunktion einhergeht.

Die vorliegende Erfindung betrifft weiterhin pharmazeutische Zusammensetzungen, enthaltend eine oder mehrere der erfindungsgemäßen Verbindungen, insbesondere nach Formel (II), sowie gegebenenfalls eine oder mehrere weitere pharmazeutisch wirksame Verbindung sowie gegebenenfalls einen oder mehrere pharmakologisch verträgliche Träger und/oder Hilfsstoffe und/oder Lösungsmittel. Die genannten pharmazeutische Zusammensetzungen enthalten beispielsweise bis 99 Gew.-% oder bis zu 90 Gew.-% oder bis zu 80 Gew.-% oder bis zu 70 Gew.-% der erfindungsgemäßen Verbindungen, wobei der Rest jeweils durch pharmakologisch verträgliche Träger und/oder Hilfsstoffe und/oder Lösungsmittel gebildet wird.

Dabei handelt es sich um übliche pharmazeutische Träger, Hilfsstoff oder Lösungsmittel. Genannte pharmazeutische Zusammensetzungen sind beispielsweise geeignet zur intravenösen, intraperitonealen, intramuskulären, intravaginalen, intrabuccalen, perkutanen, subkutanen, mucokutanen, oralen, rektalen, transdermalen, topikalen, intradermalen, intragastralen oder intrakutanen Applikation und liegen beispielsweise in der Form von Pillen, Tabletten, magensaftresistenten Tabletten, Filmtabletten, Schichttabletten, Retardformulierungen zur oralen, subkutanen oder kutanen Verabreichung (insbesondere als Pflaster), Depotformulierung, Dragees, Zäpfchen, Gelen, Salben, Sirup, Granulaten, Suppositorien, Emulsionen, Dispersionen, Mikrokapseln, Mikroformulierungen, Nanoformulierungen, liposomalen Formulierungen, Kapseln, magensaftresistente Kapseln, Pulvern, Inhalationspulvern, mikrokristallinen Formulierungen, Inhalationssprays, Puder, Tropfen, Nasentropfen, Nasensprays, Aerosolen, Ampullen, Lösungen, Säften, Suspensionen, Infusionslösungen oder Injektionslösungen etc. vor.

In einer bevorzugten Ausführungsform der Erfindung werden die EisenKomplexverbindungen in Form einer Tablette oder Kapsel verabreicht. Diese könnenbeispielsweise als säureresistente Formen oder mit pH-abhängigen Beschichtungen vorliegen.

Bevorzugt werden die erfindungsgemäßen Verbindungen sowie pharmazeutische Zusammensetzungen, enthaltend solche Verbindungen oral appliziert obwohl auch andere Formen wie parenteral, insbesondere intravenös möglich sind.

Dazu liegen die erfindungsgemäßen Verbindungen bevorzugt in pharmazeutischen Zusammensetzungen in Form von Pillen, Tabletten, magensaftresistenten Tabletten, Filmtabletten, Schichttabletten, Retardformulierungen zur oralen Verabreichung, Depotformulierungen, Dragees, Granulaten, Emulsionen, Dispersionen, Mikrokapseln, Mikroformulierungen, Nanoformulierungen, liposomalen Formulierungen, Kapseln, magensaftresistente Kapseln, Pulvern, mikrokristallinen Formulierungen, Puder, Tropfen, Ampullen, Lösungen, Suspensionen, Infusionslösungen oder Injektionslösungen vor.

Die erfindungsgemäßen Verbindungen können in pharmazeutischen Zusammensetzung verabreicht werden, die verschiedene organische oder anorganische Träger- und/oder Hilfsmaterialien enthalten können, wie sie üblicherweise für pharmazeutische Zwecke insbesondere für feste Arzneimittelformulierungen verwendet werden, wie beispielsweise Exzipienten (wie Saccharose, Stärke, Mannit, Sorbit, Lactose, Glucose, Cellulose, Talk, Calciumphosphat, Calciumcarbonat), Bindemittel (wie Cellulose, Methylcellulose, Hydroxypropylcellulose, Polypropylpyrrolidon, Gelatine, Gummiarabicum, Polyethylenglykol, Saccharose, Stärke), Desintegrationsmittel (wie Stärke, hydrolysierte Stärke, Carboxymethylcellulose, Calciumsalz von Carboxymethylcellulose, Hydroxypropylstärke, Natriumglycolstärke, Natriumbicarbonat, Calciumphosphat, Calciumcitrat), Gleit- bzw. Schmiermittel (wie Magnesiumstearat, Talk, Natriumlaurylsulfat), ein Geschmacksbildner (wie Citronensäure, Menthol, Glycin, Orangenpulver), Konservierungsmittel (wie Natriumbenzoat, Natriumbisulfit, Methylparaben, Propylparaben), Stabilisatoren (wie Citronensäure, Natriumcitrat, Essigsäure, und Multicarbonsäuren aus der Titriplex Reihe wie z.B. Diethylentriaminpentaessigsäure (DTPA), Suspendiermittel (wie Methylcellulose, Polyvinylpyrrolidon, Aluminiumstearat), Dispergiermittel, Verdünnungsmittel (wie Wasser, organische Lösungsmittel), Bienenwachs, Kakaobutter, Polyethylenglykol, weißes Petrolatum. Flüssige Arzneimittelformulierungen, wie Lösungen, Suspensionen und Gele enthalten üblicherweise einen flüssigen Träger, wie Wasser und/oder pharmazeutisch verträgliche organische Lösungsmittel. Weiterhin können derartige flüssigen Formulierungen auch pH-einstellende Mittel, Emulgatoren oder dispergierende Agenzien, puffernde Agenzien, Konservierungsmittel, Netzmittel, Geliermittel (beispielsweise Methylcellulose), Färbemittel und/oder Aromastoffe enthalten. Die Zusammensetzungen können isotonisch sein, das heisst diese können den gleichen osmotischen Druck wie Blut haben. Die Isotonie der Zusammensetzung kann durch die Verwendung von Natriumchlorid oder anderer pharmazeutisch annehmbare Agenzien wie beispielsweise Dextrose, Maltose, Borsäure, Natriumtartrat, Propylenglykol oder andere anorganische oder organisch lösliche Substanzen eingestellt werden. Die Viskosität der flüssigen Zusammensetzungen kann unter Verwendung eines pharmazeutisch annehmbaren Verdickungsmittels, wie Methylcellulose eingestellt werden. Andere geeignete Verdickungsmittel umfassen beispielsweise Xanthan, Carboxymethylcellulose, Hydroxypropylcellulose, Carbomer und dergleichen. Die bevorzugte Konzentration des Verdickungsmittels wird von dem ausgewählten Agens abhängen. Pharmazeutisch annehmbare Konservierungsmittel können verwendet werden, um die Haltbarkeit der flüssigen Zusammensetzung zu erhöhen. Benzylalkohol kann geeignet sein, obwohl eine Vielzahl von Konservierungsmitteln einschließlich beispielsweise Paraben, Thimerosal, Chlorbutanol oder Benzalkoniumchlorid ebenfalls verwendet werden können.

Der Wirkstoff kann beispielsweise mit einer Einheitsdosis von 0,001 mg/kg bis 500 mg/kg Körpergewicht beispielweise bis zu 1 bis 4 mal am Tag verabreicht werden. Die Dosierung kann jedoch je nach Alter, Gewicht, Zustand des Patienten, Schwere der Erkrankung oder Art der Verabreichung erhöht oder herabgesetzt werden.

## BEISPIELE

Die Erfindung wird durch die folgenden Beispiele näher veranschaulicht. Die Beispiele stellen lediglich Exemplifizierungen dar, und der Fachmann ist in der Lage, die spezifischen Beispiele auf weitere beanspruchte Verbindungen auszudehnen. Die Bezeichnung der Beispielnamen wurde mit Hilfe des Computerprogrammes ACD/Name Version 12 bestimmt und festgelegt.

## AUSGANGSVERBINDUNGEN:

Die in den Beispielen verwendeten Ausgangsverbindungen wurden wie folgt erhalten.

### A. $N^4$,$N^4$-Dimethyl-2-oxobutandiamid

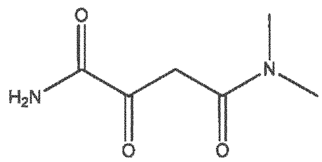

53.30g (0.475mol) Kalium-tert-butoxid wurden in 300ml Diethylether suspendiert. Man gab 73.07g (0.500mol) Diethyloxalat unter Eisbadkühlung zu. Man liess für 30 min gekühlt rühren, gab 43.57g (0.500mol) N,N-Dimethylacetamid zu und rührte die Reaktionsmischung für 2h. Zur Aufarbeitung wurde der Feststoff abfiltriert und in 260ml 4N Salzsäure und 750ml Ethylacetat zersetzt. Nach Trennung der Phasen wurde die wässrige noch zweimal mit 300ml Ethylacetat extrahiert. Die vereinigten organischen Phasen wurden über Natriumsulfat getrocknet und am Rotationsverdampfer zur Trockene eingeengt. Die Ausbeute an Ethyl 4-(dimethylamino)-2,4-dioxobutanoat betrug 54.3g (58%).
IR (in Substanz, $cm^{-1}$): 2984, 2941, 1738, 1620, 1507, 1467, 1393, 1370, 1355, 1313, 1260, 1170, 1126, 1016, 927, 860, 823, 772, 723, 628.
1H-NMR (CDCl3, 400 MHz): $\delta$ [ppm] = 1.38 (3H), 3.06 (6H), 4.33 (2H), 6.25 (1H).

32.56g (163.1 mmol) Kupferacetat monohydrat wurden in 470ml Ethanol suspendiert und auf 75°C erwärmt. 61.06g (326.2mmol) Ethyl 4-(dimethylamino)-2,4-dioxobutanoat wurde in einer Portion zugegeben. Man liess das Gemisch für 40min bei 75°C rühren. Der Kupferkomplex wurde bei 4°C ausgefällt. 32.42g (74.4mmol) des Kupferkomplexes wurden in 530ml methanolischer Ammoniak-Lösung (7N) suspendiert und unter Zugabe von 10ml Ethanol für 4h bei Raumtemperatur gerührt. Anschliessend wurde abfiltriert. Zur Aufarbeitung wurde der Kupferkomplex in 960ml Chloroform suspendiert und bei Raumtemperatur gerührt. Man gab 266ml 10%iger Schwefelsäure zu und liess für 30min rühren. Man trennte die Phasen und extrahierte die wässrige Phase noch zweimal mit 960ml Chloroform. Die vereinten organischen Phasen wurden über Natriumsulfat getrocknet und am Rotationsverdampfer zur Trockene eingeengt. Der Rückstand wurde mit 156ml Ethylacetat aufgekocht und über Nacht bei 4°C auskristallisiert. Das kristalline Produkt wurde abfiltriert und im Vakuum getrocknet. Die Ausbeute an $N,^{4}N^{4}$-Dimethyl-2-oxobutandiamid betrug 15.5g (66%).
IR (in Substanz, $cm^{-1}$): 3392, 3154, 2950, 2799, 1699, 1633, 1600, 1576, 1502, 1427, 1378, 1344, 1253, 1170, 1122, 1097, 1054, 990, 925, 908, 811, 761, 730, 665. 1H-NMR (DMSO-$d_6$, 400 MHz): $\delta$ [ppm] = 3.0 (6H), 6.2 (1H), 7.7 (2H), 15.3 (1H).

**B. 4-(Morpholin-4-yl)-2,4-dioxobutanamid**

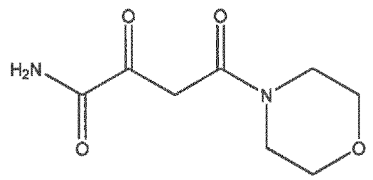

10.2g Diisopropylamin (0.1 mol) wurden in 100ml trockenem THF unter Stickstoff vorgelegt und in einer Kältemischung abgekühlt. Man tropfte 40ml n-Buthyllithium in Hexan (2.5M, 0.1 mol) langsam zu. Nach erfolgter Zugabe liess man 60 min rühren und tropfte anschliessend 12.9 g Acetylmorpholin (0.1mol) zu. Die Reaktionsmischung wurde für weitere 20 min in der Kältemischung gerührt. In einem zweiten Kolben wurden 43.7g Diethyloxalat (0.3mol) in 50 ml trockenem THF in einer Kältemischung abgekühlt und die gekühlte Reaktionsmischung unter Rühren in kleinen Portionen dazu kanüliert. Man liess über Nacht unter Rühren auf Raumtemperatur erwärmen. Das THF wurde am Rotationsverdampfer entfernt und der Rückstand in 80 ml halbkonzentrierter Salzsäure aufgenommen. Man extrahierte die wässrige Phase fünfmal mit je 150ml Ethylacetat, trocknete die vereinten organischen Phasen über Natriumsulfat und engte am Rotationsverdampfer bis zur Trockene ein. Das überschüssige Diethyloxalat wurde abdestilliert (2-3 mbar, 80°C). 5.0 g Ethyl-4-(morpholin-4-yl)-2,4-dioxobutanoat wurden in Form weisser Kristalle mit Schmelzpunkt 60°C nach Kristallisation aus PE erhalten.
1H-NMR (CDCl3, 400 MHz): $\delta$ [ppm] = 1.35 (3H), 3.50-3.73 (8H), 4.33 (2H), 6.20 (1H), 14.32 (1H).

7.68g (38.5mmol) Kupferacetat monohydrat wurden in 110ml Ethanol suspendiert und auf 75°C erwärmt. 17.63g (76.96mmol) Ethyl-4-(morpholin-4-yl)-2,4-dioxobutanoat wurden in einer Portion zugegeben. Man liess das Gemisch für 15min bei 72°C rühren. 19.86g der Kupferkomplexes wurden anschliessend bei 4°C ausgefällt.
15.7g (30.2mmol) des Kupferkomplexes wurden in 240ml methanolischer Ammoniak-Lösung (7N) suspendiert und unter Zugabe von 80ml Ethanol für 4h bei Raumtemperatur gerührt. Anschliessend wurde abfiltriert. Zur Aufarbeitung wurde der Kupferkomplex in 410ml Chloroform suspendiert und bei Raumtemperatur gerührt. Man gab 122ml 10%ige Schwefelsäure zu und liess für 35min rühren. Man trennte die Phasen und extrahierte die wässrige Phase noch zweimal mit 400ml Chloroform. Die vereinten organischen Phasen wurden über Natriumsulfat getrocknet und am Rotationsverdampfer zur Trockene eingeengt. Der Rückstand wurde mit 80ml Ethylacetat aufgekocht und über Nacht bei 4°C auskristallisiert. Das kristalline Produkt wurde abfiltriert und im Vakuum getrocknet. Die Ausbeute an 4-(Morpholin-4-yl)-2,4-dioxobutanamid betrug 5.3g (34%).
IR (in Substanz, $cm^{-1}$): 3453, 3342, 3285, 3180, 2985, 2932, 2873, 1716, 1635, 1576, 1485, 1463, 1442, 1381, 1330, 1303, 1272, 1243, 1195, 1132, 1103, 1066, 1049, 978, 940, 905, 850, 819, 795, 767, 726, 665.
Enolform
1H-NMR (DMSO-$d_6$, 400 MHz): $\delta$ [ppm] = 3.6 (4H), 6.2 (1H), 7.8 (2H), 15.0 (1H).

**C. $N^4$,$N^{4-}$Diethyl-2-oxobutandiamid**

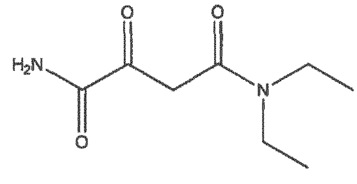

18.5g (0.165mol) Kalium-tert-butoxide wurden in 100ml Diethylether suspendiert. Man gab 25.4g (0.174mol) Diethyloxalat unter Eisbadkühlung zu. Man liess für 30 min gekühlt rühren, gab 20.0g (0.174mol) N,N-Diethylacetamid zu und rührte die Reaktionsmischung bei Raumtemperatur über Nacht. Zur Aufarbeitung wurden 260ml Diethylether zugegeben und der Feststoff abfiltriert. Dieser wurde in 35ml 6N Salzsäure, 174ml Ethylacetat und 20ml Wasser zersetzt. Nach Trennung der Phasen wurde die wässrige noch zweimal mit 100ml Ethylacetat extrahiert. Die vereinten organischen Phasen wurden über Natriumsulfat getrocknet und am Rotationsverdampfer zur Trockene eingeengt. Man kristallisierte das Rohprodukt aus Petrolether/Diethylether bei 4°C aus. Die Ausbeute an Ethyl 4-(diethylamino)-2,4-dioxobutanoat betrug 17.6g (47%).
Enolform
1 H-NMR (CDCl3, 400 MHz): δ [ppm] = 1.2 (6H), 1.4 (3H), 3.4 (4H), 4.4 (2H), 6.2 (1H) 8.16g (40.9mmol) Kupferacetat monohydrat wurden in 100ml Ethanol suspendiert und auf 73°C erwärmt. 17.6g (81.7mmol) Ethyl 4-(diethylamino)-2,4-dioxobutanoat wurden in einer Portion zugegeben. Man liess das Gemisch für 40min bei 72°C rühren. Anschliessend gab man 50ml Toluol zu und entfernte das Lösungsmittel am Rotationsverdampfer. Der Rückstand wurde noch zweimal mit 50ml Toluol evaporiert. 19.9g der Kupferkomplexes wurden als Rohprodukt erhalten und ohne weitere Reinigung weiter verwendet.

9.0g (18mmol) des Kupferkomplexes wurden in 25ml methanolischer Ammoniak-Lösung (7N) gelöst und für 4h bei Raumtemperatur gerührt. Anschliessend wurde das Lösungsmittel am Rotationsverdampfer vollständig entfernt. Zur Aufarbeitung wurde der Kupferkomplex in 190ml Dichlormethan suspendiert und bei Raumtemperatur gerührt. Man gab 53ml 10%ige Schwefelsäure zu und liess für 5min rühren. Man trennte die Phasen und extrahierte die wässrige Phase noch zweimal mit 90ml Dichlormethan. Die vereinten organischen Phasen wurden über Natriumsulfat getrocknet und am Rotationsverdampfer zur Trockene eingeengt. Der Rückstand wurde säulenchromatographisch aufgereinigt (Kieselgel, Ethylacetat). Die Ausbeute an $N^4$,$N^4$-Diethyl-2-oxobutandiamid betrug 6.3g (94%).
IR (in Substanz, $cm^{-1}$): 3371, 3193, 2974, 2935, 2875, 2324, 2051, 1982, 1787, 1741, 1683, 1635, 1586, 1495, 1459, 1400, 1380, 1362, 1308, 1269, 1239, 1218, 1158, 1130, 1098, 1081, 1048, 960, 896, 827, 782.
Enolform
1H-NMR (DMSO-$d_6$, 400 MHz): δ [ppm] = 1.1 (6H), 3.4 (4H), 6.1 (1H), 7.8 (2H), 15.4 (1H).

**D. 2,4-Dioxo-4-(pyrrolidin-1-yl)butanamid**

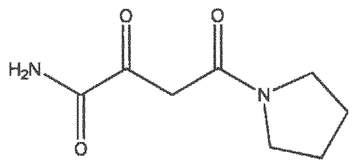

8.33g (74.4mmol) Kalium-tert-butoxide wurden in 47ml Diethylether suspendiert. Man gab 11.4g (78.3mmol) Diethyloxalat unter Eisbadkühlung zu. Man liess für 60 min gekühlt rühren, gab 8.86g (78.3mol) N-Acetylpyrrolidin zu und rührte die Reaktionsmischung bei Raumtemperatur für drei Stunden. Die Suspension wurde abfiltriert. Der Feststoff wurde in 25ml 6N Salzsäure, 120ml Ethylacetat und 15ml Wasser zersetzt. Nach Trennung der Phasen wurde die wässrige noch zweimal mit 50ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Petrolether/Ethylacetat 2/1). Die Ausbeute an Ethyl 2,4-dioxo-4-(pyrrolidin-1-yl)butanoat betrug 7.7g (46%).
Enolform
1H-NMR (DMSO-$d_6$, 400 MHz): δ [ppm] = 1.3 (3H), 1.9 (4H), 3.4 (2H), 3.5 (2H), 4.3 (2H), 6.1 (1H), 14.9 (1H).

3.59g (18.0mmol) Kupferacetat monohydrat wurden in 10ml Ethanol suspendiert und auf 73°C erwärmt. 7.66g (35.9mmol) Ethyl 2,4-dioxo-4-(pyrrolidin-1-yl)butanoat wurden in einer Portion zugegeben. Man liess das Gemisch für 30min bei 72°C rühren. Anschliessend gab man 20ml Ethanol und 10ml Wasser zu und liess weitere 20 Minuten rühren. Es wurde heiss filtriert und die Lösung bei 4°C über Nacht gelagert. 4.4g der Kupferkomplexes wurden in kristalliner Form erhalten und ohne weitere Reinigung weiter verwendet.

4.4g (9.0mmol) des Kupferkomplexes wurden in 64ml methanolischer Ammoniak-Lösung (7N) gelöst und für 4h bei Raumtemperatur gerührt. Die Suspension wurde filtriert und der Feststoff für 3 Tage bei 50°C im Feinvakuum getrocknet. Zur Aufarbeitung wurde der Kupferkomplex in 100ml Dichlormethan suspendiert und 53ml 10%ige Schwefelsäure zugegeben. Man liess solange rühren, bis zwei klare Phasen entstanden waren. Man trennte die Phasen und extrahierte die wässrige Phase noch zweimal mit 100ml Dichlormethan. Die vereinten organischen Phasen wurden über

Natriumsulfat getrocknet und am Rotationsverdampfer zur Trockene eingeengt. Der Rückstand wurde aus heissem Ethylacetat kristallisiert. Die Ausbeute an 2,4-Dioxo-4-(pyrrolidin-1-yl) butanamid betrug 1.7g (51%).
IR (in Substanz, $cm^{-1}$): 3444, 3293, 3146, 2976, 2890, 1688, 1628, 1586, 1482, 1460, 1399, 1343, 1301, 1230, 1187, 1164, 1128, 1107, 1052, 1019, 970, 913, 852, 819, 748.
Enolform
1H-NMR (DMSO-$d_6$, 400 MHz): $\delta$ [ppm] = 1.9 (4H), 3.4 (4H), 6.0 (1H), 7.8 (2H), 15.3 (1H).

**E. N,N,N',N'-Tetramethyl-2-oxobutandiamid**

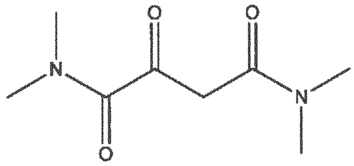

18.65g (166.3mmol) Kalium-tert-butoxide wurden in 170ml Diethylether suspendiert. Man gab 25.58g (175.0mmol) Ethyl N,N-dimethyloxamate unter Eisbadkühlung zu. Man liess für 30 min bei Raumtemperatur rühren, gab 15.25g (175.0mmol) N,N-Dimethylacetamid zu und rührte die Reaktionsmischung bei Raumtemperatur für zwei Stunden. Die Reaktionslösung wurde mit 90ml 6N Salzsäure, 420ml Ethylacetat und 15ml Wasser versetzt und fünf Minuten gerührt. Nach Trennung der Phasen wurde die wässrige noch zweimal mit 200ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Dichlormethan/Aceton 5/1). Die Ausbeute an N,N,N',N'-Tetramethyl-2-oxobutandiamid betrug 5.3g (16%).
IR (in Substanz, $cm^{-1}$): 2936, 1720, 1635, 1496, 1456, 1397, 1352, 1259, 1237, 1203, 1130, 1107, 1077, 951, 892, 849, 807, 774, 753, 720.
Ketoform
1H-NMR (DMSO-$d_6$, 400 MHz): $\delta$ [ppm] = 2.8-3.0 (12H), 3.9 (2H).

**F. 4-(4-Hydroxypiperidin-1-yl)-2,4-dioxobutanamid**

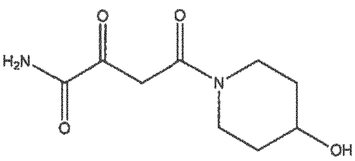

120g (1.19mol) 4-Hydroxypyridin wurden in 1700ml Dichlormethan gelöst und 132g (1.03mol) Triethylamin zugetropft. Man kühlte die Reaktionsmischung auf -40°C und tropfte 93.4g (1.19mol) Acetylchlorid zu. Anschliessend wurde für eine Stunde bei Raumtemperatur gerührt. Die Suspension wurde filtriert und das Filtrat am Rotationsverdampfer zur Trockene eingeengt. Der Rückstand wurde in 1700ml Ethylacetat aufgenommen und nochmal filtriert. Das Filtrat wurde zur Trockene eingeengt. Als Produkt wurden 120g (71%) 1-(4-Hydroxypiperidin-1-yl)ethanon erhalten. 1H-NMR ($CDCl_3$, 400 MHz): $\delta$ [ppm] = 1.5 (2H), 1.8 (2H), 2.1 (3H), 2.7 (1H), 3.2 (2H), 3.7 (1H), 3.9 (1H), 4.1 (1H).
120g (839mmol) 1-(4-Hydroxypiperidin-1-yl)ethanon wurden in 1.51 Dichlormethan suspendiert und in einem Eisbad gekühlt. 85.9g (839mmol) Triethylamin wurden zugetropft und für 10 Minuten gerührt. Anschliessend wurden 101g (839mmol) Trimethylacetylchlorid zugegeben und die Reaktionsmischung für drei Tage bei Raumtemperatur gerührt. Die Reaktionsmischung wurde auf 200ml eingeengt und filtriert. Das Filtrat wurde anschliessend zur Trockene eingeengt. Der Rückstand wurde in 200ml Ethylacetat aufgenommen und 30 Minuten gerührt. Man filtrierte ab und engte das Filtrat am Rotationsverdampfer zur Trockene ein. Zur Aufreinigung wurde das Produkt aus n-Heptan kristallisiert. Man erhielt 56g (29%) 1-Acetylpiperidin-4-yl-2,2-dimethylpropanoat in Form weisser Kristalle.
1H-NMR ($CDCl_3$, 400 MHz): $\delta$ [ppm] = 1.2 (9H), 1.6 (2H), 1.8 (2H), 2.1 (3H), 3.4 (1H), 3.6 (2H), 3.7 (1H), 5.0 (1H).
9.88g (88.0mmol) Kalium-tert-butoxide wurden in 80ml Diethylether suspendiert und 12.9g (88.0mmol) Diethyloxalat zugegeben. 20.0g (88.0mmol) 1-Acetylpiperidin-4-yl-2,2-dimethylpropanoat wurden in 80ml Diethylether gelöst

und zur Reaktionsmischung getropft. Man liess für 20 min rühren und anschliessend über Nacht ungerührt stehen. Zur Aufarbeitung wurden 160ml Petrolether zugegeben und die Suspension abfiltriert. Der Rückstand wurde in 60ml 1N HCl aufgenommen und mit Natronlauge auf pH 7 eingestellt. Anschliessend extrahierte man dreimal mit 200ml Ethylacetat, trocknete über Natriumsulfat und engte am Rotationsverdampfer zur Trockene ein. Man erhielt 15.3g (53%) Ethyl-4-{4-[(2,2-dimethylpropanoyl)oxy]piperidin-1-yl}-2,4-dioxobutanoat. Dieses wurde ohne weitere Reinigung weiter verarbeitet.

Enolform

1H-NMR (DMSO-$d_6$, 400 MHz): $\delta$ [ppm] = 1.2 (9H), 1.4 (3H), 1.7 (2H), 1.9 (2H), 3.5-3.8(4H), 4.3 (2H), 5.0 (1H), 6.3 (1H), 14.5 (1H).

189g (577mmol) ,4Ethyl-4-{4-[(2,2-dimethylpropanoyl)oxy]piperidin-1-yl}-2-dioxobutanoat wurden in 900ml trockenem Ethanol gelöst und auf 50°C erwärmt. Man tropfte 393g 21%ige Natriumethanolatlösung (1.21mol) zu und liess bei 50°C über Nacht rühren. Die Reaktionslösung wurde am Rotationsverdampfer zur Trockene eingeengt und der Rückstand in 2l 1N Salzsäure aufgenommen. Man extrahierte dreimal mit 2l Ethylacetat, trocknete über Natriumsulfat und engte zur Trockene ein. Das Rohprodukt wurde säulenchromatographisch aufgereinigt (Kieselgel, Aceton/Dichlormethan 1/1). Man erhielt 74.9g (53%) Ethyl 4-(4-hydroxypiperidin-1-yl)-2,4-dioxobutanoat.

Enolform

1H-NMR (DMSO-$d_6$, 400 MHz): $\delta$ [ppm] = 1.3 (3H), 1.4 (2H), 1.8 (2H), 3.3 (2H), 3.7 (1H), 3.8 (2H), 4.2 (2H), 4.8 (1H), 6.3 (1H), 14.9 (1H).

8.78g (44.0mmol) Kupferacetat monohydrat wurden in 125ml Ethanol suspendiert und auf Siedetemperatur erwärmt. 21.3g (88.0mmol) Ethyl 4-(4-hydroxypiperidin-1-yl)-2,4-dioxobutanoat wurden portionsweise zugegeben. Man liess das Gemisch für 40min unter Rückfluss kochen. Es wurde filtriert und die Lösung bei 2.2°C über Nacht gelagert. 13.2g der Kupferkomplexes wurden in kristalliner Form erhalten und ohne weitere Reinigung weiter verwendet.

7.18g (13.8mmol) des Kupferkomplexes wurden in 98ml methanolischer Ammoniak-Lösung (7N) gelöst und für 4h bei Raumtemperatur gerührt. Die Suspension wurde filtriert und der Feststoff bei 50°C im Feinvakuum getrocknet. Zur Aufarbeitung wurde der Kupferkomplex in 125ml Methanol suspendiert und für 30min $H_2S$ durch die Suspension geleitet. Man liess solange rühren, bis zwei klare Phasen entstanden waren. Man filtrierte zweimal über Celite® und engte das Filtrat am Rotationsverdampfer ein. Der Rückstand wurde in Ethylacetat umkristallisiert. Die Ausbeute an 4-(4-Hydroxypiperidin-1-yl)-2,4-dioxobutanamid betrug 0.5g (8%).

IR (in Substanz, $cm^{-1}$): 3282, 3118, 2981, 2324, 2164, 2051, 1981, 1797, 1546, 1413, 1349, 1264, 1161, 1138, 1086, 1043, 962, 925, 899, 821, 786, 728, 676.

Enolform

1H-NMR (DMSO-$d_6$, 400 MHz): $\delta$ [ppm] = 1.3 (2H), 1.7 (2H), 3.3 (2H), 3.7-3.9 (3H), 4.8 (1H), 6.2 (1H), 7.7 (2H), 15.3 (1H).

**G. N',$N^4$,$N^4$-Trimethyl-2-oxobutandiamid**

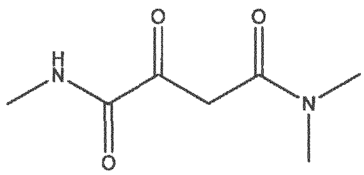

53.30g (0.475mol) Kalium-tert-butoxide wurden in 300ml Diethylether suspendiert. Man gab 73.07g (0.500mol) Diethyloxalat unter Eisbadkühlung zu. Man liess für 30 min gekühlt rühren, gab 43.57g (0.500mol) N,N-Dimethylacetamid zu und rührte die Reaktionsmischung für 2h. Zur Aufarbeitung wurden abfiltrierte und der Feststoff in 260ml 4N Salzsäure und 750ml Ethylacetat zersetzt. Nach Trennung der Phasen wurde die wässrige noch zweimal mit 300ml Ethylacetat extrahiert. Die vereinten organischen Phasen wurden über Natriumsulfat getrocknet und am Rotationsverdampfer zur Trockene eingeengt. Die Ausbeute an Ethyl 4-(dimethylamino)-2,4-dioxobutanoat betrug 54.3g (58%).

1H-NMR (CDCl3, 400 MHz): $\delta$ [ppm] = 1.38 (3H), 3.06 (6H), 4.33 (2H), 6.25 (1H) 32.56g (163.1 mmol) Kupferacetat monohydrat wurden in 470ml Ethanol suspendiert und auf 75°C erwärmt. 61.06g (326.2mmol) Ethyl 4-(dimethylamino)-2,4-dioxobutanoat wurden in einer Portion zugegeben. Man liess das Gemisch für 40min bei 75°C rühren. Der Kupferkomplex wurde bei 4°C ausgefällt. 10.0g (23.0mmol) des Kupferkomplexes wurden in 57ml ethanolischer Methylaminlösung (33%ig) suspendiert und für 2h bei Raumtemperatur gerührt. Man engte die Reaktionsmischung zur Trockene ein. Zur Aufarbeitung wurde der Kupferkomplex in 100ml Chloroform gelöst. Man gab 170ml 10%iger Schwefelsäure zu und rührte die beiden Phasen gut durch. Man trennte die Phasen und extrahierte die wässrige Phase noch zweimal mit 50ml Chloroform. Die vereinten organischen Phasen wurden über Natriumsulfat getrocknet und am Rotationsverdampfer zur Trockene eingeengt. Die Ausbeute an $N^1,N^4,N^4$-Trimethyl-2-oxobutandiamid betrug 4.1g (52%). IR (in Substanz, $cm^{-1}$): 3398, 3315, 3105, 2931, 2324, 1783, 1671, 1632, 1599, 1526, 1500, 1405, 1361, 1347, 1252, 1160, 1063, 1018, 935, 907, 838, 776, 726.
Enolform:

1H-NMR (DMSO-$d_6$, 400 MHz): δ [ppm] = 2.7 (3H), 3.0 (6H), 6.1 (1H), 8.4 (1H), 15.5 (1H).

**H. N,N'-Dimethyl-2-oxobutandiamid**

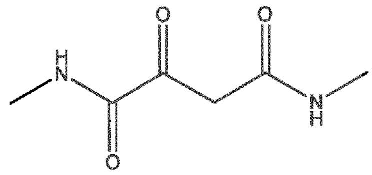

38.2g (0.556mol) Methylamin hydrochlorid wurden in 70ml Dichlormethan suspendiert und 57.3g (0.556mol) Triethylamin zugegeben. Man kühlte die Reaktionsmischung auf - 60°C und tropfte 108g (0.556mol) (2Z)-(2,2-Dimethyl-5-oxo-1,3-dioxolan-4-yliden)acetyl chlorid (hergestellt nach J. Banville et al, Tetrahedron Letters, 2010, 51, 3170 - 3173), gelöst in 70ml Dichlormethan, zu. Anschliessend rührte man über Nacht bei Raumtemperatur und kochte abschliessend für vier Stunden unter Rückfluss. Die Reaktionsmischung wurde zur Trockene eingeengt und der Rückstand in 400ml Ethylacetat aufgenommen. Die Suspension wurde filtriert und das Filtrat zur Trockene eingeengt. Der Rückstand wurde in 600ml Diethylether aufgenommen. Die Suspension wurde filtriert und das Filtrat zur Trockene eingeengt. Man erhielt 49.1g (47%) (2Z)-(2,2-dimethyl-5-oxo-1,3-dioxolan-4-yliden)-N-methylacetamid.
1H-NMR (DMSO-$d_6$, 400 MHz): δ [ppm] = 1.7 (6H), 2.6 (3H), 5.8 (1H), 8.0 (1H).

13.1g (66.0mmol) Kupferacetat monohydrat wurden in 600ml Ethanol suspendiert und 25.7g (132mmol) (2Z)-(2,2-dimethyl-5-oxo-1,3-dioxolan-4-yliden)-N-methylacetamid zugegeben. Man liess das Gemisch für vier Stunden unter Rückfluss kochen. Die Suspension wurde zur Trockene eingeengt und der Rückstand zur Entfernung der Essigsäure dreimal mit Toluol abgeschleppt. 10.6g (25.9mmol) des Kupferkomplexes wurden in 31ml ethanolischer Methylaminlösung (33%ig) suspendiert und für 2 Stunden bei Raumtemperatur gerührt. Man engte die Reaktionsmischung zur Trockene ein. Zur Aufarbeitung wurde der Kupferkomplex in 200ml Dichlormethan gelöst. Man gab 60ml 10%ige Schwefelsäure und 30ml Wasser zu und rührte die beiden Phasen gut durch. Man trennte die Phasen und extrahierte die wässrige Phase noch zweimal mit 100ml Dichlormethan. Die vereinten organischen Phasen wurden über Natriumsulfat getrocknet und am Rotationsverdampfer zur Trockene eingeengt. Das Produkt wurde aus heissem Ethylacetat kristallisiert. Die Ausbeute an N,N'-Dimethyl-2-oxobutandiamid betrug 0.4g (5%).
IR (in Substanz, $cm^{-1}$): 3367, 3310, 3133, 2942, 2051, 1624, 1584, 1532, 1396, 1275, 1247, 1133, 1078, 946, 838, 776, 752, 674.
Enolform:

1H-NMR (DMSO-$d_6$, 400 MHz): δ [ppm] = 2.7 (6H), 5.9 (1H), 8.4 (2H), 14.3 (1H).

**I. $N^1,N^1,N^4$-Trimethyl-2-oxobutandiamid**

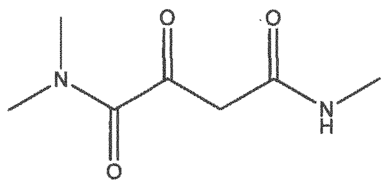

38.2g (0.556mol) Methylamin hydrochlorid wurden in 70ml Dichlormethan suspendiert und 57.3g (0.556mol) Triethylamin zugegeben. Man kühlte die Reaktionsmischung auf - 60°C und tropfte 108g (0.556mol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) gelöst in 70ml Dichlormethan zu. Anschliessend rührte man über Nacht bei Raumtemperatur und kochte abschliessend

für vier Stunden unter Rückfluss. Die Reaktionsmischung wurde zur Trockene eingeengt und der Rückstand in 400ml Ethylacetat aufgenommen. Die Suspension wurde filtriert und das Filtrat zur Trockene eingeengt. Der Rückstand wurde in 600ml Diethylether aufgenommen. Die Suspension wurde filtriert und das Filtrat zur Trockene eingeengt. Man erhielt 49.1g (47%) (2Z)-(2,2-Dimethyl-5-oxo-1,3-dioxolan-4-yliden)-N-methylacetamid.
1H-NMR (DMSO-$d_6$, 400 MHz): $\delta$ [ppm] = 1.7 (6H), 2.6 (3H), 5.8 (1H), 8.0 (1H).

1.0g (5.4mmol) (2Z)-(2,2-dimethyl-5-oxo-1,3-dioxolan-4-yliden)-N-methylacetamid wurden in 9.6ml ethanolischer Dimethylaminlösung (33%ig) gelöst und fünf Minuten bei Raumtemperatur gerührt. Man gab 6ml 6N Salzsäure zu und rührte nochmals fünf Minuten. Die Reaktionsmischung wurde zur Trockene eingeengt und der Rückstand in 15ml Wasser aufgenommen. Man extrahierte die wässrige Phase zweimal mit 50ml Ethylacetat. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Ethylacetat). Die Ausbeute an $N^1$,$N^1$,$N^4$-Trimethyl-2-oxobutandiamid betrug 280mg (30%).
IR (in Substanz, $cm^{-1}$): 3303, 3099, 2941, 1766, 1719, 1624, 1561, 1502, 1449, 1405, 1338, 1255, 1227, 1160, 1079, 1041, 947, 910, 829, 773, 657.
Ketoform:

1H-NMR (DMSO-$d_6$, 400 MHz): $\delta$ [ppm] = 2.6 (3H), 2.9 (6H), 3.6 (2H), 8.1 (1H).

**J. $N^4$,$N^4$-Diethyl-$N^1$,$N^1$-dimethyl-2-oxobutandiamid**

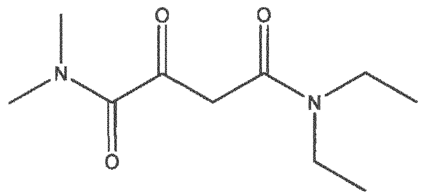

8.0g (71 mmol) Kalium-tert-butoxide wurden in 60ml Diethylether suspendiert. Man gab 10.9g (75.0mmol) Ethyl N,N-dimethyloxamat unter Eisbadkühlung zu. Man liess für 30 min bei Raumtemperatur rühren, gab 8.6g (75mmol) N,N-Diethylacetamid zu und rührte die Reaktionsmischung bei Raumtemperatur für zwei Stunden. Die Reaktionslösung wurde mit 45ml 6N Salzsäure, und 200ml Ethylacetat versetzt. Nach Trennung der Phasen wurde die wässrige noch zweimal mit 50ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Petrolether/Ethylacetat). Die Ausbeute an $N^4$,$N^4$-Diethyl-$N^1$,$N^1$-dimethyl-2-oxobutandiamid betrug 3.5g (22%).
IR (in Substanz, $cm^{-1}$):3496, 2975, 2936, 1721, 1626, 1487, 1448, 1400, 1380, 1362, 1308, 1271, 1217, 1200, 1141, 1100, 1077, 954, 927, 789, 771, 734.
Ketoform
1H-NMR (DMSO-$d_6$, 400 MHz): $\delta$ [ppm] = 1.1 (6H), 3.0 (6H), 3.3 (4H), 3.9 (2H).

**K. N,N-Dimethyl-3,4-dioxo-4-(pyrrolidin-1-yl)butanamid**

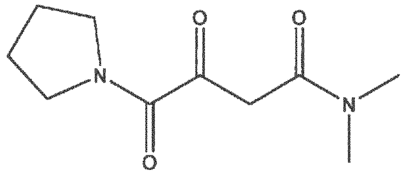

19.2g (270mmol) Pyrrolidin und 27.3g (270mmol) Triethylamin wurden in 400ml Diethylether gelöst und im Eisbad abgekühlt. Man gab tropfenweise 36.9g (270mmol) Ethyl chlorooxoacetat zu und liess auf Raumtemperatur erwärmen. Die Suspension wurde filtriert und das Filtrat am Rotationsverdampfer eingeengt. Man erhielt 46.6g Ethyloxo(pyrrolidin-1-yl)acetat in Form eines gelben Öls, welches ohne Reinigung weiter verarbeitet wurde.

27.2g (242mmol) Kalium-tert-butoxide wurden in 200ml Diethylether suspendiert. Man gab 43.6g (255mmol)

Ethyl-oxo(pyrrolidin-1-yl)acetat unter Eisbadkühlung zu. Man liess für 30 min bei Raumtemperatur rühren, gab 22.2g (255mmol) N,N-Dimethylacetamid zu und rührte die Reaktionsmischung bei Raumtemperatur für zwei Stunden. Die Reaktionslösung wurde mit 136ml 6N Salzsäure, 170ml Ethylacetat und 70ml Wasser versetzt. Nach Trennung der Phasen wurde die wässrige noch zweimal mit 100ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Petrolether/Ethylacetat). Die Ausbeute an N,N-Dimethyl-3,4-dioxo-4-(pyrrolidin-1-yl)butanamid betrug 37.1g (69%).
IR (in Substanz, $cm^{-1}$): 2952, 2880, 1722, 1632, 1602, 1503, 1445, 1415, 1354, 1338, 1253, 1224, 1163, 1141, 1060, 1033, 975, 952, 915, 890, 870, 848, 812, 765, 728, 695. Enolform
1H-NMR (DMSO-$d_6$, 400 MHz): $\delta$ [ppm] = 1.8 (4H), 3.0 (6H), 3.4 (2H), 3.6 (2H), 5.9 (1H), 15.6 (1H).

**L. $N^4$-[2-(Dimethylamino)-2-oxoethyl]-$N^1$,$N^1$,$N^4$-trimethyl-2-oxobutandiamid**

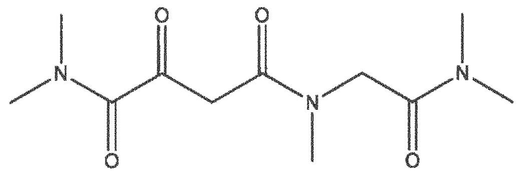

17.1g (0.112mol) N,N-Dimethyl-2-(methylamino)acetamid hydrochlorid (*Sigma-Aldrich: CDS007544)* wurden in 300ml Dichlormethan suspendiert und 22.6g (0.224mol) Triethylamin zugegeben. Man gab 21.3g (0.112mol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) portionsweise zu. Anschliessend rührte man 30 Minuten bei Raumtemperatur und kochte für vier Stunden unter Rückfluss. Die Suspension wurde zur Trockene eingeengt und der Rückstand in 300ml Ethylacetat aufgenommen. Die Suspension wurde filtriert und das Filtrat zur Trockene eingeengt. Man erhielt 9.8g (32%)$N^2$-[(2Z)-2-(2,2-Dimethyl-5-oxo-1,3-dioxoian-4-yliden)acetyl]-N,N,$N^2$-trimethylglycinamid.
1H-NMR (DMSO-$d_6$, 400 MHz): $\delta$ [ppm] = 1.7 (6H), 2.8-3.0 (9H), 4.2-4.3 (2H), 5.8-6.1 (1H).
8.8g (32mmol) $N^2$-[(2Z)-2-(2,2-Dimethyl-5-oxo-1,3-dioxolan-4-yliden)acetyl]-N,N,$N^2$-trimethylglycinamid wurden in 58ml ethanolischer Dimethylaminlösung (33%ig) gelöst und 30 Minuten bei Raumtemperatur gerührt. Die Reaktionsmischung wurde zur Trockene eingeengt. Das Rohprodukt wurde säulenchromatographisch aufgereinigt (Kieselgel, Dichlormethan:Methanol 20:1). Die Ausbeute an $N^4$-[2-(Dimethylamino)-2-oxoethyl]-$N^1$,$N^1$,$N^4$-trimethyl-2-oxobutandiamid betrug 3.0g (36%).
IR (in Substanz, $cm^{-1}$): 3492, 2936, 1720, 1631, 1492, 1398, 1364, 1332, 1261, 1239, 1217, 1139, 1106, 1078, 951, 898, 860, 812, 762, 720, 675.
Ketoform:

1H-NMR (DMSO-$d_6$, 400 MHz): $\delta$ [ppm] = 2.7-3.0 (15H), 3.7-4.0 (2H), 4.1-4.3 (2H).

**M. $N^4$-(2-Methoxyethyl)-$N^1$,$N^1$,$N^4$-trimethyl-2-oxobutandiamid**

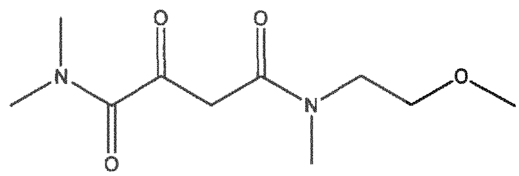

9.09g (102mmol) (2-Methoxyethyl)methylamin und 10.3g (102mmol) Triethylamin wurden in 110ml Diethylether gelöst und im Eisbad abgekühlt. Man gab tropfenweise 8.01g (102mmol) Acetylchlorid zu und liess auf Raumtemperatur erwärmen. Die Suspension wurde filtriert und das Filtrat am Rotationsverdampfer eingeengt. Man erhielt 10g N-(2-Methoxyethyl)-N-methylacetamid in Form eines farblosen Öls, welches ohne Reinigung weiter verarbeitet wurde. 8.08g (72.0mmol) Kalium-tert-butoxide wurden in 100ml Diethylether suspendiert. Man gab 10.5g (72.0mmol) Ethyl N,N-dimethyloxamat unter Eisbadkühlung zu. Man liess für 15 min bei Raumtemperatur rühren, gab 9.44g (72.0mmol) N-(2-Methoxyethyl)-N-methylacetamid zu und rührte die Reaktionsmischung bei Raumtemperatur für zwei Stunden. Die Reaktionslösung wurde mit 140ml 6N Salzsäure, 180ml Ethylacetat und 45ml Wasser versetzt. Nach Trennung der Phasen wurde die wässrige noch dreimal mit 80ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Petrolether/Ethylacetat). Die Ausbeute an $N^4$-(2-Methoxyethyl)-$N^1$,$N^1$,$N^4$-trimethyl-2-oxobutandiamid betrug 2.98g (18%).
IR (in Substanz, $cm^{-1}$): 2935, 1612, 1498, 1458, 1427, 1362, 1260, 1193, 1153, 1114, 1062, 1022, 950, 842, 814, 787, 724, 694, 674.
Ketoform
1H-NMR (DMSO-$d_6$, 400 MHz): δ [ppm] = 2.8-3.0 (9H), 3.2-3.3 (3H), 3.4-3.6 (4H), 3.9-4.0 (2H).

**N. $N^4$-(2-Methoxyethyl)-$N^1$,$N^1$-dimethyl-2-oxobutandiamid**

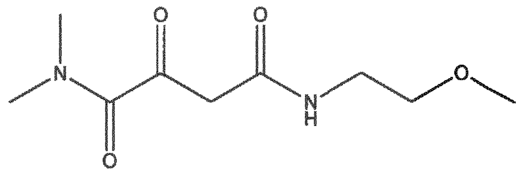

7.1g (94mmol) 2-Methoxyethylamin wurden in 300ml Dichlormethan suspendiert und 19.0g (188mmol) Triethylamin zugegeben. Man gab 18.0g (94mmol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) gelöst in 70ml Dichlormethan zu. Anschliessend rührte man 30 Minuten bei Raumtemperatur und kochte für vier Stunden unter Rückfluss. Die Suspension wurde zur Trockene eingeengt und der Rückstand in 300ml Ethylacetat aufgenommen. Die Suspension wurde filtriert und das Filtrat zur Trockene eingeengt. Der Rückstand wurde in 350ml Diethylether aufgenommen und gerührt. Die Suspension wurde filtriert und das Filtrat zur Trockene eingeengt. Man erhielt 15.4g (71%) (2Z)-2-(2,2-Dimethyl-5-oxo-1,3-dioxolan-4-yliden)-N-(2-methoxyethyl)acetamid. 1H-NMR (DMSO-$d_6$, 400 MHz): δ [ppm] = 1.7 (6H), 3.3 (3H), 3.3-3.4 (4H), 5.9 (1H), 8.2 (1H).
3.0g (13mmol) (2Z)-2-(2,2-Dimethyl-5-oxo-1,3-dioxolan-4-yliden) -N-(2-methoxyethyl)acetamid wurden in 23ml ethanolischer Dimethylaminlösung (33%ig) gelöst und 5 Minuten bei Raumtemperatur gerührt. Man gab 18ml 6N HCl-Lösung zu, rührte für 5 Minuten und engte die Reaktionsmischung zur Trockene ein. Die Ausbeute $N^4$-(2-Methoxyethyl)-$N^1$,$N^1$-dimethyl-2-oxobutandiamid betrug 2.3g (82%).
IR (in Substanz, $cm^{-1}$): 3307, 3086, 2932, 2884, 1720, 1634, 1548, 1503, 1452, 1401, 1324, 1266, 1195, 1121, 1084, 1041, 948, 819, 772, 718.
Ketoform:

1H-NMR (DMSO-$d_6$, 400 MHz): δ [ppm] = 2.9 (6H), 3.3 (3H), 3.2-3.4 (4H), 3.6 (2H), 8.2 (1H).

**O. Ethyl-N-[4-(dimethylamino)-3,4-dioxobutanoyl]-N-methylglycinat**

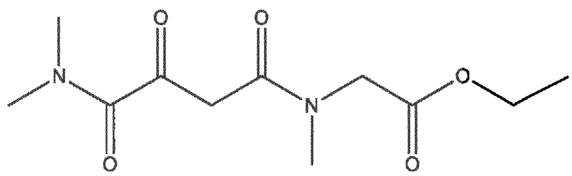

15.0g (97.7mmol) Sarkosin ethylester hydrochlorid wurden in 300ml Dichlormethan suspendiert und 19.8g (195mmol) Triethylamin zugegeben. Man gab 18.8g (98.7mmol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) gelöst in 70ml Dichlormethan zu. Anschliessend rührte man 30 Minuten bei Raumtemperatur und kochte anschliessend für vier Stunden unter Rückfluss. Die Suspension wurde zur Trockene eingeengt und der Rückstand in 300ml Ethylacetat aufgenommen. Die Suspension wurde filtriert und das Filtrat zur Trockene eingeengt. Man erhielt 12g (45%) Ethyl-N-[(2Z)-2-(2,2-dimethyl-5-oxo-1,3-dioxolan-4-yliden)acetyl]-N-methylglycinat.
1H-NMR (DMSO-$d_6$, 400 MHz): δ [ppm] = 1.2 (3H), 1.8 (6H), 3.1 (3H), 4.1 (2H), 4.1-4.3 (2H).

3.0g (11mmol) Ethyl-N-[(2Z)-2-(2,2-dimethyl-5-oxo-1,3-dioxolan-4-yliden)acetyl]-N-methylglycinat wurden in 20ml ethanolischer Dimethylaminlösung (33%ig) gelöst und 5 Minuten bei Raumtemperatur gerührt. Man gab 18ml 6N HCl-Lösung zu, rührte für 5 Minuten und engte die Reaktionsmischung zur Trockene ein. Der Rückstand wurde in 50ml Wasser aufgenommen und dreimal mit 150ml Ethylacetat extrahiert. Die vereinten organischen Phasen wurden vereint und über Natriumsulfat getrocknet. Die Ausbeute Ethyl-N-[4-(dimethylamino)-3,4-dioxobutanoyl]-N-methylglycinat betrug 2.3g (81%).
IR (in Substanz, $cm^{-1}$): 2938, 1743, 1638, 1489, 1399, 1374, 1354, 1197, 1107, 1077, 1031, 950, 900, 851, 811, 765, 717.
Ketoform:

1H-NMR (DMSO-$d_6$, 400 MHz): δ [ppm] = 1.2 (3H), 2.8-3.1 (9H), 3.8-4.0 (2H), 4.1 (2H), 4.1-4.3 (2H).

### P. $N^1$-(2-Methoxyethyl)-$N^4$,$N^4$-dimethyl-2-oxobutanamid

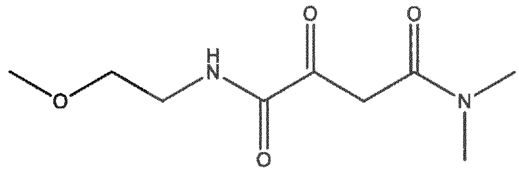

53.30g (0.475mol) Kalium-tert-butoxide wurden in 300ml Diethylether suspendiert. Man gab 73.07g (0.500mol) Diethyloxalat unter Eisbadkühlung zu. Man liess für 30 min gekühlt rühren, gab 43.57g (0.500mol) N,N-Dimethylacetamid zu und rührte die Reaktionsmischung für 2h. Zur Aufarbeitung wurden abfiltrierte und der Feststoff in 260ml 4N Salzsäure und 750ml Ethylacetat zersetzt. Nach Trennung der Phasen wurde die wässrige noch zweimal mit 300ml Ethylacetat extrahiert. Die vereinten organischen Phasen wurden über Natriumsulfat getrocknet und am Rotationsverdampfer zur Trockene eingeengt. Die Ausbeute an Ethyl 4-(dimethylamino)-2,4-dioxobutanoat betrug 54.3g (58%).

1H-NMR (CDCl3, 400 MHz): δ [ppm] = 1.38 (3H), 3.06 (6H), 4.33 (2H), 6.25 (1H) 32.56g (163.1 mmol) Kupferacetat monohydrat wurden in 470ml Ethanol suspendiert und auf 75°C erwärmt. 61.06g (326.2mmol) Ethyl 4-(dimethylamino)-2,4-dioxobutanoat wurden in einer Portion zugegeben. Man liess das Gemisch für 40min bei 75°C rühren. Der Kupferkomplex wurde bei 4°C ausgefällt.

10.0g (22.9mmol) des Kupferkomplexes wurden in 40ml Ethanol gelöst, 17.2g (229mmol) 2-Methoxyethylamin zugegeben und zwei Stunden bei Raumtemperatur gerührt. Anschliessend engte man die Reaktionsmischung zur Trockene ein. Zur Aufarbeitung wurde der Kupferkomplex in 80ml Dichlormethan suspendiert und 70ml 10%ige Schwefelsäure zugegeben. Nach kräftigem Rühren trennte man die Phasen und extrahierte die wässrige Phase noch zweimal mit 50ml Dichlormethan. Die vereinten organischen Phasen wurden über Natriumsulfat getrocknet und am Rotationsverdampfer zur Trockene eingeengt. Der Rückstand wurde in Diethlyether/Petrolether umkristallisiert. Das kristalline Produkt wurde abfiltriert und im Vakuum getrocknet. Die Ausbeute an $N^1$-(2-Methoxyethyl)-$N^4$,$N^4$-dimethyl-2-oxobutanamid 5.96g (60%).
IR (in Substanz, $cm^{-1}$): 3372, 3311, 3202, 2974, 2934, 2879, 2830, 1787, 1741, 1674, 1634, 1530, 1495, 1473, 1439, 1400, 1358, 1267, 1193, 1176, 1158, 1124, 1107, 1064, 1016, 960, 901, 825, 815, 767, 715.
Enolform
1H-NMR (DMSO-$d_6$, 400 MHz): δ [ppm] = 3.0 (6H), 3.2 (3H), 3.3-3.4 (4H), 6.1 (1H), 8.2 (1H), 15.5 (1H).

### Q. Ethyl-N-[4-(dimethylamino)-2,4-dioxobutanoyl]-N-methylglycinat

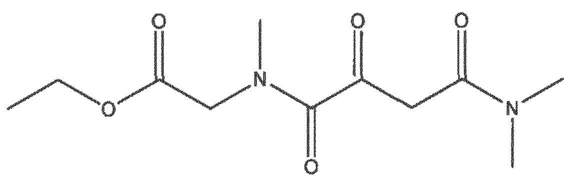

4.73g (58.0mmol) Dimethylaminhydrochlorid wurden in 100ml Dichlormethan suspendiert und 11.1g (58.0mmol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) gelöst in 100ml Dichlormethan zugetropft. Man rührte bei Raumtemperatur über Nacht, gab anschliessend 11.7g (116mmol) Triethylamin zu und kochte für zwei Stunden unter Rückfluss. Die Suspension wurde abfiltriert und das Filtrat zur Trockene eingeengt. Der Rückstand wurde in 170ml Ethylacetat aufgenommen, filtriert und erneut zur Trockene eingeengt. Man erhielt 11g (55%) (2Z)-2-(2,2-Dimethyl-5-oxo-1,3-dioxolan-4-yliden)-N,N-dimethylacetamid.
1H-NMR (DMSO-$d_6$, 400 MHz): $\delta$ [ppm] = 1.7 (6H), 2.9 (3H), 3.0 (3H), 6.0 (1H).
15.4 (100mmol) Sarkosinethylester hydrochlorid und 10.1g (100mmol) Triethylamin wurden in 100ml Dichlormethan suspendiert. Es wurden 5.0g (25mmol) (2Z)-2-(2,2-Dimethyl-5-oxo-1,3-dioxolan-4-yliden)-N,N-dimethylacetamid zugegeben und für eine Stunde unter Rückfluss gekocht. Die Reaktionsmischung wurde zur Trockene eingeengt und der Rückstand in 20ml Ethylacetat aufgenommen. Es wurde abfiltriert und das Filtrat zur Trockene eingeengt. Das Rohprodukt wurde säulenchromatographisch (Kieselgel, Ethylacetat) aufgereinigt. Die Ausbeute an Ethyl-N-[4-(dimethylamino)-2,4-dioxobutanoyl]-N-methylglycinat betrug 2.7g (42%).
IR (in Substanz, $cm^{-1}$): 3351, 2982, 2939, 1729, 1682, 1634, 1508, 1397, 1375, 1353, 1257, 1198, 1096, 1021, 982, 916, 864, 822, 771.
Ketoform:

1H-NMR (DMSO-$d_6$, 400 MHz): $\delta$ [ppm] = 1.2 (3H), 2.8-3.1 (9H), 3.8-4.0 (2H), 4.1 (2H), 4.1-4.3 (2H).

**R. $N^1$-[2-(Dimethylamino)-2-oxoethyl]-$N^1$,$N^4$,$N^4$-trimethyl-2-oxobutandiamid**

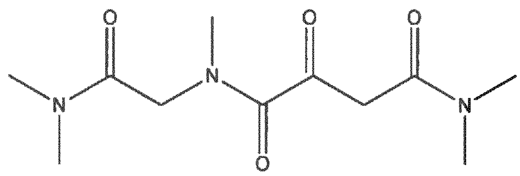

4.73g (58.0mmol) Dimethylaminhydrochlorid wurden in 100ml Dichlormethan suspendiert und 11.1g (58.0mmol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) gelöst in 100ml Dichlormethan zugetropft. Man rührte bei Raumtemperatur über Nacht, gab anschliessend 11.7g (116mmol) Triethylamin zu und kochte für zwei Stunden unter Rückfluss. Die Suspension wurde abfiltriert und das Filtrat zur Trockene eingeengt. Der Rückstand wurde in 170ml Ethylacetat aufgenommen, filtriert und erneut zur Trockene eingeengt. Man erhielt 11g (55%) (2Z)-2-(2,2-Dimethyl-5-oxo-1,3-dioxolan-4-yliden)-N,N-dimethyl acetamid.
1H-NMR (DMSO-$d_6$, 400 MHz): $\delta$ [ppm] = 1.7 (6H), 2.9 (3H), 3.0 (3H), 6.0 (1H).
3.83g (25.1mmol) N,N-Dimethyl-2-(methylamino)acetamid hydrochlorid (*Sigma-Aldrich: CDS007544*) und 2.79g (27.6mmol) Triethylamin wurden in 8ml Ethanol suspendiert. Es wurden 1.00g (5.02mmol) (2Z)-2-(2,2-Dimethyl-5-oxo-1,3-dioxolan-4-yliden)-N,N-dimethylacetamid zugegeben und für 30 Minuten bei Raumtemperatur gerührt. Man gab 5ml 6N HCl-Lösung zu und engte die Reaktionsmischung zur Trockene ein. Man nahm den Rückstand in 10ml Wasser und 20 ml Ethylacetat auf. Die Phasen wurden getrennt und die wässrige noch zweimal mit Ethylacetat extrahiert. Die vereinten organischen Phasen wurden über Natriumsulfat getrocknet und zur Trockene eingeengt. Das Rohprodukt wurde säulenchromatographisch (Kieselgel, Dichlormethan/Ethanol) aufgereinigt. Die Ausbeute an $N^1$-[2-(Dimethylamino)-2-oxoethyl]-$N^1$,$N^4$,$N^4$-trimethyl-2-oxobutandiamid betrug 0.94g (77%).
IR (in Substanz, $cm^{-1}$): 3487, 2935, 1780, 1721, 1632, 1492, 1397, 1354, 1335, 1305, 1259, 1144, 1095, 1054, 981, 942, 857, 813, 760.
Ketoform:

1H-NMR (DMSO-$d_6$, 400 MHz): $\delta$ [ppm] = 2.9-3.1 (15H), 3.9 (2H), 4.1-4.3 (2H).

**S. Ethyl-N-[4-(dimethylamino)-2,4-dioxobutanoyl]glycinat**

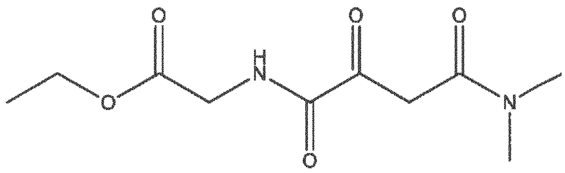

53.30g (0.475mol) Kalium-tert-butoxide wurden in 300ml Diethylether suspendiert. Man gab 73.07g (0.500mol) Diethyloxalat unter Eisbadkühlung zu. Man liess für 30 min gekühlt rühren, gab 43.57g (0.500mol) N,N-Dimethylacetamid zu und rührte die Reaktionsmischung für 2h. Zur Aufarbeitung wurden abfiltrierte und der Feststoff in 260ml 4N Salzsäure und 750ml Ethylacetat zersetzt. Nach Trennung der Phasen wurde die wässrige noch zweimal mit 300ml Ethylacetat extrahiert. Die vereinten organischen Phasen wurden über Natriumsulfat getrocknet und am Rotationsverdampfer zur Trockene eingeengt. Die Ausbeute an Ethyl 4-(dimethylamino)-2,4-dioxobutanoat betrug 54.3g (58%).

1H-NMR (CDCl3, 400 MHz): $\delta$ [ppm] = 1.38 (3H), 3.06 (6H), 4.33 (2H), 6.25 (1H) 32.56g (163.1 mmol) Kupferacetat monohydrat wurden in 470ml Ethanol suspendiert und auf 75°C erwärmt. 61.06g (326.2mmol) Ethyl 4-(dimethylamino)-2,4-dioxobutanoat wurden in einer Portion zugegeben. Man liess das Gemisch für 40min bei 75°C rühren. Der Kupferkomplex wurde bei 4°C ausgefällt.

7.0g (68mmol) Glycinethylester (hergestellt nach E. Fischer, Chemische Berichte, 1906, vol. 39, p. 541) wurden in 13ml Ethanol gelöst, 1.0g (2.3mmol) des Kupferkomplexes zugegeben und eine Stunde bei Raumtemperatur gerührt. Anschliessend engte man die Reaktionsmischung zur Trockene ein. Zur Aufarbeitung wurde der Kupferkomplex in 80ml Dichlormethan suspendiert und 50ml 10%ige Schwefelsäure zugegeben. Nach kräftigem Rühren trennte man die Phasen und extrahierte die wässrige Phase noch zweimal mit 50ml Dichlormethan. Die vereinten organischen Phasen wurden über Natriumsulfat getrocknet und am Rotationsverdampfer zur Trockene eingeengt. Das Rohprodukt wurde säulenchromatographisch (Kieselgel, Ethylacetat/Petrolether) aufgereinigt. Die Ausbeute an Ethyl-N-[4-(dimethylamino)-2,4-dioxobutanoyl]glycinat betrug 0.5g (45%).
IR (in Substanz, $cm^{-1}$): 3389, 2983, 2942, 2324, 2083, 1982, 1739, 1678, 1631, 1608, 1520, 1404, 1354, 1278, 1255, 1179, 1144, 1097, 1084, 1065, 1023, 973, 915, 860, 819, 773, 717.
Enolform
1H-NMR (DMSO-$d_6$, 400 MHz): $\delta$ [ppm] = 1.2 (3H), 3.0 (6H), 3.9 (2H), 4.1 (2H), 6.2 (1H), 8.7 (1H), 15.5 (1H).

### T. $N^{1-}$(2-Methoxyethyl)-$N^1$,$N^4$,$N^4$-trimethyl-2-oxobutandiamid

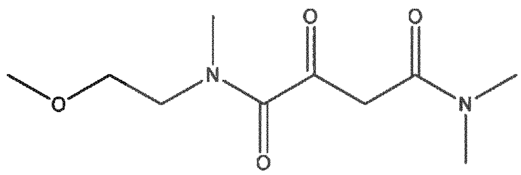

19.5g (219mmol) N-(2-Methoxyethyl)methylamin und 22.2g (219mmol) Triethylamin wurden in 400ml Diethylether gelöst und im Eisbad abgekühlt. Man gab tropfenweise 30.0g (219mmol) Ethyl chlorooxoacetat zu und liess auf Raumtemperatur erwärmen. Die Suspension wurde filtriert und das Filtrat am Rotationsverdampfer eingeengt. Man erhielt 36.9g Ethyl-[(2-methoxyethyl)(methyl)amino](oxo)acetat in Form eines gelben Öls, welches ohne Reinigung weiter verarbeitet wurde.

20.8g (185mmol) Kalium-tert-butoxide wurden in 200ml Diethylether suspendiert. Man gab 36.9g (195mmol) Ethyl-[(2-methoxyethyl)(methyl)amino](oxo)acetat unter Eisbadkühlung zu. Man liess für 30 min bei Raumtemperatur rühren, gab 17.0g (195mmol) N,N-Dimethylacetamid zu und rührte die Reaktionsmischung bei Raumtemperatur für zwei Stunden. Die Reaktionslösung wurde mit 123ml 6N Salzsäure, 130ml Ethylacetat und 30ml Wasser versetzt. Nach Trennung der Phasen wurde die wässrige noch dreimal mit 100ml Ethylacetat extrahiert. Die vereinten organischen Phasen wurden über Natriumsulfat getrocknet und am Rotationsverdampfer zur Trockene eingeengt. Das Rohprodukt wurde säulenchromatographisch aufgereinigt (Kieselgel, Dichlormethan/Aceton). Die Ausbeute an $N^1$-(2-Methoxyethyl)-$N^1$,$N^4$,$N^4$-trimethyl-2-oxobutandiamid betrug 4.5g (10%).
IR (in Substanz, $cm^{-1}$): 3482, 2935, 1720, 1631, 1492, 1454, 1401, 1355, 1293, 1260, 1198, 1115, 1069, 1014, 972, 828, 772721.
Ketoform
1H-NMR (DMSO-$d_6$, 400 MHz): $\delta$ [ppm] = 2.8-3.0 (9H), 3.3 (3H), 3.4-3.5 (4H), 3.9 (2H).

**U. N4-(2-Hydroxyethyl)-N1,N1,N4-trimethyl-2-oxobutandiamid**

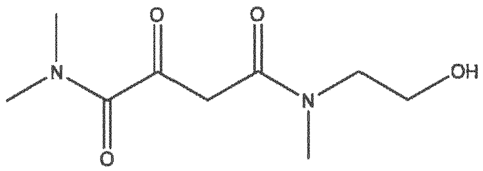

1.18g (15.7mmol) 2-(Methylamino)ethanol wurden in 50ml Dichlormethan gelöst und 2.39g (23.6mmol) Triethylamin zugegeben. Man gab 3.00g (15.7mmol) (2Z)-(2,2-dimethyl-5-oxo-1,3-dioxolan-4-yliden)acetyl chlorid (hergestellt nach J. Banville et al, Tetrahedron Letters, 2010, 51, 3170 - 3173) zu und rührt bei Raumtemperatur für 30 Minuten. Anschliessend wurde noch für vier Stunden unter Rückfluss gekocht. Man engte die Reaktionsmischung zur Trockene ein und nahm den Rückstand in 300ml Ethylacetat auf. Die Suspension wurde abfiltriert und das Filtrat zur Trockene eingeengt. Der Rückstand wurde säulenchromatographisch (Kieselgel, Dichloremthan/Ethanol) aufgereinigt. Man erhielt 1.5g (42%) (2Z)-2-(2,2-Dimethyl-5-oxo-1,3-dioxolan-4-yliden)-N-(2-hydroxyethyl)-N-methylacetamid.
1H-NMR (DMSO-$d_6$, 400 MHz): $\delta$ [ppm] = 1.7 (6H), 3.0 (3H), 3.4-3.6 (4H), 4.7-4.9 (1H), 6.0-6.2 (1H).

1.0g (4.4mmol) (2Z)-2-(2,2-Dimethyl-5-oxo-1,3-dioxolan-4-yliden)-N-(2-hydroxyethyl)-N-methylacetamid wurden in 8ml ethanolischer Dimethylaminlösung (33%ig) gelöst und 15 Minuten bei Raumtemperatur gerührt. Man engte zur Trockene ein und erhielt 0.95g (99%) $N^4$-(2-Hydroxyethyl)-$N^1$,$N^1$,$N^4$-trimethyl-2-oxobutandiamid.
IR (in Substanz, $cm^{-1}$): 3401, 2935, 1720, 1626, 1490, 1448, 1401, 1359, 1297, 1261, 1208, 1110, 1074, 1047, 950, 880, 863, 806, 773, 719.
Ketoform:

1H-NMR (DMSO-$d_6$, 400 MHz): $\delta$ [ppm] = 2.8-3.1 (9H), 3.3-3.5 (4H), 3.9-4.0 (2H), 4.9 (1H).

**TESTMETHODE:**

Die hervorragenden Fe-Utilisationen, die durch die erfindungsgemäßen Fe-Komplexe, erreicht werden können, wurden durch folgendes Mäuse-Modell gemessen.

(Es wurden jeweils 6 Messtiere pro Substanz verwendet. Es gab eine Negativ-Gruppe (Wasser) mit 6 Tieren und als Positiv-Gruppe wurde $FeSO_4$ verwendet jeweils auch mit 6 Tieren).

Männliche NMRI (SPF)-Mäuse (ca. 3 Wochen alt) wurden ca. 3 Wochen mit eisenarmer Diät (ca. 5 ppm Eisen) gefüttert. Dann wurde ihnen während 2 mal 5 Tagen mit einer Unterbrechung von 2 Tagen (Tage 1 - 5 und 8 - 12) die Eisenkomplexe mittels Schlundsonde verabreicht (2 mg Eisen/kg Körpergewicht/Tag). Die Utilisation am Tag 15 wurde berechnet aus der Hämoglobinzunahme und der Körpergewichtszunahme nach der Formel

$$\text{Utilisation (\%)} = \frac{\Delta\,\text{Eisenutilisation} * 100}{\text{Fe Dos.}} = \frac{(\text{Fe ut.} - \text{Fe ut. Control}) * 100}{\text{Fe Dos.}}$$

$$= [(Hb_{2(3)} * BW_{9(14)} - Hb_1 * BW_4) * 0{,}07 * 0{,}0034 - (Hb_{2(3)\,Control} * BW_{9(14)\,Control} - Hb_{1\,Control} * BW_{4\,Control}) * 0{,}07 * 0{,}0034)] * 100 / \text{Fe Dos.}$$

$$= [(Hb_{2(3)} * BW_{9(14)} - Hb_1 * BW_4) * 0{,}000238 - (Hb_{2(3)\,Control} * BW_{9(14)\,Control} - Hb_{1\,Control} * BW_{4\,Control}) * 0{,}000238] * 100 / \text{Fe Dos.}$$

$$= (Hb_{2(3)} * BW_{9(14)} - Hb_1 * BW_4 - Hb_{2(3)\,Control} * BW_{9(14)\,Control} + Hb_{1\,Control} * BW_{4\,Control}) * 0{,}0238 / \text{Fe Dos.}$$

| | |
|---|---|
| 0.07 | = Faktor für 70 ml Blut per kg Körpergewicht (BW) |
| 0.0034 | = Faktor für 0,0034 g Fe/g Hb |
| $Hb_1$ | = Hämoglobin-Wert (g/l) am Tag 1 |
| $Hb_{2(3)}$ | = Hämoglobin-Wert (g/l) am Tag 8 (or 15) |
| $BW_4$ | = Körpergewicht (g) am Tag 1 |
| $BW_{9(14)}$ | = Körpergewicht (g) am Tag 8 (or 15) |
| $Hb_{1\ Control}$ | = Durchschnitts-Hämoglobinwert (g/l) am Tag 1 in der Kontrollgruppe, |
| $Hb_{2(3)\ Control}$ | = Durchschnitts-Hämoglobinwert (g/l) am Tag 8 (oder 15) in der Kontrollgruppe, |
| $BW_{4\ Control}$ | = Durchschnittskörpergewicht (g) am Tag 1 in der Kontrollgruppe, |
| $BW_{9(14)\ Control}$ | = Durchschnittskörpergewicht (g) am Tag 8 (oder 15) in der Kontrollgruppe, |
| Fe Dos. | = Gesamtes verabreichtes Eisen (mg Fe) während 5 oder 10 Tage, |
| Fe ut. | = $(Hb_{2(3)} * BW_{9(14)} - Hb_1 * BW_4) * 0.07 * 0.0034$ (mg Fe) |
| Δ Utilisation | = Fe tot. utilisiert (Untersuchte Gruppe) - Fe ut. Kontrollgruppe, utilisiert aus Nahrung, (mg Fe) |

Die nachfolgende Tabelle 1 zeigt die Eisenutilisation der Verbindung von Beispiel 1 und vergleicht diese mit dem entsprechenden Wert, der für die Verbindung von Beispiel 32 der WO11117225A1 erhalten wurde:

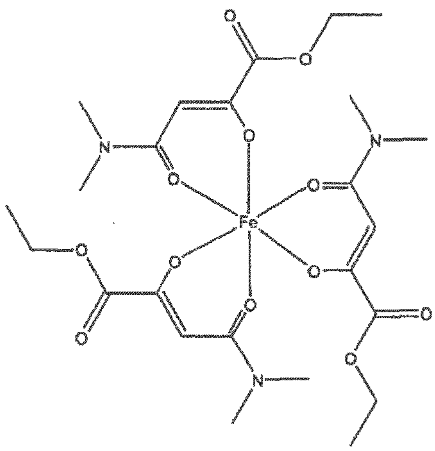

Tabelle 1:

| Beispiel-Nr. | Utilisation n 15 d (abs. %) |
|---|---|
| 1 | 98 |
| 2 | 75 |
| 4 | 92 |
| 5 | 82 |
| 7 | 68 |
| 11 | 50 |
| 12 | 80 |
| 13 | 70 |
| 16 | 65 |
| 17 | 91 |
| 18 | 78 |
| 21 | 86 |
| Vergleichsbeispiel (Beispiel 32 der WO11117225A1) | 81 |

Die nachfolgende Tabelle zeigt, dass die gegenüber der WO11117225A1 vorgenommene strukturelle Änderung

im allgemeinen zu einer verbesserten Eisenutilisation führt:

| Erfindungsgemäßes Beispiel | Beispiel aus WO11117225A1 |
|---|---|
| Beispiel 2 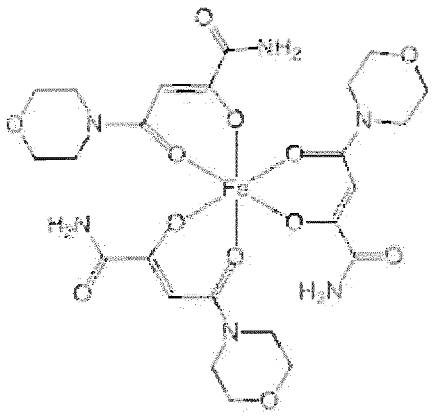 75% | Beispiel 62 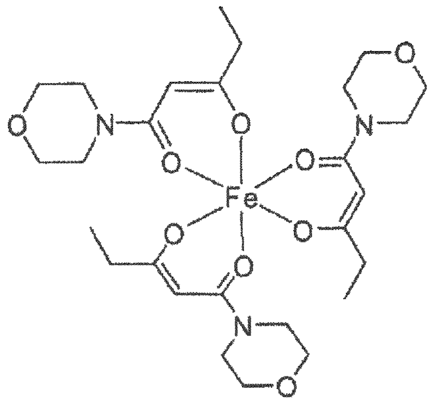 68% |
| Beispiel 1 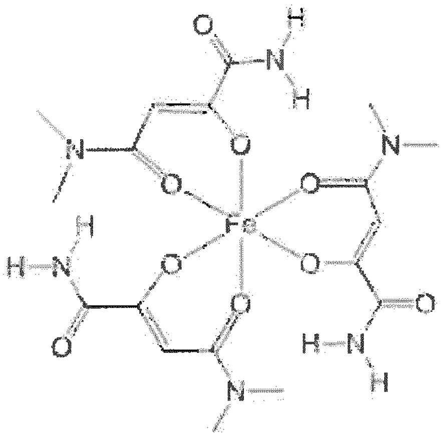 98% | Beispiel 32 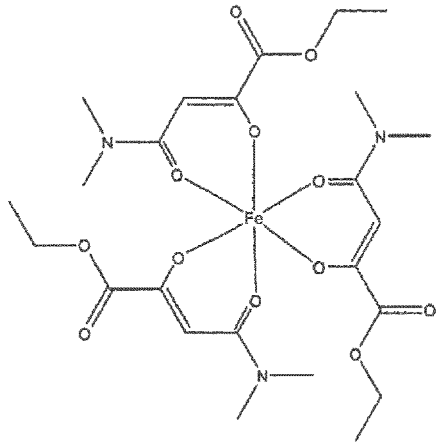 81% |
| Beispiel 1 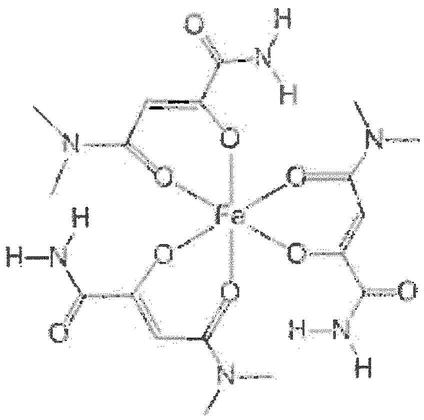 98% | Beispiel 19 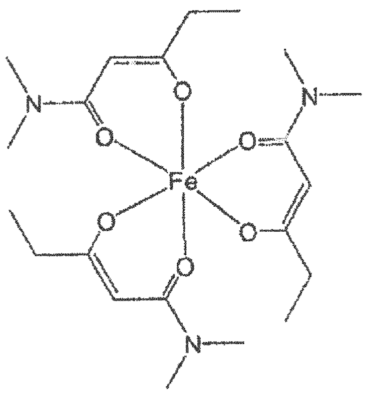 73% |
| Beispiel 5 | Beispiel 19 |

(fortgesetzt)

| Erfindungsgemäßes Beispiel | Beispiel aus WO11117225A1 |
|---|---|
| 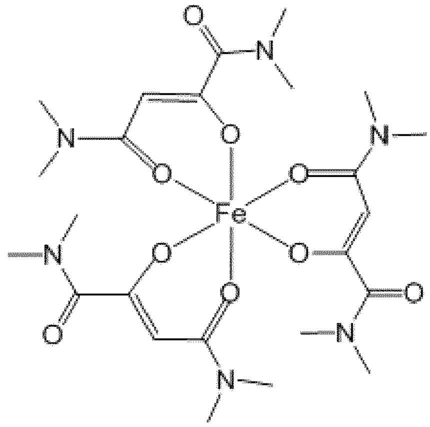<br>82% | 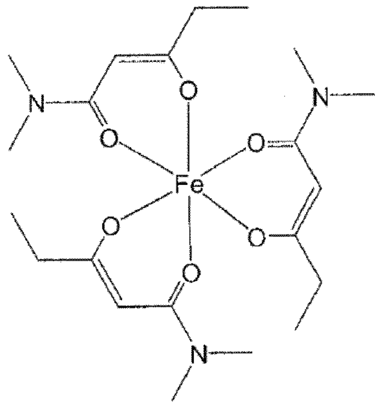<br>73% |
| Beispiel 21<br>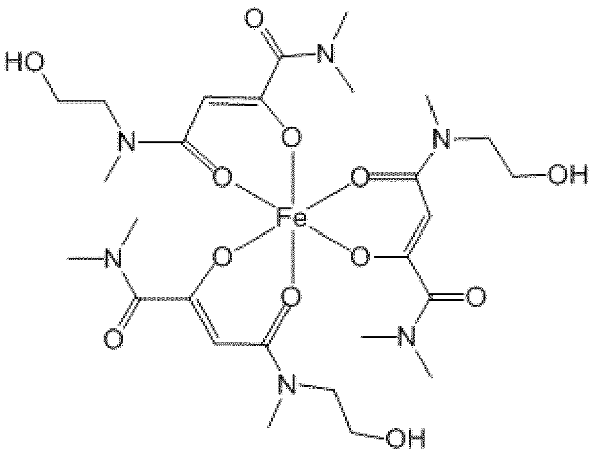<br>86% | Beispiel 42<br>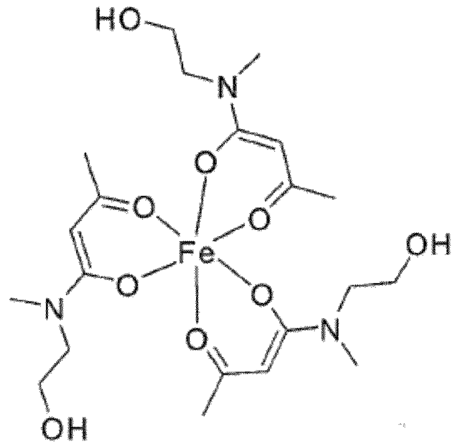<br>30% |

Die Testergebnisse zeigen, dass die erfindungsgemäßen Eisenkomplexverbindungen über eine hervorragende Eisenutilisation verfügen, so dass sie als Mittel zur Behandlung von Eisenmangelanämien und der damit verbundenen Symptome geeignet sind.

Die Testergebnisse zeigen weiterhin, dass die Verbindung gemäß Beispiel 1 der vorliegenden Anmeldung eine signifikant verbesserte Eisenutilisation im Vergleich zu Beispiel 32 der WO11117225A1 aufweist.

**Vergleich der pH Wert-abhängigen Löslichkeit:**

Hierzu wurde eine bestimmte Menge an zu untersuchendem Eisen(III) Komplex (Einwaage) im allgemeinen im Bereich einer Konzentration von 0.4 - 0.8 mg/ml, bezogen auf die Menge des Eisens im Komplex, in ein wässriges Medium (Wasser) mit eingestelltem pH Wert (pH 6.5) gegeben und bei 25°C für die angegebene Zeit (4h) gerührt. Der pH Wert wurde mit Phthalat-Puffer (0,1 m) auf 6,5 eingestellt.
Anschliessend wurde der Eisengehalt der Lösung in mg/ml bezogen auf Eisen photometrisch bestimmt (Küvetten: Einmalküvetten 1cm Plastibrand PS 2.5ml ISO zert. 9001 14001; Gerät: UV Gerät vom Typ SPECORD 205 Hersteller Jena Analytik). Dieser Wert ist die Löslichkeit des Eisenkomplexes. Wenn sich die eingewogenen Menge des Eisenkomplexes vollständig löste, wurde vor den gemessenen Wert der Löslichkeit ein ">"-Zeichen gesetzt. Die Ergebnisse sind in nachfolgender Tabelle 2 gezeigt. Es zeigt sich, dass die erfindungsgemäßen Verbindungen deutlich höhere Löslichkeiten als die Verbindung von Beispiel 32 der WO11117225A1 besitzen.
Die erfindungsgemäßen Eisen(III)-Komplexverbindungen weisen bevorzugt eine Löslichkeit in Wasser bei 25°C bei pH 6.5 von mindestens 0.3 mg/ml bezogen auf Eisen auf, welches wie zuvor dargelegt wurde photometrisch bestimmt wird. Bevorzugte Eisen(III)-Komplexverbindungen weisen eine Löslichkeit in Wasser bei 25°C bei pH 6.5 von mindestens 0.4

mg/ml bezogen auf Eisen auf. Besonders bevorzugte Eisen(III)-Komplexverbindungen weisen eine Löslichkeit in Wasser bei 25°C bei pH 6.5 von mindestens 0.5 mg/ml bezogen auf Eisen auf.

Eine gute Wasserlöslichkeit der Eisen(III)-Komplexverbindungen ist eine Voraussetzung für eine verbesserte orale Aufnahme. Orale Medikamente, die eine bessere Löslichkeit haben, führen im Allgemeinen zu einem besseren Eisen-Uptake. Wasserunlösliche Verbindungen können im Allgemeinen nicht für eine orale Applikation verwendet werden, da praktisch keine Absorption erfolgt.

**Tabelle 2 - Vergleich der pH Wert-abhängigen Löslichkeit:**

| Beispiel | Fe Gehalt % | pH | Zeit | Einwaage mg | mg/ml Fe | Optische Beurteilung | | | Volumen Aliquot in ml | Absorption bei 515nm | Löslichkeit bezogen auf Menge Eisen in mg/ml |
|---|---|---|---|---|---|---|---|---|---|---|---|
| | | | | | | Gelöst? | Farbe filtriert | Intensität | | | |
| Vergleichsbeispiel (Beispiel 32 der WO11117225A1 ) | 9,43 | 6,5 | 4h | 6,7 | 0,75 | nein | rot | +++ | 0,1 | 0,3764 | 0,206 |
| Beispiel 1 | 11,28 | 6,5 | 4h | 5,5 | 0,61 | ja | rot | +++ | 0,1 | 0,9643 | > 0,529 |
| Beispiel 5 | 8,43 | 6,5 | 4h | 7,5 | 0,53 | ja | rot | +++ | 0,1 | 1,1340 | > 0,55 |
| Beispiel 11 | 7,88 | 6,5 | 4h | 7,7 | 0,51 | ja | orange | +++ | 0,1 | 0,6783 | > 0,33 |
| Beispiel 12 | 6,20 | 6,5 | 4h | 9,5 | 0,49 | ja | orange | +++ | 0,1 | 1,0464 | > 0,51 |
| Beispiel 13 | 6,02 | 6,5 | 4h | 9,8 | 0,49 | ja | orange | +++ | 0,1 | 1,0656 | > 0,52 |
| Beispiel 16 | 7,21 | 6,5 | 4h | 8,2 | 0,49 | nein | orange | +++ | 0,1 | 0,9226 | 0,45 |
| Beispiel 17 | 6,64 | 6,5 | 4h | 9,2 | 0,51 | nein | orange | +++ | 0,1 | 0,9607 | 0,47 |
| Beispiel 18 | 5,05 | 6,5 | 4h | 12,0 | 0,51 | nein | orange | +++ | 0,1 | 1,2031 | 0,58 |
| Beispiel 21 | 7,34 | 6,5 | 4h | 7,0 | 0,43 | nein | orange | +++ | 0,1 | 1,0239 | 0,49 |

**HERSTELLUNGSBEISPIELE:**

**Beispiel 1**

**Tris-($N^4$,$N^4$-dimethyl-2-oxobutandiamid)-eisen(III)-Komplex:**

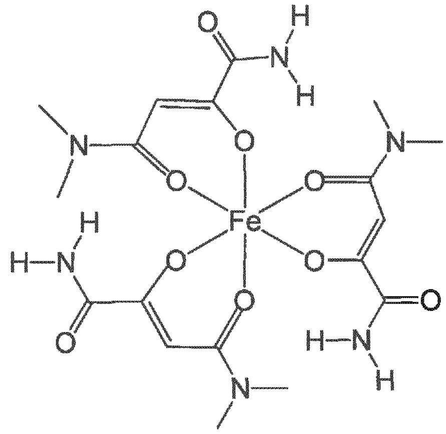

1.75 g (8.78 mmol) Eisen(III)acetat wurden in 160 ml 94%igem Ethanol gelöst und 5.01 g (31.6 mmol) $N^4$,$N^4$-Dimethyl-2-oxobutandiamid zugegeben. Man kochte für eine Stunde unter Rückfluss. Der fertige Eisenkomplex wurde bei 4°C ausgefällt. Der ausgefallene Eisenkomplex wurde abfiltriert und einen Tag bei 50°C im Vakuum getrocknet. Man erhielt 3.74 g (81% Ausbeute) Produkt als roten Feststoff.
IR (in Substanz, $cm^{-1}$): 3437, 3405, 3153, 2931, 2323, 1680, 1594, 1567, 1498, 1403, 1352, 1258, 1171, 1142, 1058, 1001, 936, 814, 766, 735, 686.
Elementaranalyse: C 41.17%, H 5.2%, N 15.9%.
Fe-Gehalt: 10.3% [m/m].

**2. Tris-(4-(morpholin-4-yl)-2,4-dioxobutanamid)-eisen(III)-Komplex**

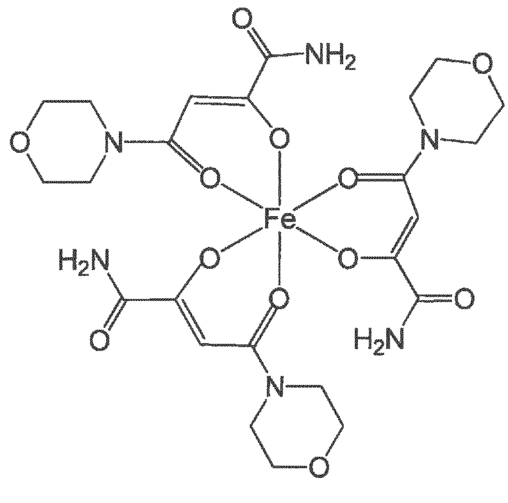

0.60 g (3.1 mmol) Eisen(III)acetat wurden in 150 ml 94%igem Ethanol gelöst und 2.00 g (10.0 mmol) 4-(Morpholin-4-yl)-2,4-dioxobutanamid zugegeben. Man kochte für eine Stunde unter Rückfluss. Der fertige Eisenkomplex wurde nach Zusatz von Impfkristallen bei 4°C ausgefällt und abfiltriert. Man trocknete das Produkt für einen Tag bei 50°C im Feinvakuum. Man erhielt 1.1 g (54% Ausbeute) Produkt als roten Feststoff.
IR (in Substanz, $cm^{-1}$): 3608, 3352, 3135, 2960, 2918, 2850, 2324, 2050, 1981, 1685, 1570, 1498, 1460, 1443, 1373,

1301, 1273, 1245, 1145, 1112, 1050, 1017, 986, 936, 850,817,764,737,676.
Elementaranalyse: C 42.71%, H 5.7%, N 11.94% .
Fe-Gehalt: 8.3% [m/m].

**3. Tris-($N^4$,$N^4$-diethyl-2-oxobutandiamid)-eisen(III)-Komplex**

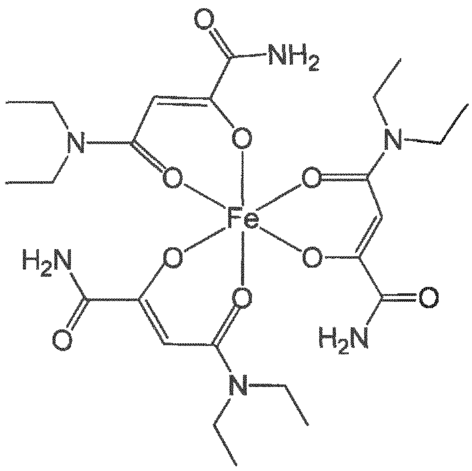

0.13 g (0.83 mmol) Eisen(III)chlorid wurden in 10ml Wasser gelöst und 0.50 g (2.5 mmol) $N^4$,$N^4$-diethyl-2-oxobutandiamid zugegeben. Man rührte 15 Minuten bei Raumtemperatur und kühlte die Reaktionsmischung danach im Eisbad. Man gab 0.45g (3.3mmol) Natriumacetat trihydrat zu und liess weitere 15min im Eisbad rühren. Der ausgefallene Eisenkomplex wurde abfiltriert und über Nacht bei 50°C im Feinvakuum getrocknet. Man erhielt 0.5 g (92% Ausbeute) Produkt als orangen Feststoff.
IR (in Substanz, $cm^{-1}$): 3458, 3301, 2975, 2935, 1681, 1618, 1566, 1496, 1453, 1437, 1376, 1355, 1307, 1270, 1215, 1160, 1078, 1035, 964, 925, 818, 771, 743, 659.
Elementaranalyse: C 46.31 %, H 6.5%, N 13.56% .
Fe-Gehalt: 9.1% [m/m].

**4. Tris-(2,4-dioxo-4-(pyrrolidin-1-yl)butanamid)-eisen(III)-Komplex**

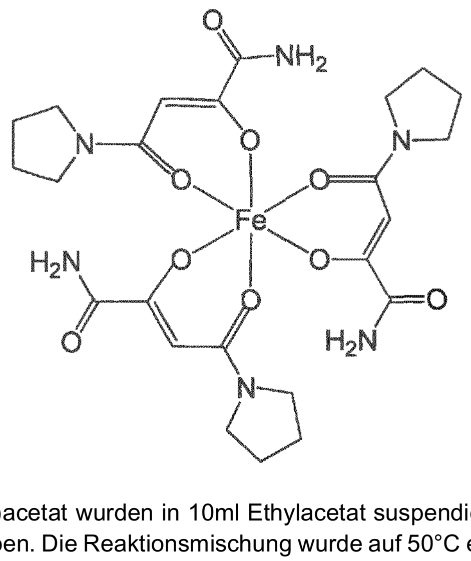

0.28 g (1.4 mmol) Eisen(III)acetat wurden in 10ml Ethylacetat suspendiert und 0.80 g (4.3 mmol) 2,4-Dioxo-4-(pyrrolidin-1-yl)butanamid zugegeben. Die Reaktionsmischung wurde auf 50°C erwärmt. Anschliessend wurden 30ml Ethylacetat und 20ml Ethanol zugegeben und die Suspension auf 90°C erwärmt. Man kühlte die Reaktionsmischung danach im Eisbad. Der ausgefallene Eisenkomplex wurde abfiltriert und für drei Tage bei 50°C im Feinvakuum getrocknet.

Man erhielt 0.67 g (62% Ausbeute) Produkt als roten Feststoff.
IR (in Substanz, $cm^{-1}$): 3454, 3367, 3178, 2968, 2877, 1685, 1627, 1560, 1493, 1474, 1457, 1367, 1254, 1225, 1182, 1134, 1116, 1059, 1026, 972, 938, 916, 856, 814, 769, 717, 659.
Elementaranalyse: C 47.45%, H 5.6%, N 13.32% .
Fe-Gehalt: 8.8% [m/m].

**5. Tris-(N,N,N',N'-tetramethyl-2-oxobutandiamid)-eisen(III)-Komplex**

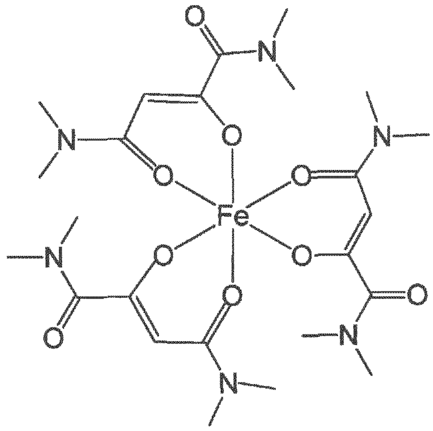

0.54 g (3.3 mmol) Eisen(III)chlorid wurden in 20ml Wasser gelöst und 2.0 g (10 mmol) N,N,N',N'-Tetramethyl-2-oxobutandiamid zugegeben. Man rührte 15 Minuten bei Raumtemperatur und kühlte die Reaktionsmischung danach im Eisbad. Man gab 1.8g (13mmol) Natriumacetat trihydrat zu und liess weitere 10min im Eisbad rühren. Man extrahierte die wässrige Reaktionslösung dreimal mit 50ml Chloroform. Die vereinten organischen Phasen wurden am Rotationsverdampfer zur Trockene eingeengt und noch zweimal mit 50ml Toluol evaporiert. Der Eisenkomplex wurde über Nacht bei 50°C im Feinvakuum getrocknet. Man erhielt 1.6 g (73% Ausbeute) Produkt als roter Feststoff.
IR (in Substanz, $cm^{-1}$):3496, 2929, 1634, 1568, 1486, 1392, 1354, 1259, 1203, 1174, 1118, 1059, 1006, 962, 900, 811, 771, 710, 665.
Elementaranalyse: C 45.95%, H 6.4%, N 13.19% .
Fe-Gehalt: 9.1% [m/m].

**6. Tris-(4-(4-hydroxypiperidin-1-yl)-2,4-dioxobutanamid)-eisen(III)-Komplex**

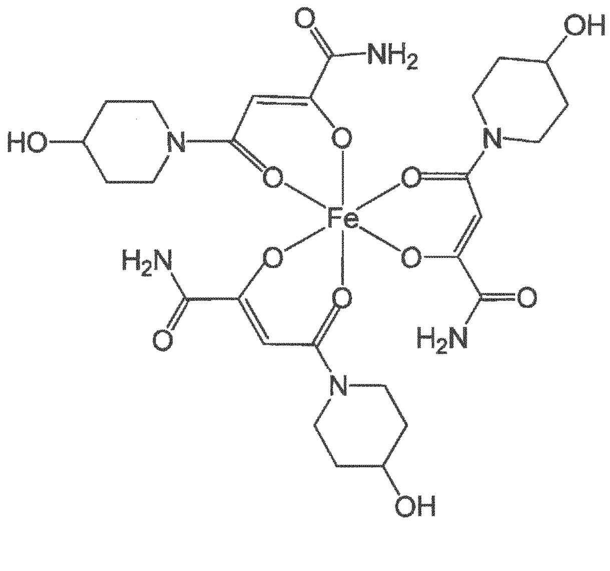

144mg (0.734 mmol) Eisen(III)acetat wurden in 50ml Ethanol suspendiert und 471mg (2.20 mmol) 4-(4-hydroxypiperidin-1-yl)-2,4-dioxobutanamid zugegeben. Die Reaktionsmischung wurde für eine Stunde unter Rückfluss gekocht. Man gab 60ml Toluol zu und engte zur Trockene ein. Der Rückstand wurde noch zweimal in Ethanol/Toluol-Mischung (40ml/60ml) aufgenommen und zur Trockene eingeengt. Der Rückstand wurde bei 50°C im Feinvakuum getrocknet. Man erhielt 0.5 g (98% Ausbeute) Produkt als roten Feststoff.
IR (in Substanz, $cm^{-1}$): 3445, 3298, 2946, 2924, 2324, 2051, 1981, 1687, 1666, 1598, 1561, 1493, 1448, 1370, 1262, 1227, 1138, 1075, 1050, 1023, 985, 928, 834, 816, 767, 664.
Elementaranalyse: C 46.26%, H 5.8%, N 11.60% .
Fe-Gehalt: 7.7% [m/m].

**7. Tris-($N^1$,$N^4$,$N^4$-trimethyl-2-oxobutandiamid)-eisen(III)-Komplex**

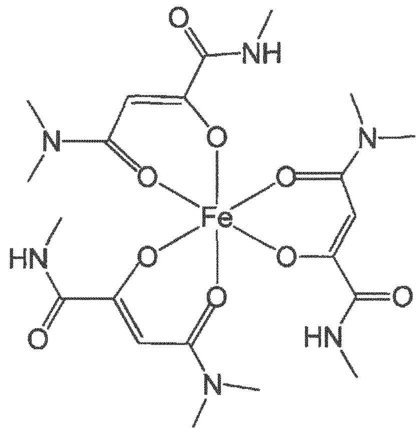

0.784 g (4.83 mmol) Eisen(III)chlorid wurden in 20ml Wasser gelöst und 2.55 g (14.5 mmol) $N^1$,$N^4$,$N^4$-trimethyl-2-oxobutandiamid zugegeben. Man rührte 15 Minuten bei Raumtemperatur und kühlte die Reaktionsmischung danach im Eisbad. Man gab 2.16g (19.2mmol) Natriumacetat trihydrat zu und liess weitere 15min im Eisbad rühren. Der ausgefallene Eisenkomplex wurde abfiltriert und über Nacht bei 50°C im Feinvakuum getrocknet. Man erhielt 1.5 g (53% Ausbeute) Produkt als orangen Feststoff.
IR (in Substanz, $cm^{-1}$): 3352, 2934, 2877, 2324, 1674, 1598, 1566, 1504, 1405, 1356, 1253, 1176, 1152, 1063, 992, 980, 961, 930, 896, 847, 813, 769, 748, 736, 706.
Elementaranalyse: C 43.74%, H 5.9%, N 14.53% .
Fe-Gehalt: 9.5% [m/m].

**8. Tris-(N,N'-Dimethyl-2-oxobutandiamid)-eisen(III)-Komplex**

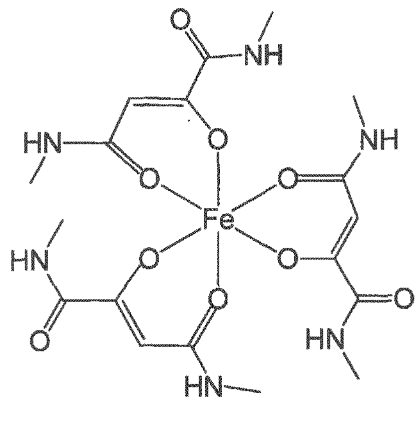

242mg (1.49 mmol) Eisen(III)chlorid wurden in 10ml Wasser gelöst und 707mg (4.47 mmol) N,N'-Dimethyl-2-oxobutandiamid zugegeben. Man rührte 15 Minuten bei Raumtemperatur. Man gab 812mg (5.97mmol) Natriumacetat trihydrat zu und liess weitere 30min rühren. Der ausgefallene Eisenkomplex wurde abfiltriert und für 4 Tage bei 50°C im Feinvakuum getrocknet. Man erhielt 587mg (74% Ausbeute) Produkt als orangen Feststoff.
IR (in Substanz, $cm^{-1}$): 3334, 3256, 3129, 2940, 1673, 1603, 1546, 1507, 1407, 1278, 1239, 1160, 1139, 1087, 1028, 963, 897, 817, 800, 777, 724, 691.
Elementaranalyse: C 40.04%, H 5.1%, N 15.49% .
Fe-Gehalt: 10.2% [m/m].

**9. Tris-($N^1$,$N^1$,$N^4$-Trimethyl-2-oxobutandiamid)-eisen(III)-Komplex**

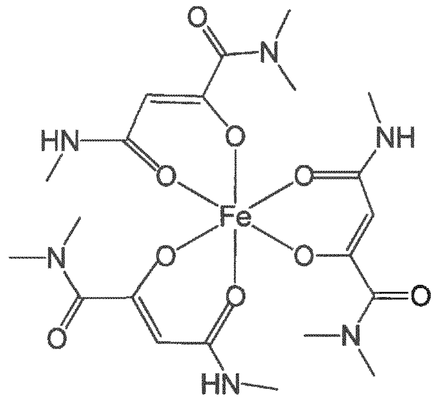

75mg (0.46 mmol) Eisen(III)chlorid wurden in 3ml Wasser gelöst und 239mg (1.39mmol) $N^1$,$N^1$,$N^4$-Trimethyl-2-oxobutandiamid zugegeben. Man rührte 15 Minuten bei Raumtemperatur, gab 252mg (1.85mmol) Natriumacetat trihydrat zu und liess weitere 30min rühren. Man engte die Reaktionsmischung zur Trockene ein und nahm den Rückstand in 20ml Chloroform auf. Die unlöslichen Bestandteile wurden abfiltriert und das Filtrat zur Trockene eingeengt. Man trocknete über Nacht bei 50°C im Feinvakuum. Man erhielt 230mg Produkt in einer Reinheit von 78%.
IR (in Substanz, $cm^{-1}$): 3270, 3118, 2932, 2051, 1578, 1491, 1433, 1395, 1278, 1192, 1157, 1123, 1076, 966, 902, 778.
Elementaranalyse: C 41.89%, H 6.1%, N 11.20% .
Fe-Gehalt: 7.7% [m/m].

**10. Tris-($N^4$,$N^4$-Diethyl-$N^1$,$N^1$-dimethyl-2-oxobutandiamid)-eisen(III)-Komplex**

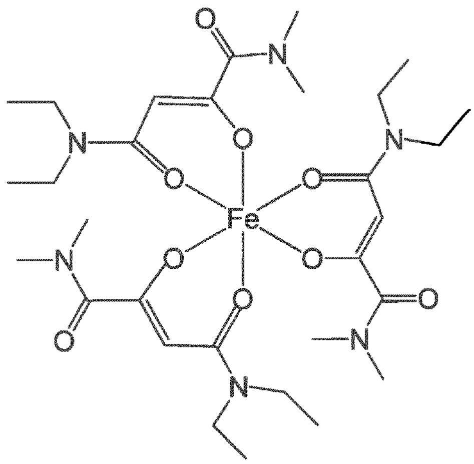

0.49g (3.0 mmol) Eisen(III)chlorid wurden in 20ml Wasser gelöst und 2.0g (9.0mmol) $N^4$,$N^4$-Diethyl-$N^1$,$N^1$-dimethyl-2-oxobutandiamid zugegeben. Man rührte 15 Minuten bei Raumtemperatur und kühlte die Reaktionsmischung im Eisbad. Man gab 1.63g (11.8mmol) Natriumacetat trihydrat zu und liess weitere 30min rühren. Man extrahierte die

Reaktionsmischung dreimal mit 50ml Chloroform, trocknete über Natriumsulfat und engte zur Trockene ein. Zur Entfernung von Essigsäure wurde der Rückstand dreimal mit 50ml Toluol abgeschleppt. Man trocknete den Rückstand über Nacht bei 50°C im Feinvakuum und erhielt 1.8g (83%) Produkt in Form eines weinroten Feststoffs.
IR (in Substanz, $cm^{-1}$): 3481, 2973, 2933, 2050, 1636, 1602, 1563, 1511, 1487, 1435, 1392, 1376, 1357, 1308, 1274, 1218, 1198, 1160, 1120, 1080, 1036, 964, 929, 887, 808, 789, 767, 707.
Elementaranalyse: C 51.12%, H 7.2%, N 11.68% .
Fe-Gehalt: 8.0% [m/m].

**11. Tris-(N,N-Dimethyl-3,4-dioxo-4-(pyrrolidin-1-yl)butanamid)-eisen(III) Komplex**

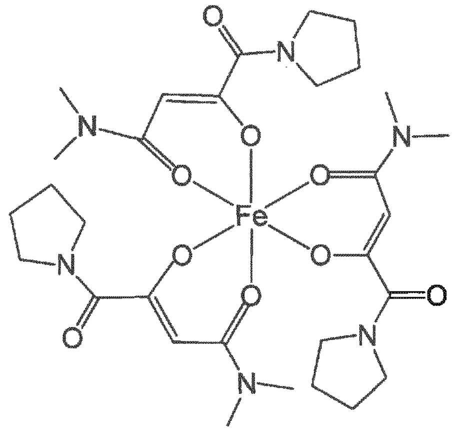

0.48g (3.0 mmol) Eisen(III)chlorid wurden in 20ml Wasser gelöst und 2.0g (8.9mmol) N,N-Dimethyl-3,4-dioxo-4-(pyrrolidin-1-yl)butanamid zugegeben. Man rührte 15 Minuten bei Raumtemperatur und kühlte die Reaktionsmischung im Eisbad. Man gab 1.61g (11.8mmol) Natriumacetat trihydrat zu und liess weitere 30min rühren. Man extrahierte die Reaktionsmischung dreimal mit 50ml Chloroform, trocknete über Natriumsulfat und engte zur Trockene ein. Zur Entfernung von Essigsäure wurde der Rückstand dreimal mit 50ml Toluol abgeschleppt. Man trocknete den Rückstand über Nacht bei 50°C im Feinvakuum und erhielt 2.1g (97%) Produkt in Form eines weinroten Feststoffs.
IR (in Substanz, $cm^{-1}$): 3465, 2949, 2875, 2324, 2051, 1694, 1605, 1563, 1504, 1402, 1357, 1294, 1255, 1225, 1162, 1061, 1012, 980, 960, 920, 896, 850, 809, 762, 705.
Elementaranalyse: C 51.51%, H 6.4%, N 11.73% .
Fe-Gehalt: 8.1% [m/m].

**12. Tris-($N^4$-[2-(dimethylamino)-2-oxoethyl]-$N^1$,$N^1$,$N^4$-trimethyl-2-oxobutandiamid)-eisen(III)-Komplex**

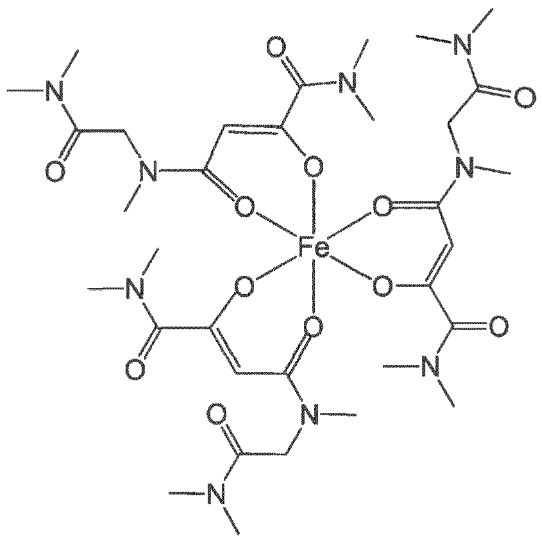

0.40g (2.5mmol) Eisen(III)chlorid wurden in 20ml Wasser gelöst und 2.0g (7.5mmol) N4-[2-(dimethylamino)-2-oxoethyl]-N1,N1,N4-trimethyl-2-oxobutandiamid gelöst in 3ml Ethanol zugegeben. Man rührte 15 Minuten bei Raumtemperatur, gab 1.4g (9.9mmol) Natriumacetat trihydrat zu und liess weitere 30min rühren. Die Reaktionslösung wurde dreimal mit 50ml Chloroform extrahiert, über Natriumsulfat getrocknet und zur Trockene eingeengt. Zur Entfernung von Essigsäure wurde der Rückstand zweimal mit 50ml Toluol abgeschleppt. Man trocknete über Nacht bei 50°C im Feinvakuum und erhielt 2.1g (98%) Produkt in Form eines roten Feststoffs.
IR (in Substanz, $cm^{-1}$): 3453, 2933, 2324, 2164, 2051, 1981, 1628, 1566, 1510, 1485, 1395, 1364, 1335, 1298, 1254, 1218, 1143, 1112, 1060, 1039, 964, 905, 825, 808, 763, 713, 668.
Elementaranalyse: C 46.06%, H 6.8%, N 14.46% .
Fe-Gehalt: 6.2% [m/m].

### 13. Tris-($N^4$-(2-methoxyethyl)-$N^1$,$N^1$,$N^4$-trimethyl-2-oxobutandiamid)-eisen(III)-Komplex

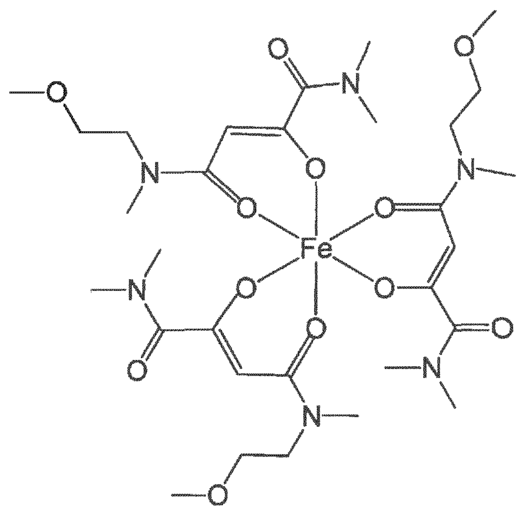

0.44g (2.7mmol) Eisen(III)chlorid wurden in 20ml Wasser gelöst und 1.9g (8.1 mmol) $N^4$-(2-methoxyethyl)-$N^1$,$N^1$,$N^4$-trimethyl-2-oxobutandiamid zugegeben. Man rührte 15 Minuten bei Raumtemperatur, gab 1.47g (10.8mmol) Natriumacetat trihydrat zu und liess weitere 20min rühren. Man extrahierte die Reaktionsmischung dreimal mit 50ml Chloroform, trocknete über Natriumsulfat und engte zur Trockene ein. Der Rückstand wurde zweimal mit 50ml Toluol abgeschleppt. Man trocknete für drei Tage bei 50°C im Feinvakuum. Man erhielt 1.8g Produkt in einer Reinheit von 80%.

IR (in Substanz, $cm^{-1}$): 3487, 2929, 1719, 1635, 1602, 1564, 1514, 1486, 1391, 1359, 1302, 1261, 1197, 1164, 1112, 1064, 1033, 1004, 964, 906, 889, 827, 807, 770, 713, 664.
Elementaranalyse: C 48.17%, H 6.9%, N 10.98% .
Fe-Gehalt: 6.0% [m/m].

**14. Tris-($N^4$-(2-methoxyethyl)-$N^1$,$N^1$-dimethyl-2-oxobutandiamid)-eisen(III)-Komplex**

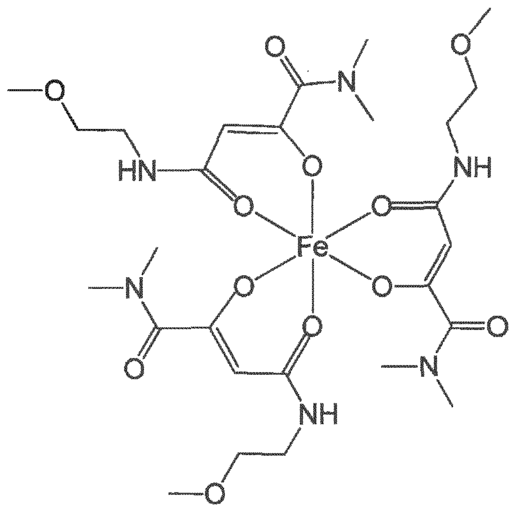

358mg (2.20mmol) Eisen(III)chlorid wurden in 16ml Wasser gelöst und 1.43g (6.61mmol) $N^4$-(2-methoxyethyl)-$N^1$,$N^1$-dimethyl-2-oxobutandiamid zugegeben. Man rührte 15 Minuten bei Raumtemperatur, gab 1.20g (8.82mmol) Natriumacetat trihydrat zu und liess weitere 30min rühren. Man extrahierte die Reaktionsmischung fünfmal mit Chloroform, trocknete über Natriumsulfat und engte zur Trockene ein. Der Rückstand wurde zweimal mit 50ml Toluol abgeschleppt. Man trocknete für drei Tage bei 50°C im Feinvakuum. Man erhielt 1.39g Produkt in einer Reinheit von 83%.
IR (in Substanz, $cm^{-1}$): 3274, 3119, 2930, 1720, 1622, 1569, 1523, 1491, 1437, 1393, 1273, 1193, 1118, 1085, 1025, 962, 861, 777, 721, 691 .
Elementaranalyse: C 45.27%, H 6.5%, N 11.64%.
Fe-Gehalt: 6.6% [m/m].

**15. Tris-(ethyl-N-[4-(dimethylamino)-3,4-dioxobutanoyl]-N-methylglycinat)-eisen(III)-Komplex**

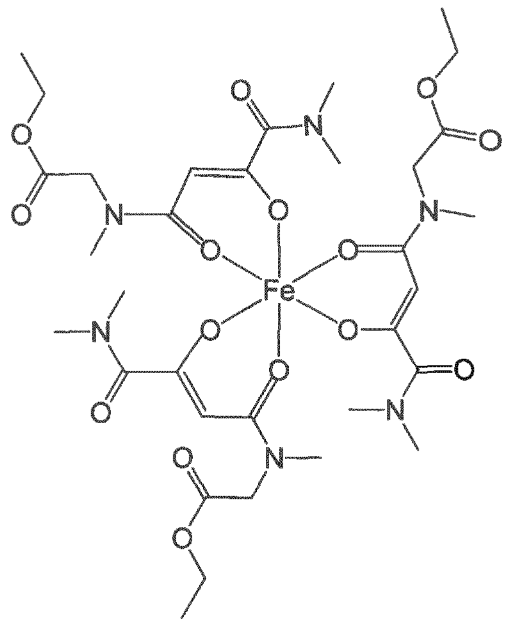

237mg (1.46mmol) Eisen(III)chlorid wurden in 16ml Wasser gelöst und 1.13g (4.38mmol) Ethyl-N-[4-(dimethylamino)-3,4-dioxobutanoyl]-N-methylglycinat zugegeben. Man rührte 15 Minuten bei Raumtemperatur, gab 794mg (5.84mmol) Natriumacetat trihydrat zu und liess weitere 30min rühren. Man extrahierte die Reaktionsmischung fünfmal mit Chloroform, trocknete über Natriumsulfat und engte zur Trockene ein. Der Rückstand wurde zweimal mit 50ml Toluol abgeschleppt. Man trocknete für drei Tage bei 50°C im Feinvakuum und erhielt 860mg (71%) Produkt.
IR (in Substanz, $cm^{-1}$): 3468, 2936, 2324, 2051, 1981, 1739, 1634, 1603, 1563, 1515, 1487, 1444, 1392, 1362, 1294, 1256, 1195, 1149, 1113, 1044, 1022, 964, 905, 861, 817, 764, 707, 667.
Elementaranalyse: C 46.67%, H 6.1%, N 9.80% .
Fe-Gehalt: 6.6% [m/m].

**16. Tris-($N^1$-(2-methoxyethyl)-$N^4$,$N^4$-dimethyl-2-oxobutanamid)-eisen(III)-Komplex**

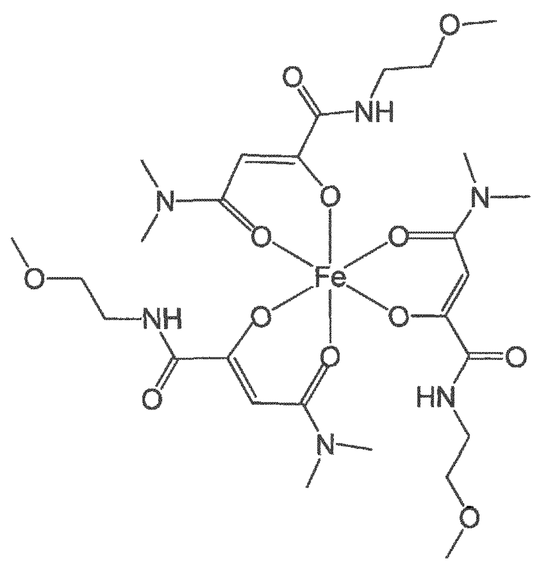

0.49g (3.0mmol) Eisen(III)chlorid wurden in 10ml Wasser gelöst und 2.0g (9.1 mmol) $N^1$-(2-methoxyethyl)-$N^4$,$N^4$-dimethyl-2-oxobutanamid zugegeben. Man rührte 15 Minuten bei Raumtemperatur und kühlte die Reaktionsmischung anschliessend im Eisbad ab. Man gab 1.66g (12.2mmol) Natriumacetat trihydrat zu und liess weitere 15 Minuten rühren. Man extrahierte die Reaktionsmischung dreimal mit Chloroform, trocknete über Natriumsulfat und engte

zur Trockene ein. Der Rückstand wurde zweimal mit 50ml Toluol abgeschleppt. Man trocknete über Nacht bei 50°C im Feinvakuum. Man erhielt 1.5g (70%) Produkt in Form eines roten Feststoffes.
IR (in Substanz, $cm^{-1}$): 3397, 2929, 2885, 1671, 1619, 1580, 1516, 1480, 1403, 1353, 1259, 1173, 1149, 1117, 1088, 1026, 1001, 941, 901, 881, 815, 769, 734.
Elementaranalyse: C 44.99%, H 6.6%, N 11.43% .
Fe-Gehalt: 7.7% [m/m].

**17. Tris-(ethyl-N-[4-(dimethylamino)-2,4-dioxobutanoyl]-N-methylglycinat)-eisen(III)-Komplex**

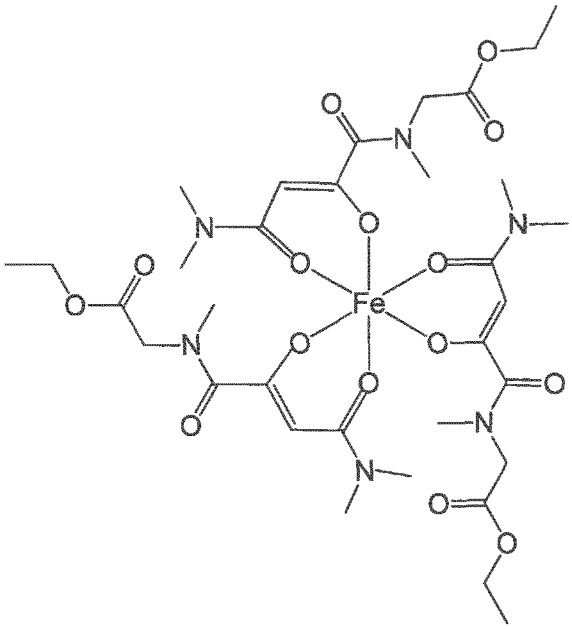

0.21g (1.3mmol) Eisen(III)chlorid wurden in 10ml Wasser gelöst und 1.02g (3.8mmol) Ethyl-N-[4-(dimethylamino)-2,4-dioxobutanoyl]-N-methylglycinat zugegeben. Man rührte 15 Minuten bei Raumtemperatur und kühlte die Reaktionsmischung anschliessend im Eisbad ab. Man gab 0.71g (5.2mmol) Natriumacetat trihydrat zu und liess weitere 15 Minuten rühren. Man extrahierte die Reaktionsmischung dreimal mit 50ml Chloroform, trocknete über Natriumsulfat und engte zur Trockene ein. Der Rückstand wurde zweimal mit 50ml Toluol abgeschleppt. Man trocknete über Nacht bei 50°C im Feinvakuum. Man erhielt 0.9g (82%) Produkt in Form eines roten Öls.
IR (in Substanz, $cm^{-1}$): 3485, 2981, 2936, 1738, 1641, 1601, 1571, 1508, 1477, 1444, 1397, 1357, 1297, 1258, 1199, 1176, 1105, 1027, 989, 951, 905, 865, 814, 766, 723, 662.
Elementaranalyse: C 47.10%, H 6.7%, N 9.11%.
Fe-Gehalt: 6.6% [m/m].

**18. Tris-($N^1$-[2-(dimethylamino)-2-oxoethyl]-$N^1$,$N^4$,$N^4$-trimethyl-2-oxobutandiamid)-eisen(III)-Komplex**

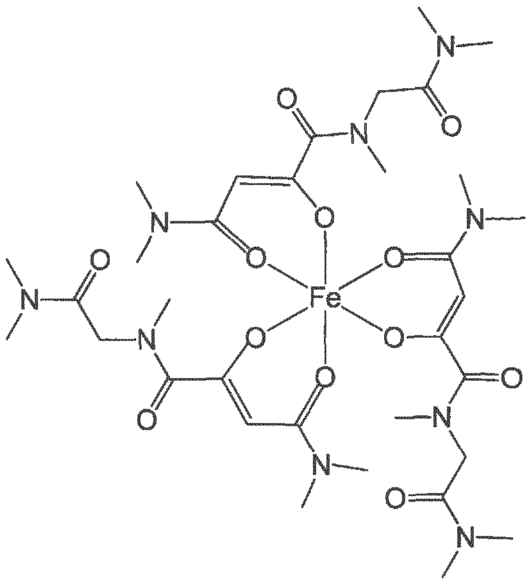

0.11g (0.70mmol) Eisen(III)chlorid wurden in 10ml Wasser gelöst und 0.54g (2.1mmol) $N^1$-[2-(dimethylamino)-2-oxoethyl]-$N^1$,$N^4$,$N^4$-trimethyl-2-oxobutandiamid in 0.1ml Ethanol zugegeben. Man rührte 15 Minuten bei Raumtemperatur und gab 0.38g (2.8mmol) Natriumacetat trihydrat zu und liess weitere 15 Minuten rühren. Man extrahierte die Reaktionsmischung fünfmal mit Chloroform, trocknete über Natriumsulfat und engte zur Trockene ein. Der Rückstand wurde zweimal mit 50ml Toluol abgeschleppt. Man trocknete über Nacht bei 50°C im Feinvakuum. Man erhielt 0.52g Produkt in einer Reinheit von 85%.
IR (in Substanz, $cm^{-1}$): 3455, 2934, 1779, 1630, 1572, 1508, 1479, 1398, 1357, 1303, 1259, 1178, 1152, 1106, 1060, 1027, 989, 945, 905, 823, 804, 765, 720.
Elementaranalyse: C 43.02%, H 6.9%, N 13.05% .
Fe-Gehalt: 5.8% [m/m].

**19. Tris-(ethyl-N-[4-(dimethylamino)-2,4-dioxobutanoyl]glycinat)-eisen(III)-Komplex**

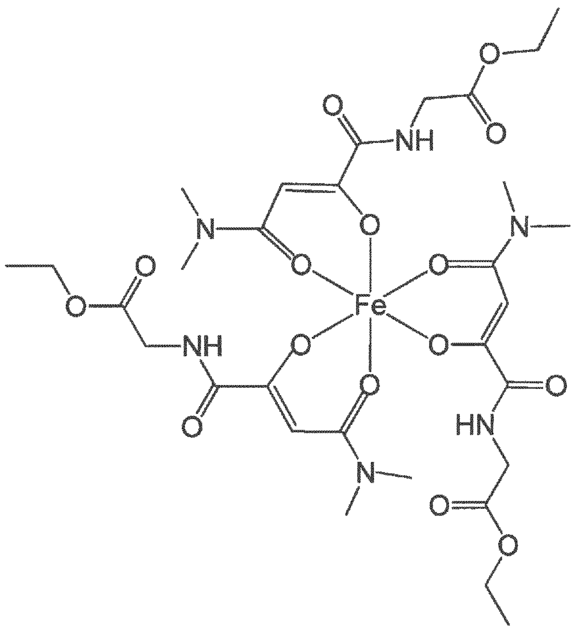

97mg (0.60mmol) Eisen(III)chlorid wurden in 10ml Wasser gelöst und 459mg (1.80mmol) Ethyl N-[4-(dimethylamino)-2,4-dioxobutanoyl]glycinat zugegeben. Man rührte 15 Minuten bei Raumtemperatur und kühlte die Reaktionsmischung anschliessend im Eisbad ab. Man gab 327mg (2.40mmol) Natriumacetat trihydrat zu und liess weitere 20 Minuten rühren. Der ausgefallene Feststoff wurde abfiltriert und über Nacht bei 50°C im Feinvakuum getrocknet. Man erhielt 0.35g (71%) Produkt in Form eines orangen Feststoffs.
IR (in Substanz, $cm^{-1}$): 3396, 3317, 2983, 2938, 2289, 2051, 1760, 1743, 1723, 1668, 1625, 1588, 1483, 1428, 1406, 1361, 1280, 1256, 1201, 1178, 1095, 1061, 1022, 994, 931, 873, 853, 822, 770, 751.
Elementaranalyse: C 44.88%, H 5.9%, N 10.4% .
Fe-Gehalt: 6.8% [m/m].

**20. Tris-($N^1$-(2-methoxyethyl)-$N^1$,$N^4$,$N^4$-trimethyl-2-oxobutandiamid)-eisen(III)-Komplex**

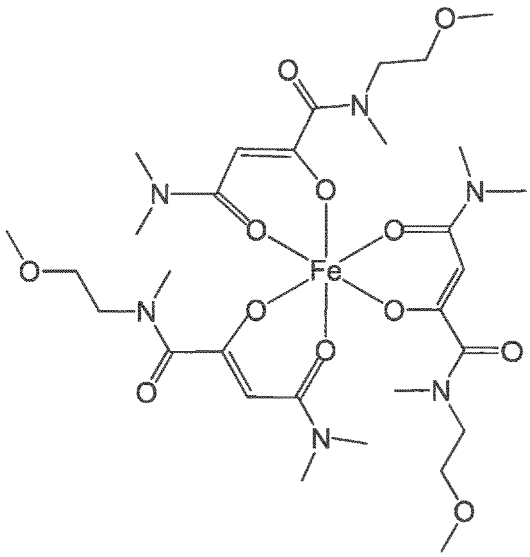

0.46g (2.8mmol) Eisen(III)chlorid wurden in 20ml Wasser gelöst und 2.0g (8.5mmol) $N^1$-(2-methoxyethyl)-$N^1$,$N^4$,$N^4$-trimethyl-2-oxobutandiamid zugegeben. Man rührte 15 Minuten bei Raumtemperatur und kühlte die Reaktionsmischung anschliessend im Eisbad ab. Man gab 1.5g (11 mmol) Natriumacetat trihydrat zu und liess weitere 20 Minuten rühren. Man extrahierte die Reaktionsmischung dreimal mit 50ml Chloroform, trocknete über Natriumsulfat und engte zur Trockene ein. Der Rückstand wurde zweimal mit 50ml Toluol abgeschleppt. Man trocknete über Nacht bei 50°C im Feinvakuum. Man erhielt 2.0g (93%) Produkt in Form eines roten Feststoffs.
IR (in Substanz, $cm^{-1}$): 3492, 2929, 1633, 1605, 1568, 1510, 1479, 1449, 1427, 1398, 1354, 1287, 1260, 1200, 1175, 1108, 1067, 1006, 977, 938, 904, 892, 827, 809, 769, 712, 662.
Elementaranalyse: C 48.06%, H 7.0%, N 11.0% .
Fe-Gehalt: 6.9% [m/m].

**21. Tris-($N^4$-(2-hydroxyethyl)-$N^1$,$N^1$,$N^4$-trimethyl-2-oxobutandiamid)-eisen(III)-Komplex**

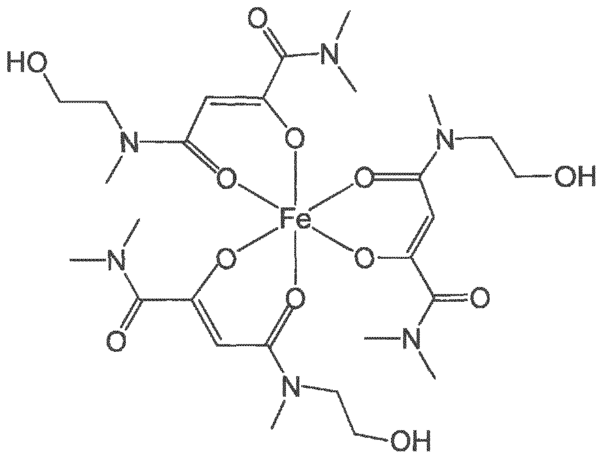

0.20g (1.0 mmol) Eisen(III)acetat wurden in 10ml Ethylacetat suspendiert und 0.65g (3.0 mmol) $N^4$-(2-hydroxyethyl)-$N^1$,$N^1$,$N^4$-trimethyl-2-oxobutandiamid zugegeben. Die Reaktionsmischung wurde auf 50°C erwärmt und 20ml Ethanol zugegeben. Man rührt für 20 Minuten und engte die Reaktionsmischung zur Trockene ein. Der Rückstand wurde noch zweimal in Ethanol/Toluol-Mischung (5ml/50ml) aufgenommen und zur Trockene eingeengt. Der Rückstand wurde bei 50°C im Feinvakuum getrocknet. Man erhielt 0.7 g (99% Ausbeute) Produkt als roten Feststoff.
IR (in Substanz, $cm^{-1}$): 3380, 2931, 1621, 1604, 1567, 1516, 1485, 1441, 1396, 1358, 1298, 1260, 1207, 1117, 1051, 1013, 964, 940, 906, 889, 860, 805, 770, 713, 663.
Elementaranalyse: C 44.50%, H 6.7%, N 10.85% .
Fe-Gehalt: 8.3% [m/m].

## Patentansprüche

1. Eisen(III)-2-oxo-butandiamid-Komplexverbindungen oder pharmazeutische verträgliche Salze davon zur Anwendung in der Behandlung und Prophylaxe von Eisenmangelerscheinungen und Eisenmangelanämien.

2. Eisen(III)-Komplexverbindungen zur Anwendung in der Behandlung und Prophylaxe von Eisenmangelerscheinungen und Eisenmangelanämien nach Anspruch 1, enthaltend mindestens einen Liganden der Formel (I):

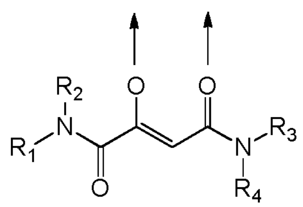

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können, oder
$R_1$ und $R_2$ gemeinsam mit dem Stickstoffatom, an welches sie gebunden sind, einen gegebenenfalls substituierten 3- bis 6-gliedrigen Ring bilden, der gegebenenfalls ein weiteres Heteroatom aufweisen kann,
$R_3$ und $R_4$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können, oder
$R_3$ und $R_4$ gemeinsam mit dem Stickstoffatom, an welches sie gebunden sind, einen gegebenenfalls substituierten 3- bis 6-gliedrigen Ring bilden, der gegebenenfalls ein weiteres Heteroatom aufweisen kann.

**3.** Eisen(III)-Komplexverbindungen zur Anwendung in der Behandlung und Prophylaxe von Eisenmangelerscheinungen und Eisenmangelanämien nach Anspruch 1 oder 2, enthaltend mindestens einen Liganden der Formel (I):

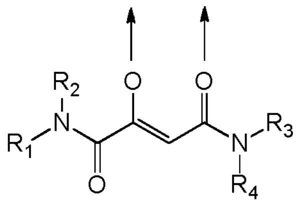

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, i-Propyl, n-Butyl, sek-Butyl und tert-Butyl, und worin die genannten Alkylgruppen substituiert sein können mit einem Substituenten, der ausgewählt wird aus der Gruppe, die besteht aus: Alkoxy, Alkoxycarbonyl, Aminocarbonyl ($H_2NCO$-), Monoalkylaminocarbonyl und Dialkylaminocarbonyl, oder
$R_1$ und $R_2$ zusammen mit dem Stickstoffatom, an das sie gebunden sind, einen gegebenenfalls substituierten 5- bis 6-gliedrigen Ring bilden, der gegebenenfalls ein weiteres Heteroatom aufweisen kann,
$R_3$ und $R_4$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein weiteres Heteroatom aufweisen kann.

**4.** Eisen(III)-Komplexverbindungen zur Anwendung in der Behandlung und Prophylaxe von Eisenmangelerscheinungen und Eisenmangelanämien nach Anspruch 1 oder 2, enthaltend mindestens einen Liganden der Formel (I):

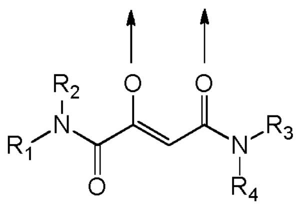

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, i-Propyl, n-Butyl, sek-Butyl und tert-Butyl, und worin die Alkylgruppen substituiert sein können mit einem Substituenten, der ausgewählt wird aus der Gruppe, die besteht aus: Alkoxy, Alkoxycarbonyl, Aminocarbonyl ($H_2NCO$-), Monoalkylaminocarbonyl und Dialkylaminocarbonyl, oder
$R_1$ und $R_2$ zusammen mit dem Stickstoffatom, an das sie gebunden sind, Pyrrolidinyl bilden,
$R_3$ und $R_4$ gleich oder verschieden sind, und jeweils ausgewählt werden aus der Gruppe, die besteht aus Wasserstoff, Alkyl und Cycloalkyl, worin Alkyl und Cycloalkyl substituiert sein können mit einem Substituenten, der ausgewählt wird aus der Gruppe, die besteht aus: Hydroxy, Alkoxy, Alkoxycarbonyl, Aminocarbonyl ($H_2NCO$-), Monoalkylaminocarbonyl und Dialkylaminocarbonyl,
$R_3$ und $R_4$ gemeinsam mit dem Stickstoffatom, an das sie gebunden sind, Morpholinyl oder Pyrrolidinyl bilden, worin die genannten Ringgruppen mit Hydroxy substituiert sein können.

**5.** Eisen(III)-Komplexverbindungen zur Anwendung in der Behandlung und Prophylaxe von Eisenmangelerscheinungen und Eisenmangelanämien nach Anspruch 1 oder 2, enthaltend mindestens einen Liganden der Formel (I):

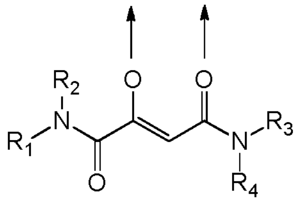

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff, Methyl, Ethyl, Propyl, i-Propyl, n-Butyl, sek-Butyl und tert-Butyl,
$R_3$ und $R_4$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können, oder
$R_3$ und $R_4$ gemeinsam mit dem Stickstoffatom, an das sie gebunden sind, einen gegebenenfalls substituierten 3- bis 6-gliedrigen Ring bilden, der gegebenenfalls ein weiteres Heteroatom aufweisen kann.

**6.** Eisen(III)-Komplexverbindungen zur Anwendung in der Behandlung und Prophylaxe von Eisenmangelerscheinungen und Eisenmangelanämien nach einem der Ansprüche 1, 2 oder 5, enthaltend mindestens einen Liganden der Formel (I):

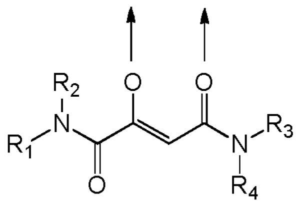

(I)

worin
die Pfeile jeweils eine koordinative Bindung zu einem oder verschiedenen Eisenatomen darstellen,

$R_1$ und $R_2$ gleich oder verschieden sind, und jeweils ausgewählt werden aus der Gruppe, die besteht aus Wasserstoff und Methyl,
$R_3$ und $R_4$ gleich oder verschieden sind, und jeweils ausgewählt werden aus der Gruppe, die besteht aus Wasserstoff, gegebenenfalls substituiertem Alkyl und gegebenenfalls substituiertem Cycloalkyl, wobei in den genannten Alkylgruppen gegebenenfalls ein oder zwei Kohlenstoffatome durch Sauerstoff ersetzt sein können.

**7.** Eisen(III)-Komplexverbindungen zur Anwendung in der Behandlung und Prophylaxe von Eisenmangelerscheinungen und Eisenmangelanämien nach einem der Ansprüche 1 bis 6 der Formel (II):

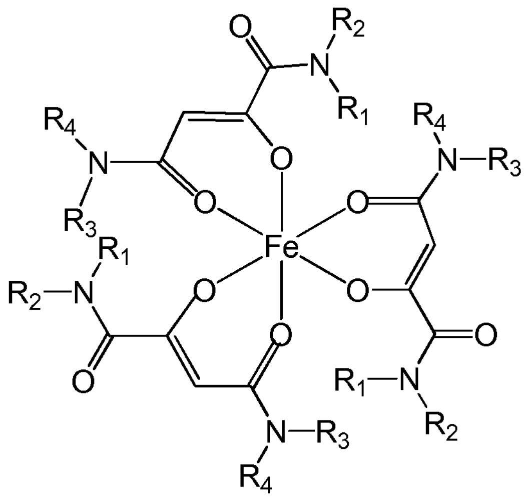

(II)

worin $R_1$, $R_2$, $R_3$ und $R_4$ wie oben definiert sind.

**8.** Eisen(III)-Komplexverbindungen zur Anwendung in der Behandlung und Prophylaxe von Eisenmangelerscheinungen und Eisenmangelanämien nach einem der Ansprüche 2, 5 bis 7, worin
$R_1$ und $R_2$ jeweils Wasserstoff ist, und
$R_3$ und $R_4$ gleich oder verschieden sind ausgewählt wird aus der Gruppe, die besteht aus Methyl und Ethyl.

**9.** Eisen(III)-Komplexverbindungen oder pharmazeutische verträgliche Salze davon zur Anwendung in der Behandlung und Prophylaxe von Eisenmangelerscheinungen und Eisenmangelanämien nach einem der Ansprüche 1 bis 8, worin die Eisen(III)-Komplexverbindungen ausgewählt werden aus der Gruppe, die besteht aus:

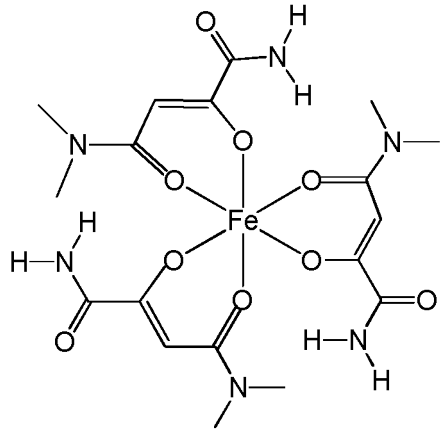

,

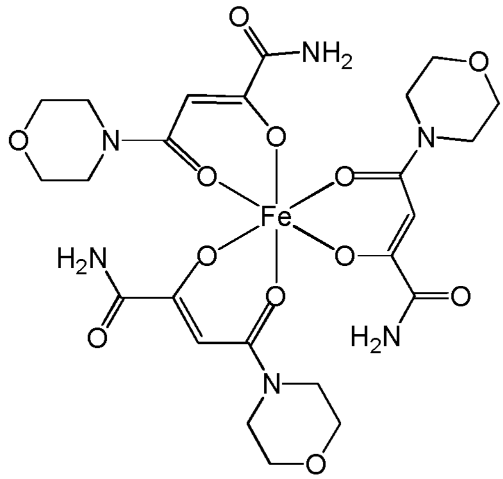

,

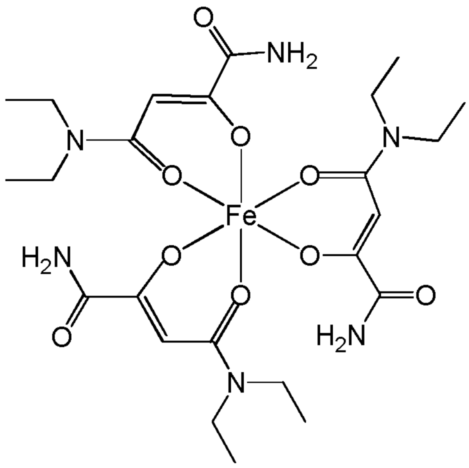

,

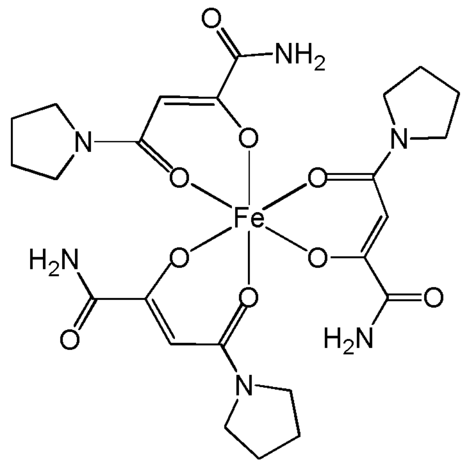

,

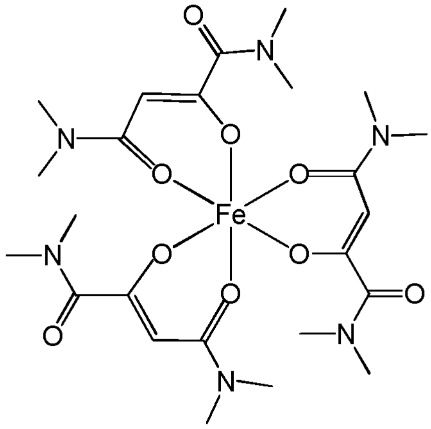

,

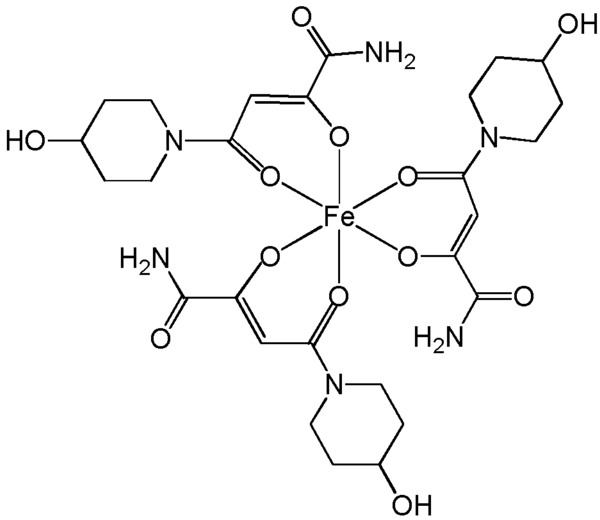

,

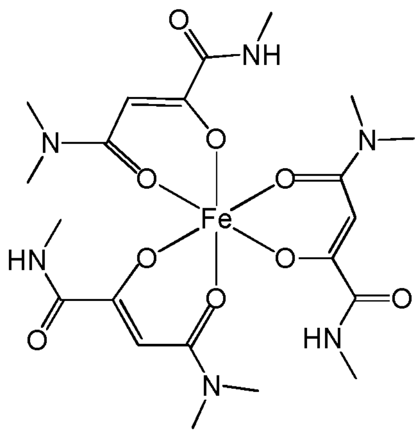

,

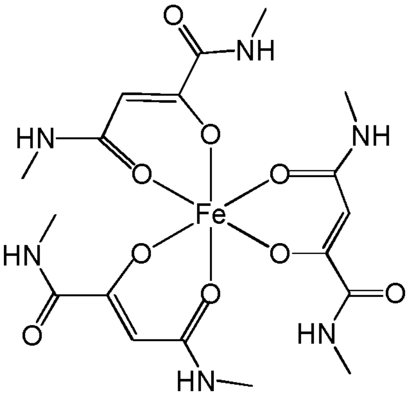

,

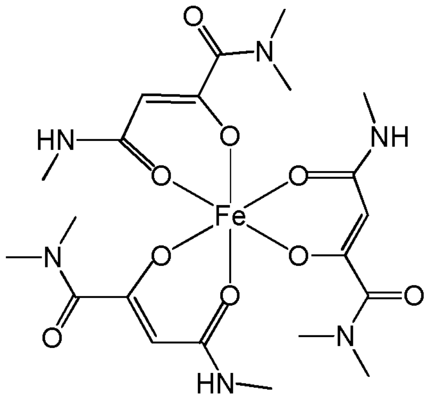

,

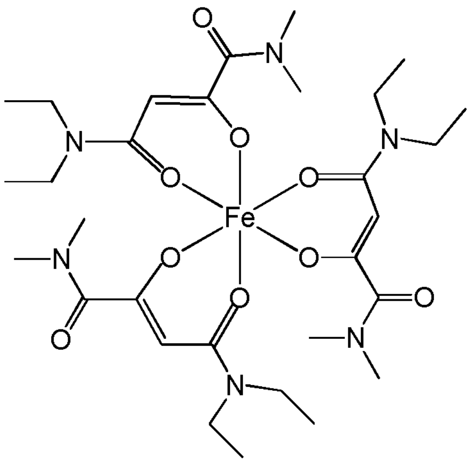

,

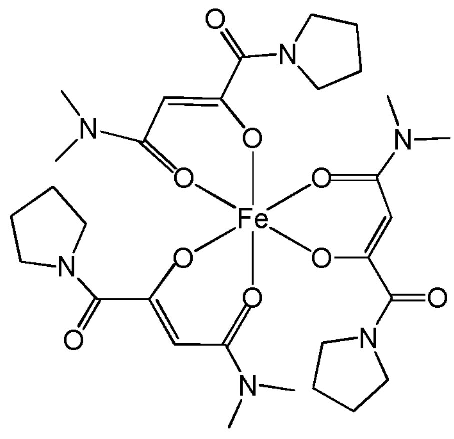

,

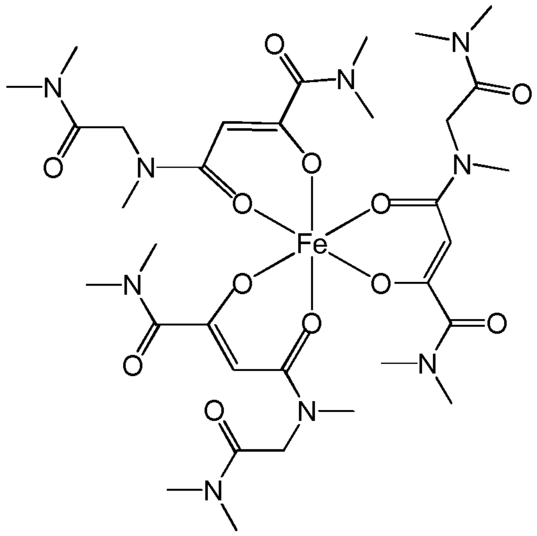

,

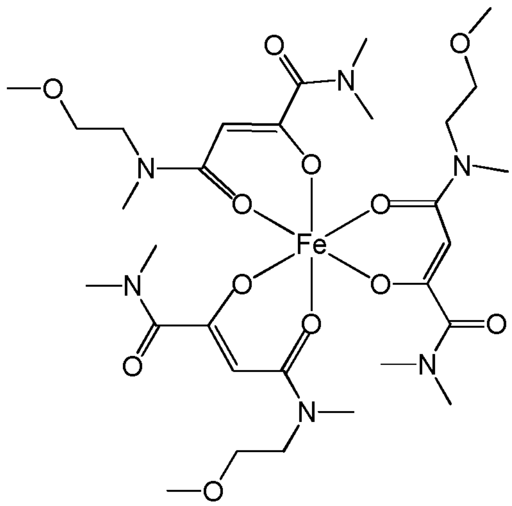

,

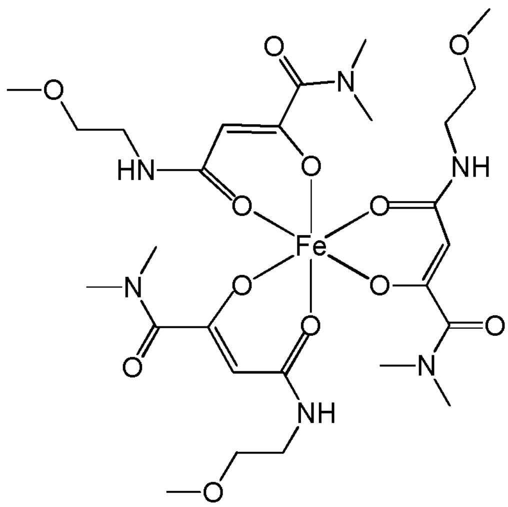

,

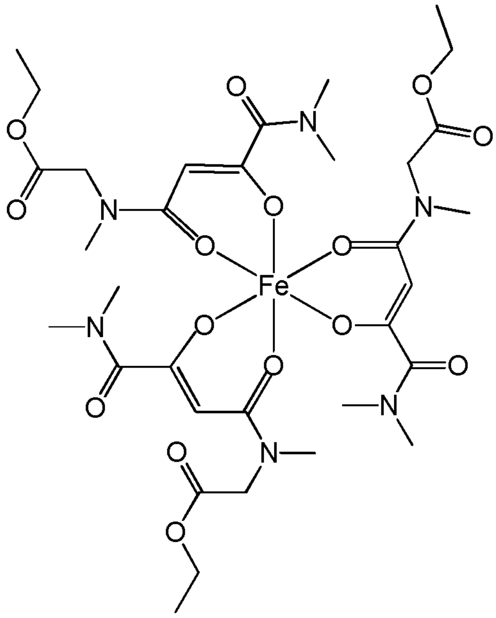

,

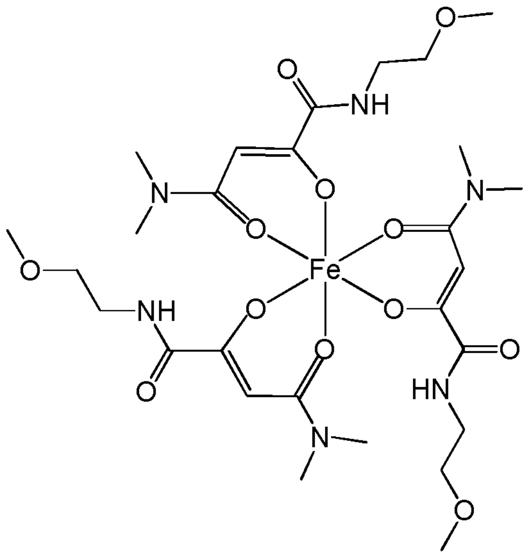

,

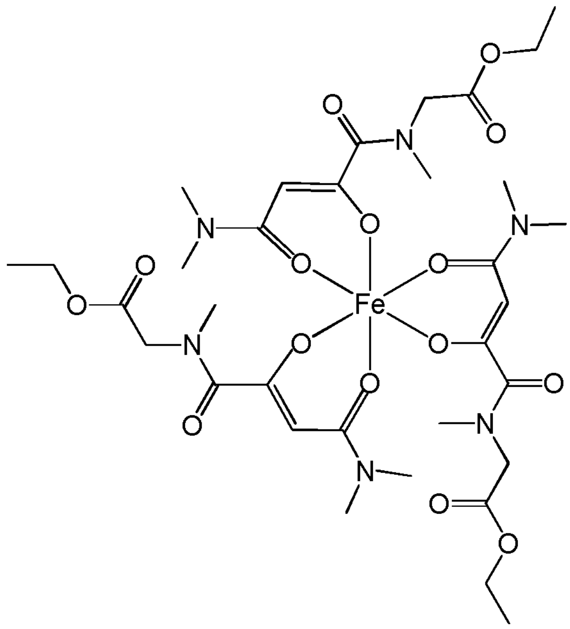

,

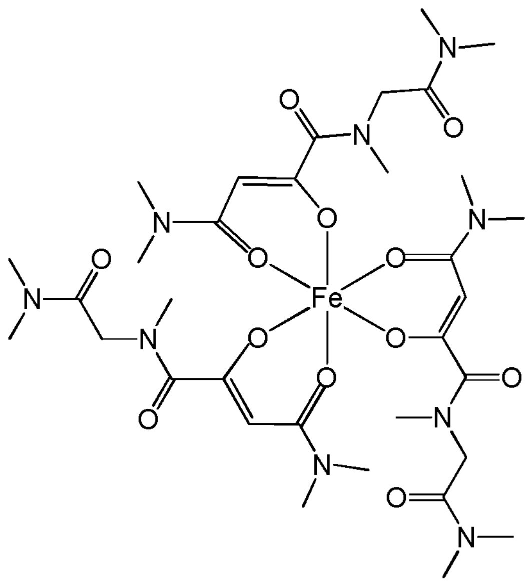

,

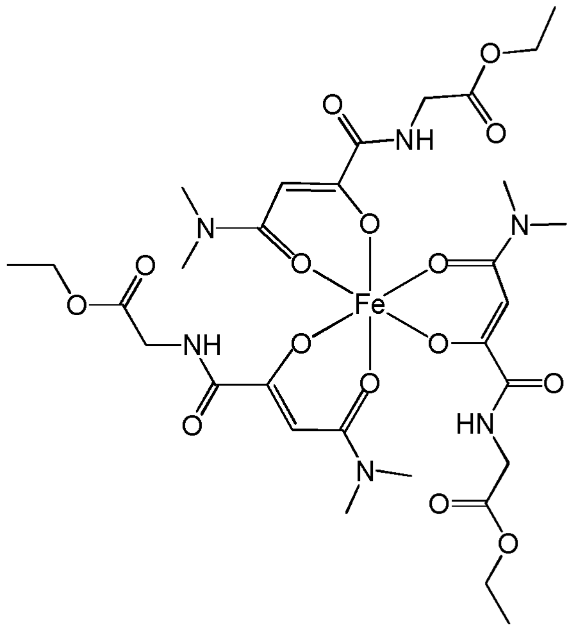

,

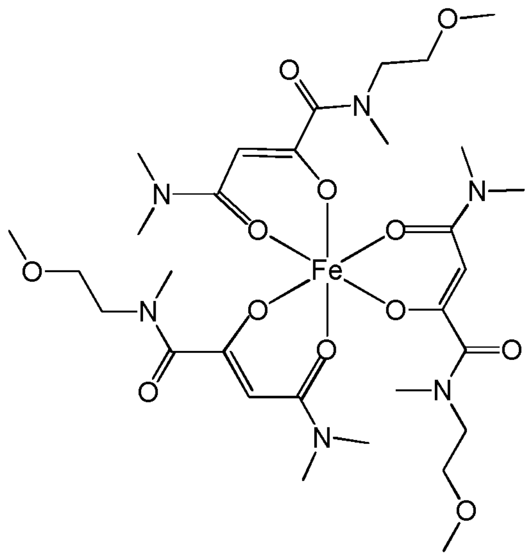

,

und

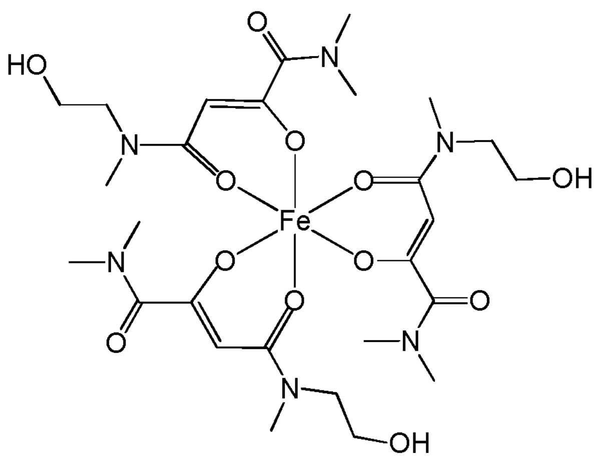

.

**10.** Eisen(III)-Komplexverbindungen oder pharmazeutische verträgliche Salze davon zur Anwendung in der Behandlung und Prophylaxe von Eisenmangelerscheinungen und Eisenmangelanämien nach einem der Ansprüche 1 bis 9 mit einer Löslichkeit in Wasser bei 25°C bei pH 6.5 von mindestens 0.3 mg/ml bezogen auf Eisen.

**11.** Eisen(III)-Komplexverbindungen zur Anwendung in der Behandlung und Prophylaxe von Eisenmangelerscheinungen und Eisenmangelanämien nach einem der Ansprüche 1 bis 10 zur Behandlung und Prophylaxe der Symptome von Eisenmangelerscheinungen und Eisenmangelanämien,
worin die Symptome insbesondere einschließen: Ermüdung, Antriebslosigkeit, Konzentrationsschwäche, geringe kognitive Effizienz, Schwierigkeiten beim Finden der richtige Worte, Vergesslichkeit, unnatürliche Blässe, Reizbarkeit, Beschleunigung der Herzfrequenz (Tachykardie), wunde oder geschwollene Zunge, vergrößerte Milz, Schwangerengelüste (Pica), Kopfschmerzen, Appetitlosigkeit, erhöhte Infektionsanfälligkeit, depressive Verstimmungen.

**12.** Eisen(III)-Komplexverbindungen zur Anwendung in der Behandlung und Prophylaxe von Eisenmangelerscheinungen und Eisenmangelanämien nach einem der Ansprüche 1 bis 11 zur Behandlung und Prophylaxe der Eisenmangelanämie bei Schwangeren, der latenten Eisenmangelanämie bei Kindern und Heranwachsenden, der Eisenmangelanämie infolge gastrointestinaler Abnormalitäten, der Eisenmangelanämie infolge von Blutverlusten, wie durch gastrointestinale Blutungen infolge von Geschwüren, Karzinomen, Hämorriden, entzündlichen Störungen, sowie der Einnahme von Acetylsalicylsäure, der Eisenmangelanämie infolge von Menstruation, der Eisenmangelanämie infolge von Verletzungen, der Eisenmangelanämie infolge von Psilosis (sprue), der Eisenmangelanämie infolge verminderter Eisenaufnahme bei der Ernährung, insbesondere bei selektiv essenden Kindern und Heranwachsenden, der Immunschwäche hervorgerufen durch Eisenmangelanämie, der Beeinträchtigung der Hirnleistung hervorgerufen durch Eisenmangelanämien, des Restless Leg Syndrom hervorgerufen durch Eisenmangelanämien, Eisenmangelanämien bei Krebs, Eisenmangelanämien ausgelöst durch Chemotherapien, Eisenmangelanämien ausgelöst durch Inflammation (AI), Eisenmangelanämien bei kongestiver Herzinsuffizienz (CHF;congestive heart failure), Eisenmangelanämien bei chronischer Niereninsuffizienz Stadium 3-5 (CKD 3-5; chronic kidney diseases stage 3-5), Eisenmangelanämien ausgelöst durch chronische Inflammation (ACD), Eisenmangelanämien bei rheumatischer Arthritis (RA; rheumatoid arthritis), Eisenmangelanämien bei systemischem Lupus erythemotodes (SLE; systemic lupus erythematosus), Eisenmangelanämien bei inflammatorischen Darmerkrankungen (IBD; inflammatory bowel diseases), des Eisenmangels bei Haemoglobin-Werten im Normbereich.

**13.** Eisen(III)-Komplexverbindungen zur Anwendung in der Behandlung und Prophylaxe von Eisenmangelerscheinungen und Eisenmangelanämien nach einem der Ansprüche 1 bis 12 zur oralen Verabreichung, insbesondere zur Verabreichung als Tablette oder Kapsel.

**14.** Arzneimittel zur Anwendung in der Behandlung und Prophylaxe von Eisenmangelerscheinungen und Eisenmangelanämien, enthaltend Eisen(III)-Komplexverbindungen wie in einem der Ansprüche 1 bis 10 definiert.

**15.** Arzneimittel zur Anwendung in der Behandlung und Prophylaxe von Eisenmangelerscheinungen und Eisenmangelanämien, enthaltend Eisen(III)-Komplexverbindungen wie in einem der Ansprüche 1 bis 10 definiert sowie mindestens einen physiologisch verträglichen Träger bzw. Exzipienten und/oder mindestens ein weiteres Arzneimittel welches auf den Eisenmetabolismus wirkt.

## Claims

**1.** Iron(III)-2-oxo-butanediamide complex compounds or pharmaceutically acceptable salts thereof for use in the treatment and prophylaxis of iron deficiency diseases and iron deficiency anemias.

**2.** Iron(III) complex compounds for the use in the treatment and prophylaxis of iron deficiency diseases and iron deficiency anemias according to claim 1, comprising at least one ligand of the formula (I):

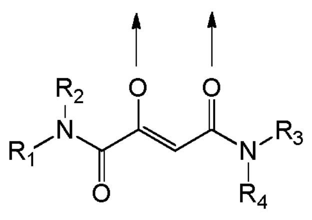

(I)

wherein
the arrows each represent a coordinate bond to one or different iron atoms,

$R_1$ and $R_2$ are the same or different and are individually selected from the group consisting of hydrogen, optionally substituted alkyl, and optionally substituted cycloalkyl, wherein in said alkyl groups optionally one or two carbon atoms may be replaced by oxygen, or
$R_1$ and $R_2$ together with the nitrogen atom, to which they are bonded, form an optionally substituted 3- to 6-membered ring, which may optionally contain one further heteroatom,
$R_3$ and $R_4$ are the same or different and are individually selected from the group consisting of hydrogen, optionally substituted alkyl, and optionally substituted cycloalkyl, wherein in said alkyl groups optionally one or two carbon atoms may be replaced by oxygen, or
$R_3$ and $R_4$ together with the nitrogen atom, to which they are bonded, form an optionally substituted 3- to 6-membered ring, which may optionally contain one further heteroatom.

**3.** Iron(III) complex compounds for the use in the treatment and prophylaxis of iron deficiency diseases and iron deficiency anemias according to claim 1 or 2, comprising at least one ligand of the formula (I):

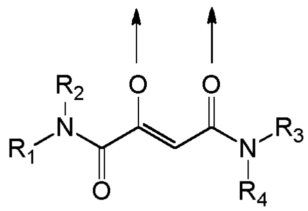

(I)

wherein
the arrows each represent a coordinate bond to one or different iron atoms,

$R_1$ and $R_2$ are the same or different and are individually selected from the group consisting of hydrogen, methyl, ethyl, propyl, i-propyl, n-butyl, sec-butyl and tert-butyl, and wherein said alkyl groups may be substituted with a substituent which is selected from the group consisting of alkoxy, alkoxycarbonyl, aminocarbonyl ($H_2NCO$-), monoalkylaminocarbonyl and dialkylaminocarbonyl, or
$R_1$ and $R_2$ together with the nitrogen atom, to which they are bonded, form an optionally substituted 5- to 6-membered ring, which may optionally contain one further heteroatom,
$R_3$ and $R_4$ are the same or different and are individually selected from the group consisting of hydrogen, optionally substituted alkyl, and optionally substituted cycloalkyl, wherein in said alkyl groups optionally one or two carbon atoms may be replaced by oxygen, or
$R_3$ and $R_4$ together with the nitrogen atom, to which they are bonded, form an optionally substituted 3- to 6-membered ring, which may optionally contain one further heteroatom.

**4.** Iron(III) complex compounds for the use in the treatment and prophylaxis of iron deficiency diseases and iron deficiency anemias according to claim 1 or 2, comprising at least one ligand of the formula (I):

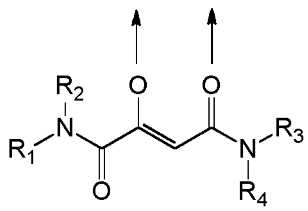

(I)

wherein
the arrows each represent a coordinate bond to one or different iron atoms,

$R_1$ and $R_2$ are the same or different and are individually selected from the group consisting of hydrogen, methyl, ethyl, propyl, i-propyl, n-butyl, sec-butyl and tert-butyl, and wherein said alkyl groups may be substituted with a substituent which is selected from the group consisting of alkoxy, alkoxycarbonyl, aminocarbonyl ($H_2NCO$-), monoalkylaminocarbonyl and dialkylaminocarbonyl, or

$R_1$ and $R_2$ together with the nitrogen atom, to which they are bonded, form pyrrolidinyl,
$R_3$ and $R_4$ are the same or different and are individually selected from the group consisting of hydrogen, alkyl, and cycloalkyl, wherein alkyl and cycloalkyl may be substituted with a substituent selected from the group consisting of: hydroxy, alkoxy, alkoxycarbonyl, aminocarbonyl ($H_2NCO$-), monoalkylaminocarbonyl and di-alkylaminocarbonyl,
$R_3$ and $R_4$ together with the nitrogen atom, to which they are bonded, form morpholinyl or pyrrolidinyl, wherein said ring groups may be substituted by hydroxy.

**5.** Iron(III) complex compounds for the use in the treatment and prophylaxis of iron deficiency diseases and iron deficiency anemias according to claim 1 or 2, comprising at least one ligand of the formula (I):

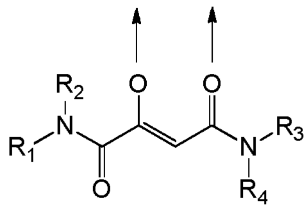

(I)

wherein
the arrows each represent a coordinate bond to one or different iron atoms,

$R_1$ and $R_2$ are the same or different and are individually selected from the group consisting of hydrogen, methyl, ethyl, propyl, i-propyl, n-butyl, sec-butyl and tert-butyl,
$R_3$ and $R_4$ are the same or different and are individually selected from the group consisting of hydrogen, optionally substituted alkyl, and optionally substituted cycloalkyl, wherein in said alkyl groups optionally one or two carbon atoms may be replaced by oxygen, or
$R_3$ and $R_4$ together with the nitrogen atom, to which they are bonded, form an optionally substituted 3- to 6-membered ring, which may optionally contain one further heteroatom.

**6.** Iron(III) complex compounds for the use in the treatment and prophylaxis of iron deficiency diseases and iron deficiency anemias according to one of claims 1, 2 or 5, comprising at least one ligand of the formula (I):

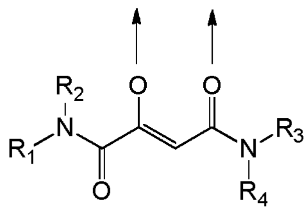

(I)

wherein
the arrows each represent a coordinate bond to one or different iron atoms,

$R_1$ and $R_2$ are the same or different and are individually selected from the group consisting of hydrogen and methyl,
$R_3$ and $R_4$ are the same or different and are individually selected from the group consisting of hydrogen, optionally substituted alkyl and optionally substituted cycloalkyl, wherein in said alkyl groups optionally one or two carbon atoms may be replaced by oxygen.

**7.** Iron(III) complex compounds for the use in the treatment and prophylaxis of iron deficiency diseases and iron deficiency anemias according to one of claims 1 to 6 of the formula (II):

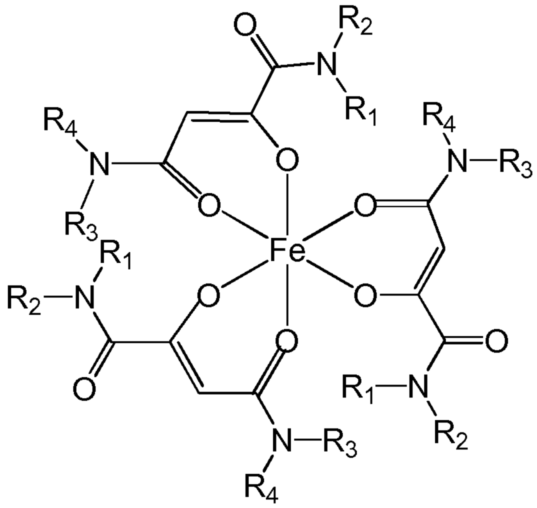

(II)

wherein $R_1$, $R_2$, $R_3$ and $R_4$ are as defined above.

**8.** Iron(III) complex compounds for the use in the treatment and prophylaxis of iron deficiency diseases and iron deficiency anemias according to one of claims 2, 5 to 7, wherein
$R_1$ and $R_2$ each are hydrogen, and
$R_3$ and $R_4$ are the same or different and are selected from the group consisting methyl and ethyl.

**9.** Iron(III) complex compounds or pharmaceutically acceptable salts thereof for the use in the treatment and prophylaxis of iron deficiency diseases and iron deficiency anemias according to one of claims 1 to 8, wherein the iron(III) complex compounds are selected from the group consisting of:

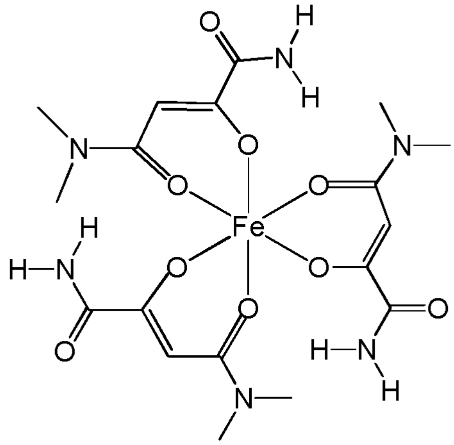

,

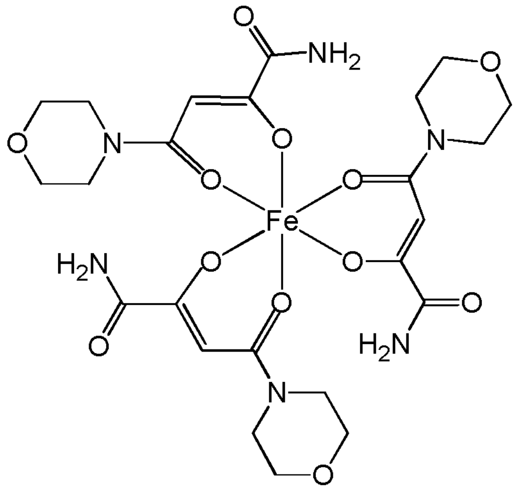

,

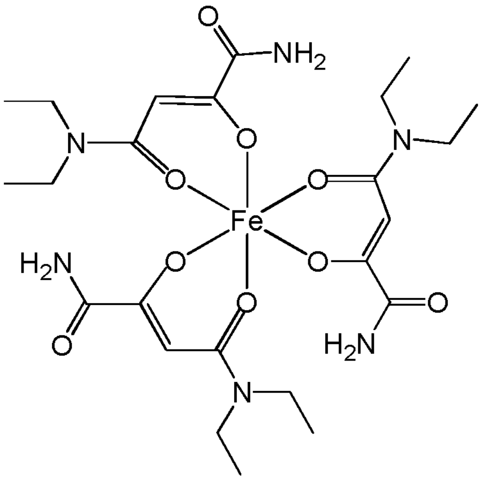

,

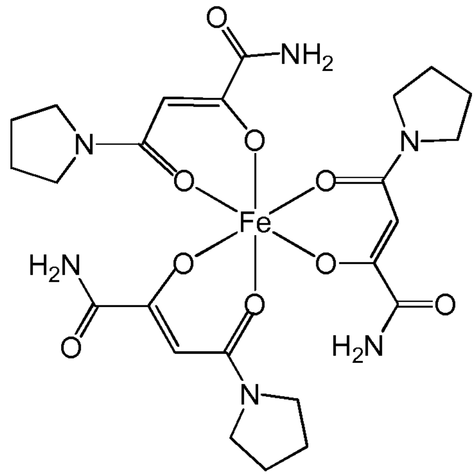

,

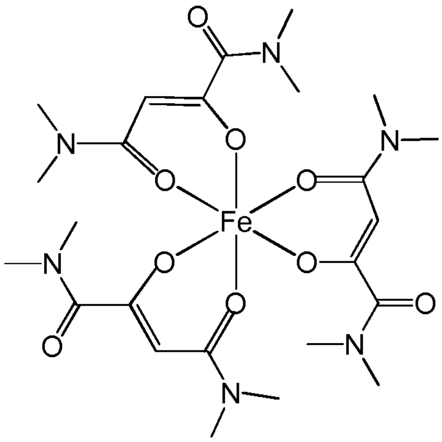

,

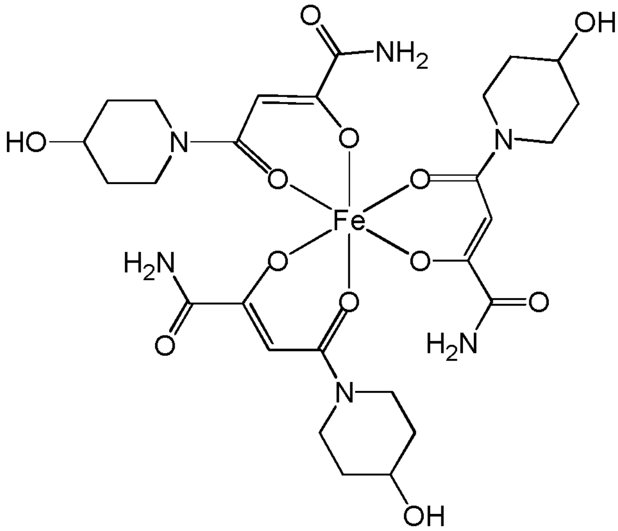

,

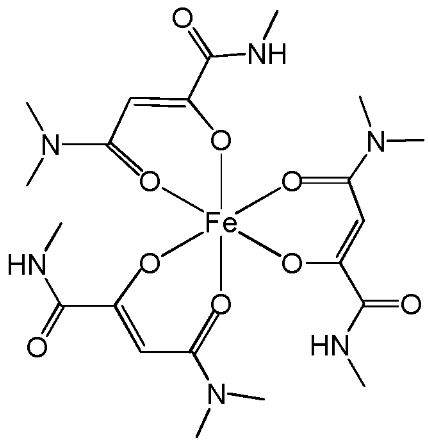

,

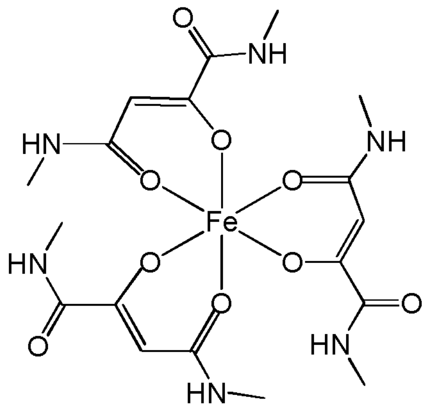

,

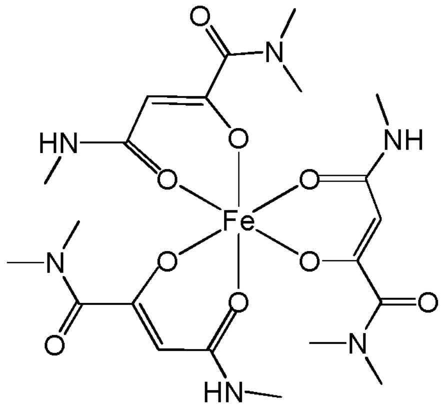

,

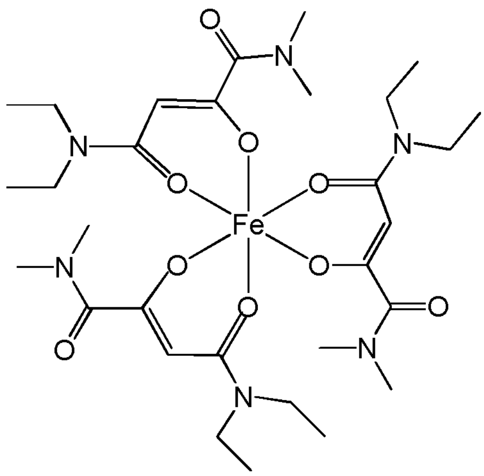

,

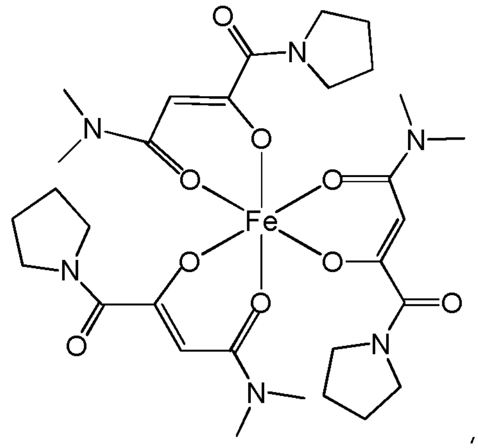

,

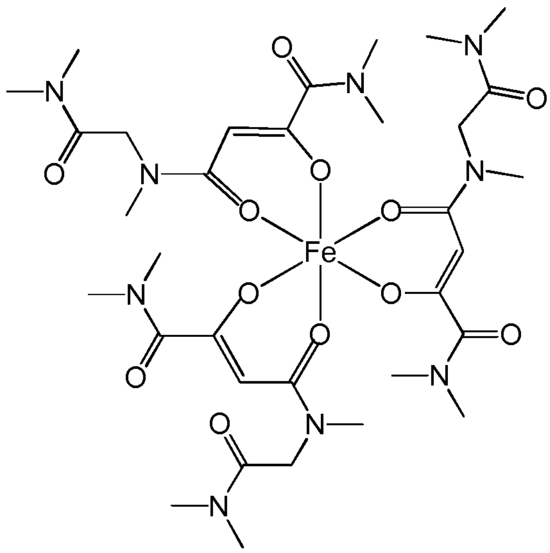

,

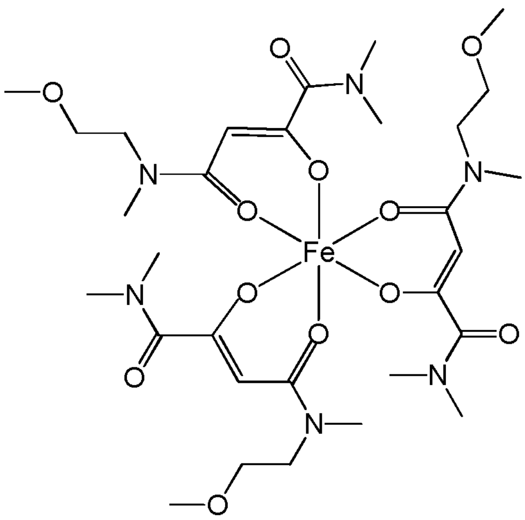

,

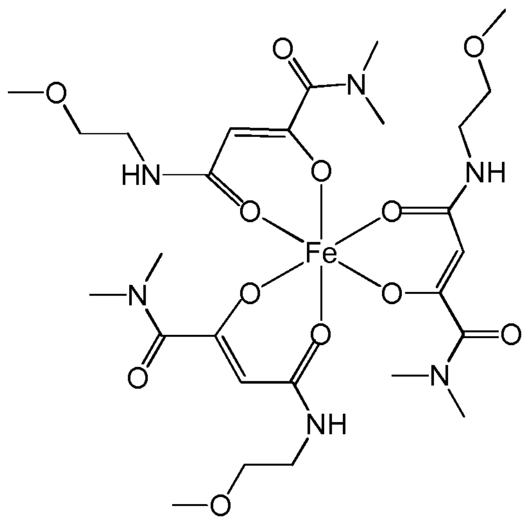

,

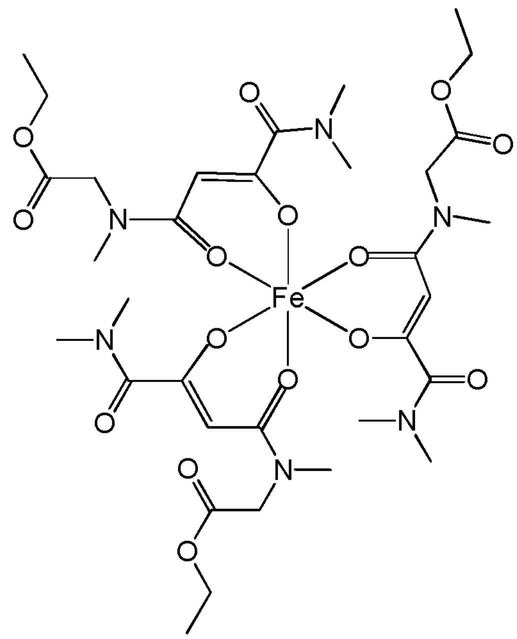

,

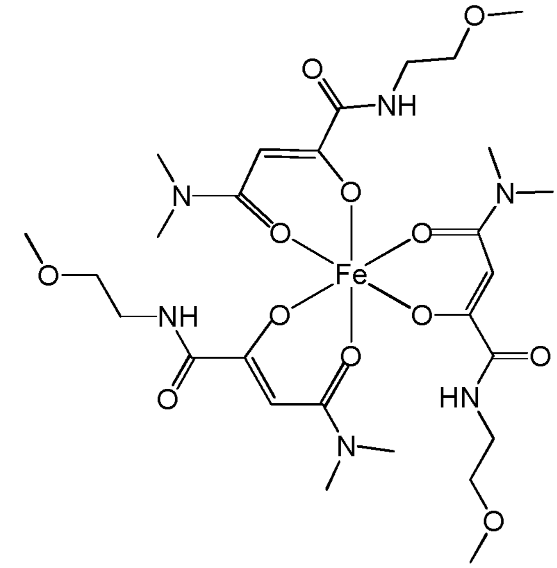

,

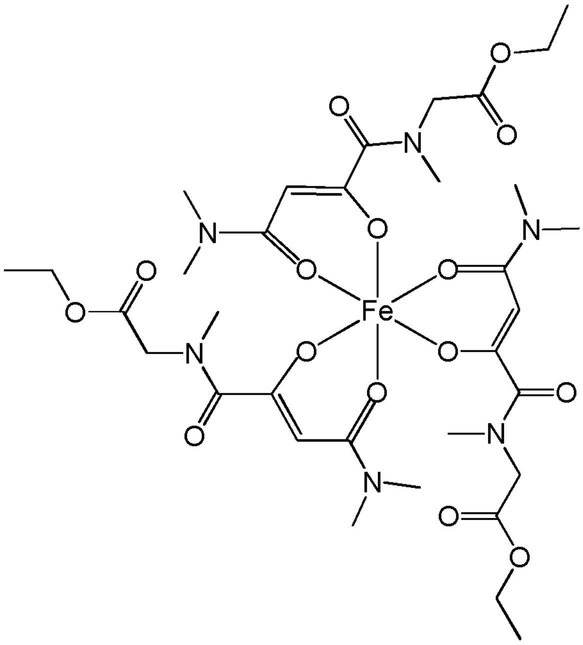

,

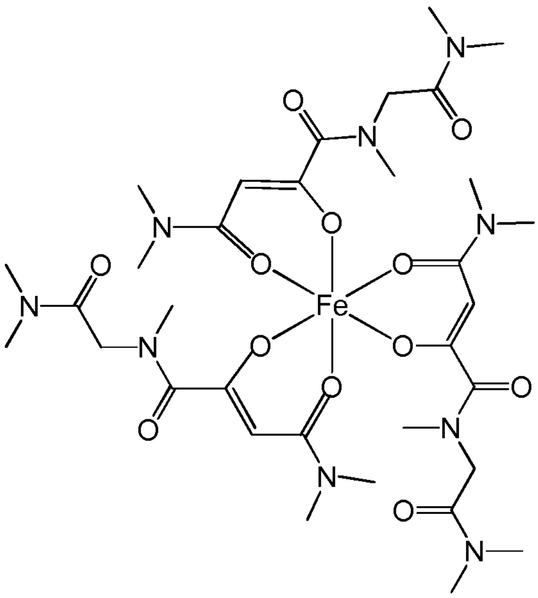

,

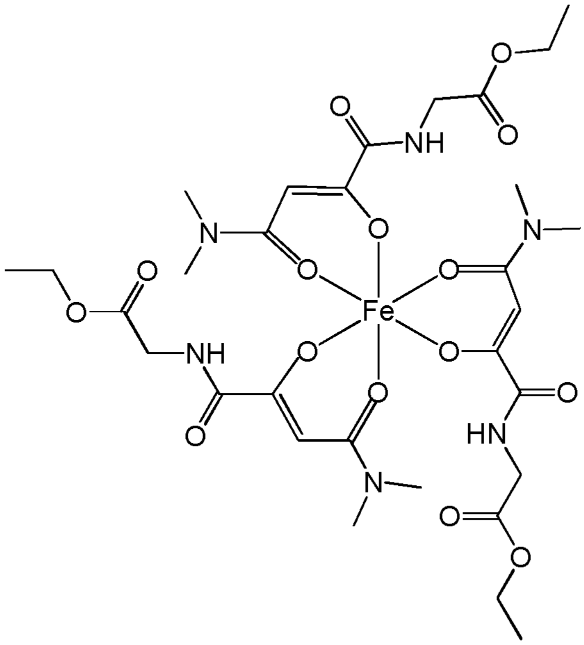

,

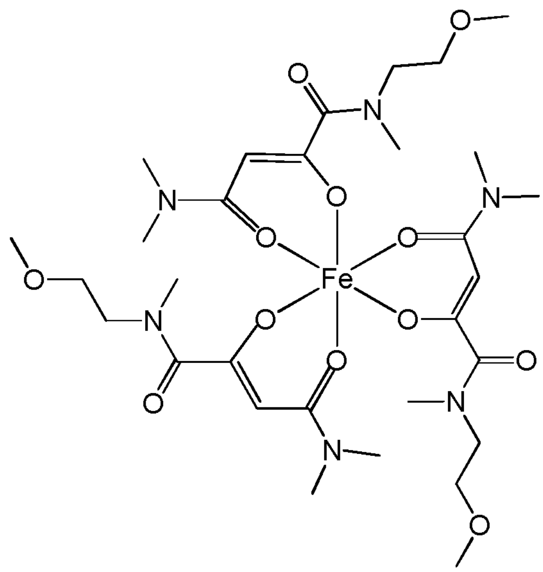

,

and

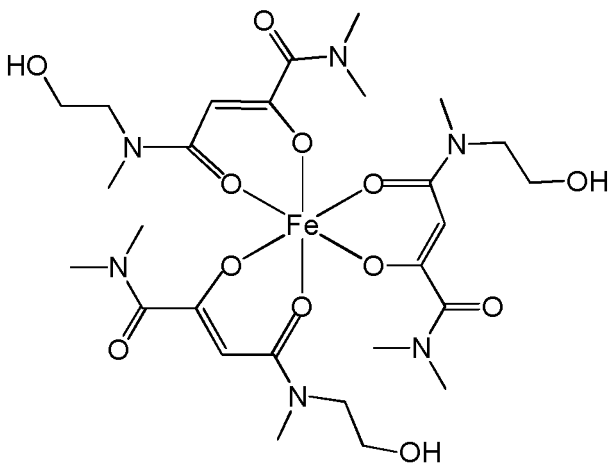

.

**10.** Iron(III) complex compounds or pharmaceutically acceptable salts thereof for the use in the treatment and prophylaxis of iron deficiency diseases and iron deficiency anemias according to one of claims 1 to 9, with a solubility in water at 25°C and at pH 6.5 of at least 0.3 mg/ml based on iron.

**11.** Iron(III) complex compounds for the use in the treatment and prophylaxis of iron deficiency diseases and iron deficiency anemias according to one of claims 1 to 10 for the treatment and prophylaxis of the symptoms of iron deficiency diseases and iron deficiency anemias, wherein the symptoms in particular include: fatigue, listlessness, lack of concentration, low cognitive efficiency, difficulties in finding the right words, forgetfulness, unnatural pallor, irritability, acceleration of heart rate (tachycardia), sore or swollen tongue, enlarged spleen, desire for strange foods (pica), headaches, lack of appetite, increased susceptibility to infections, depressive moods.

**12.** Iron(III) complex compounds for the use in the treatment and prophylaxis of iron deficiency diseases and iron deficiency anemias according to one of claims 1 to 11 for the treatment and prophylaxis of iron deficiency anemia in pregnant women, latent iron deficiency anemia in children and adolescents, iron deficiency anemia caused by gastrointestinal abnormalities, iron deficiency anemia due to blood loss, such as gastrointestinal hemorrhage due to ulcers, carcinoma, hemorrhoids, inflammatory disorders, as well as from taking of acetylsalicylic acid, iron deficiency anemia due to menstruation, iron deficiency anemia due to injuries, iron deficiency anemia due to sprue, iron deficiency anemia due to reduced dietary iron uptake, in particular in selectively eating children and adolescents, immunodeficiency caused by iron deficiency anemia, brain function impairment caused by iron deficiency anemia, restless leg syndrome caused by iron deficiency anemias, iron deficiency anemias in the case of cancer, iron

deficiency anemias caused by chemotherapies, iron deficiency anemias triggered by inflammation (AI), iron deficiency anemias in the case of congestive cardiac insufficiency (CHF; congestive heart failure), iron deficiency anemias in the case of chronic renal insufficiency stage 3-5 (CKD 3-5; chronic kidney diseases stage 3-5), iron deficiency anemias triggered by chronic inflammation (ACD), iron deficiency anemias in the case of rheumatoid arthritis (RA), iron deficiency anemias in the case of systemic lupus erythematosus (SLE), iron deficiency anemias in the case of inflammatory bowel diseases (IBD), iron deficiency with the hemoglobin value being in the normal range.

**13.** Iron(III) complex compounds for the use in the treatment and prophylaxis of iron deficiency diseases and iron deficiency anemias according to one of claims 1 to 12 for oral administration, in particular for administration in the form of a tablet or capsule.

**14.** Medicament for the use in the treatment and prophylaxis of iron deficiency diseases and iron deficiency anemias containing iron(III) complex compounds as defined in one of claims 1 to 10.

**15.** Medicament for the use in the treatment and prophylaxis of iron deficiency diseases and iron deficiency anemias containing iron(III) complex compounds as defined in one of claims 1 to 10 and at least one physiologically acceptable carrier or excipient and/or at least one further medicament which acts on the iron metabolism.

## Revendications

**1.** Des composés complexes de 2-oxobutanediamide de fer (III) ou des sels pharmaceutiquement acceptables de ceux-ci pour l'utilisation dans le traitement et la prophylaxie des symptômes de carence en fer et des anémies ferriprives.

**2.** Des composés complexes de fer (III) pour l'utilisation dans le traitement et la prophylaxie des symptômes de carence en fer et des anémies ferriprives selon la revendication 1, contenant au moins un ligand de la formule (I) :

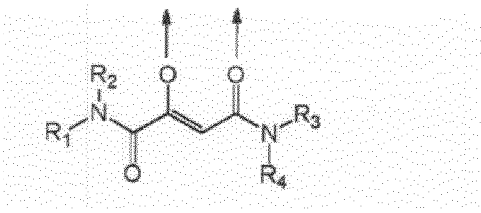

(I)

dans laquelle
les flèches représentent chacune une liaison covalente de coordination à un atome de fer ou à des atomes de fer différents,

$R_1$ et $R_2$ sont identiques ou différents et sont chacun sélectionnés dans le groupe, qui est constitué de l'hydrogène, de l'alkyle éventuellement substitué et du cycloalkyle éventuellement substitué, un ou deux atomes de carbone dans les groupes d'alkyle mentionnés pouvant être éventuellement remplacés par l'oxygène,
ou
$R_1$ et $R_2$ forment un cycle à 3 jusqu'à 6 chaînons éventuellement substitué ensemble avec l'atome d'azote, auquel ils sont liés, lequel cycle peut éventuellement comprendre un autre hétéroatome,
$R_3$ et $R_4$ sont identiques ou différents et sont chacun sélectionnés dans le groupe, qui consiste en hydrogène, de l'alkyle éventuellement substitué et du cycloalkyle éventuellement substitué, un ou deux atomes de carbone dans les groupes d'alkyle mentionnés pouvant être éventuellement remplacés par l'oxygène, ou
$R_3$ et $R_4$ forment un cycle à 3 jusqu'à 6 chaînons éventuellement substitué ensemble avec l'atome d'azote, auquel ils sont liés, lequel cycle peut éventuellement comprendre un autre hétéroatome.

**3.** Des composés complexes de fer (III) pour l'utilisation dans le traitement et la prophylaxie des symptômes de carence en fer et des anémies ferriprives selon la revendication 1 ou la revendication 2, contenant au moins un ligand de la formule (I) :

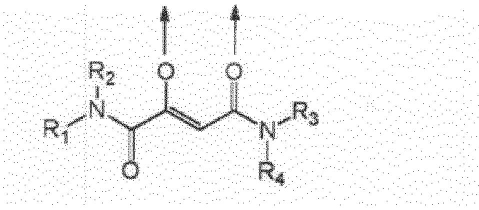

dans laquelle
les flèches représentent chacune une liaison covalente de coordination à un atome de fer ou à des atomes de fer différents,

$R_1$ et $R_2$ sont identiques ou différents et sont chacun sélectionnés dans le groupe, qui est constitué de l'hydrogène, du méthyle, de l'éthyle, du propyle, du i-propyle, du n-butyle, du sec-butyle et du t-butyle, et dans laquelle les groupes alkyle mentionnés peuvent être substitués par un substituant, qui est sélectionné dans le groupe constitué de : alcoxy, alcoxycarbonyle, aminocarbonyle ($H_2NCO$-), monoalkylaminocarbonyle et dialkylaminocarbonyle, ou
$R_1$ et $R_2$ forment un cycle à 5 jusqu'à 6 chaînons éventuellement substitué ensemble avec l'atome d'azote, auquel ils sont liés, lequel cycle peut éventuellement comprendre un autre hétéroatome,
$R_3$ et $R_4$ sont identiques ou différents et sont chacun sélectionnés dans le groupe, qui est constitué de l'hydrogène, de l'alkyle éventuellement substitué et du cycloalkyle éventuellement substitué, un ou deux atomes de carbone dans les groupes d'alkyle mentionnés pouvant être éventuellement remplacés par l'oxygène, ou
$R_3$ et $R_4$ forment un cycle à 3 jusqu'à 6 chaînons éventuellement substitué ensemble avec l'atome d'azote, auquel ils sont liés, lequel cycle peut éventuellement comprendre un autre hétéroatome.

4. Des composés complexes de fer (III) pour l'utilisation dans le traitement et la prophylaxie des symptômes de carence en fer et des anémies ferriprives selon la revendication 1 ou la revendication 2, contenant au moins un ligand de la formule (I) :

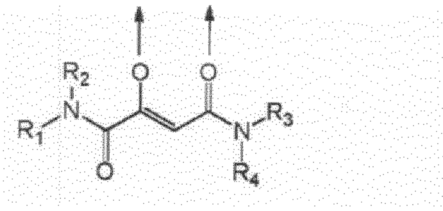

dans laquelle
les flèches représentent chacune une liaison covalente de coordination à un atome de fer ou à des atomes de fer différents,

$R_1$ et $R_2$ sont identiques ou différents et sont chacun sélectionnés dans le groupe, qui est constitué de l'hydrogène, du méthyle, de l'éthyle, du propyle, du i-propyle, du n-butyle, du sec-butyle et du t-butyle, et dans laquelle les groupes alkyle peuvent être substitués par un substituant, qui est sélectionné dans le groupe constitué de l'alcoxy, de l'alcoxycarbonyle, de l'aminocarbonyle ($H_2NCO$-), du monoalkylaminocarbonyle et du dialkylaminocarbonyle, ou
$R_1$ et $R_2$ forment du pyrrolidinyle ensemble avec l'atome d'azote, auquel ils sont liés,
$R_3$ et $R_4$ sont identiques ou différents et sont chacun sélectionnés dans le groupe, qui est constitué de l'hydrogène, de l'alkyle et du cycloalkyle, l'alkyle et le cycloalkyle pouvant être substitués par un substituant, qui est sélectionné dans le groupe constitué de l'hydroxy, de l'alcoxy, de l'alcoxycarbonyle, de l'aminocarbonyle ($H_2NCO$-), du monoalkylaminocarbonyle et du dialkylaminocarbonyle,
$R_3$ et $R_4$ forment du morpholinyle ou du pyrrolidinyle ensemble avec l'atome d'azote, auquel ils sont liés, les groupes cycliques mentionnés pouvant être substitués par l'hydroxy.

5. Des composés complexes de fer (III) pour l'utilisation dans le traitement et la prophylaxie des symptômes de carence en fer et des anémies ferriprives selon la revendication 1 ou la revendication 2, contenant au moins un ligand de la formule (I) :

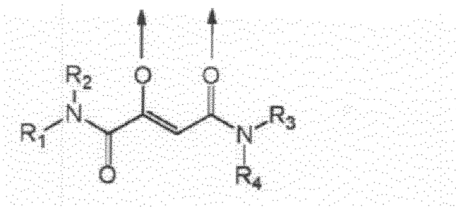

(I)

dans laquelle
les flèches représentent chacune une liaison covalente de coordination à un atome de fer ou à des atomes de fer différents,

$R_1$ et $R_2$ sont identiques ou différents et sont chacun sélectionnés dans le groupe, qui est constitué de l'hydrogène, du méthyle, de l'éthyle, du propyle, du i-propyle, du n-butyle, du sec-butyle et du t-butyle
$R_3$ et $R_4$ sont identiques ou différents et sont chacun sélectionnés dans le groupe, qui est constitué de l'hydrogène, de l'alkyle éventuellement substitué et du cycloalkyle éventuellement substitué, un ou deux atomes de carbone dans les groupes d'alkyle mentionnés pouvant être éventuellement remplacés par l'oxygène, ou
$R_3$ et $R_4$ forment un cycle à 3 jusqu'à 6 chaînons éventuellement substitué ensemble avec l'atome d'azote, auquel ils sont liés, lequel cycle peut éventuellement comprendre un autre hétéroatome.

**6.** Des composés complexes de fer (III) pour l'utilisation dans le traitement et la prophylaxie des symptômes de carence en fer et des anémies ferriprives selon l'une des revendications 1, 2 ou 5 contenant au moins un ligand de la formule (I) :

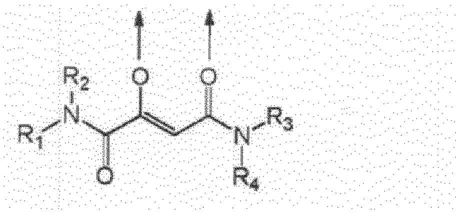

(I)

dans laquelle les flèches représentent chacune une liaison covalente de coordination à un atome de fer ou à des atomes de fer différents,

$R_1$ et $R_2$ sont identiques ou différents et sont chacun sélectionnés dans le groupe, qui est constitué de l'hydrogène et du méthyle,
$R_3$ et $R_4$ sont identiques ou différents et sont chacun sélectionnés dans le groupe, qui est constitué de l'hydrogène, de l'alkyle éventuellement substitué et du cycloalkyle éventuellement substitué, un ou deux atomes de carbone dans les groupes d'alkyle mentionnés pouvant être éventuellement remplacés par l'oxygène.

**7.** Des composés complexes de fer (III) pour l'utilisation dans le traitement et la prophylaxie des symptômes de carence en fer et des anémies ferriprives selon l'une des revendications 1 à 6 de la formule (II) :

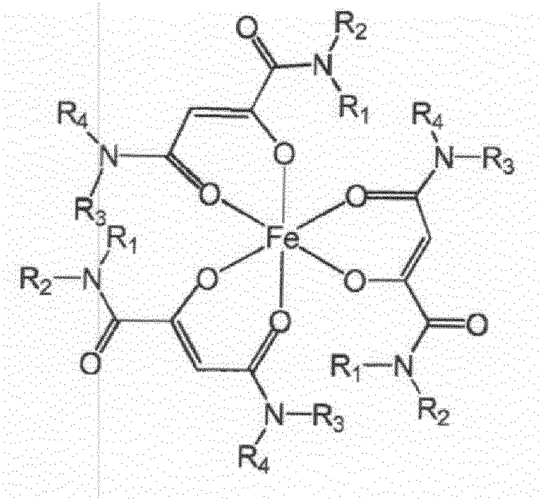

(II)

dans laquelle $R_1$, $R_2$, $R_3$ et $R_4$ sont tels que définis ci-dessus.

**8.** Des composés complexes de fer (III) pour l'utilisation dans le traitement et la prophylaxie des symptômes de carence en fer et des anémies ferriprives selon l'une des revendications 2, 5 à 7, dans lesquels
$R_1$ et $R_2$ sont chacun de l'hydrogène, et
$R_3$ et $R_4$ sont identiques ou différents et sont sélectionnés dans le groupe, qui est constitué du méthyle et de l'éthyle.

**9.** Des composés complexes de fer (III) ou des sels pharmaceutiquement acceptables de ceux-ci pour l'utilisation dans le traitement et la prophylaxie des symptômes de carence en fer et des anémies ferriprives selon l'une des revendications 1 à 8, les composés complexes de fer (III) étant sélectionnés dans le groupe, qui est constitué de :

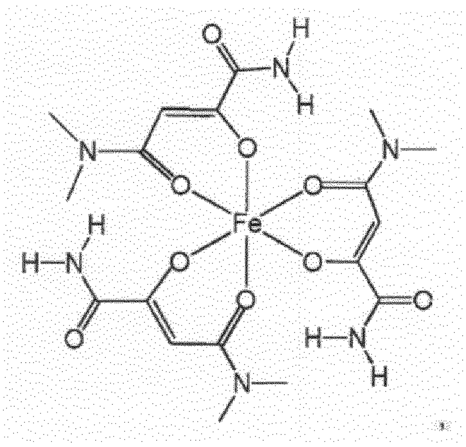

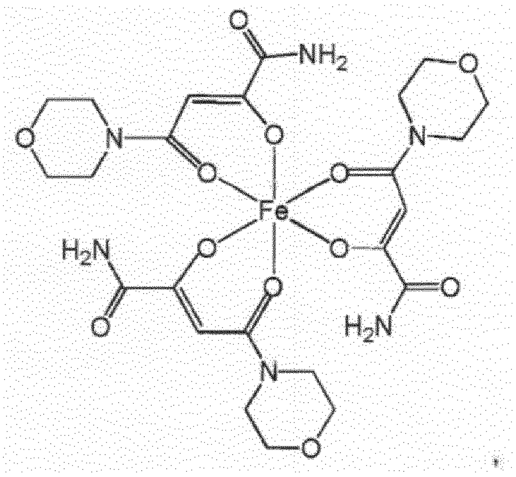
,

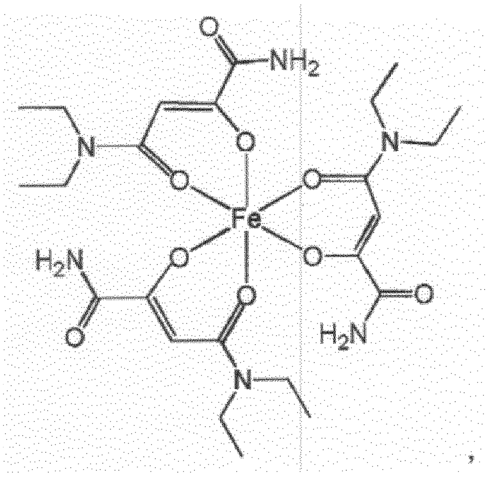
,

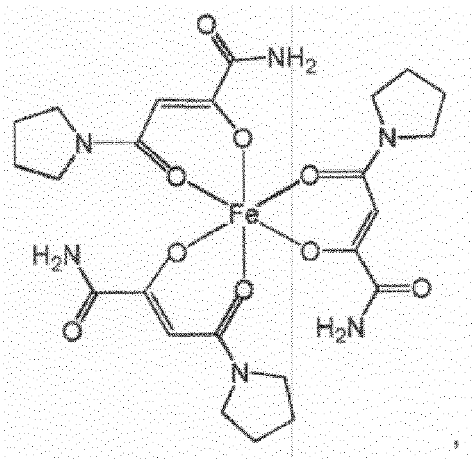
,

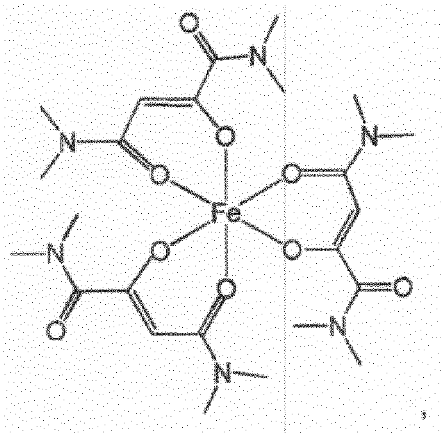

,

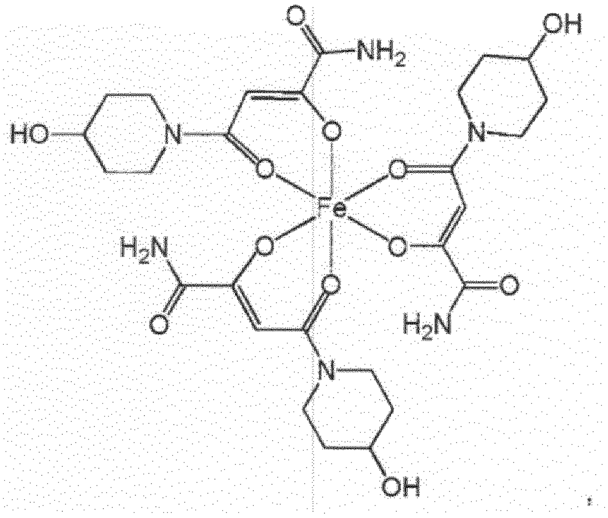

,

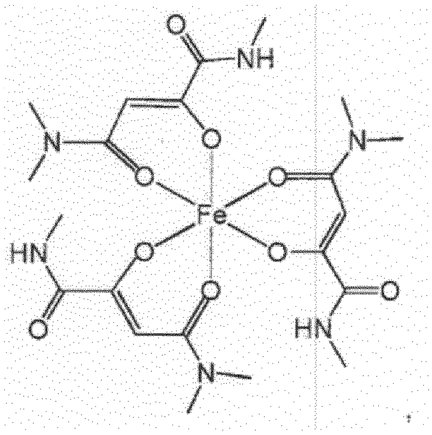

,

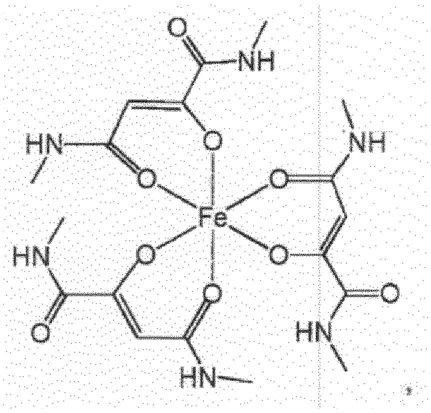

,

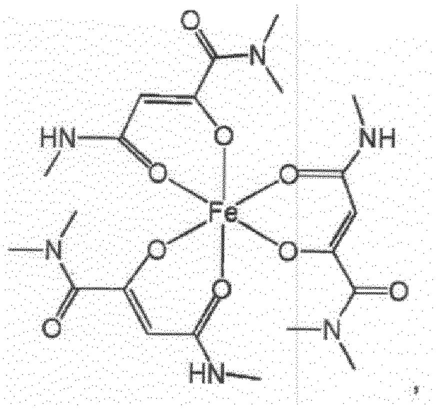

,

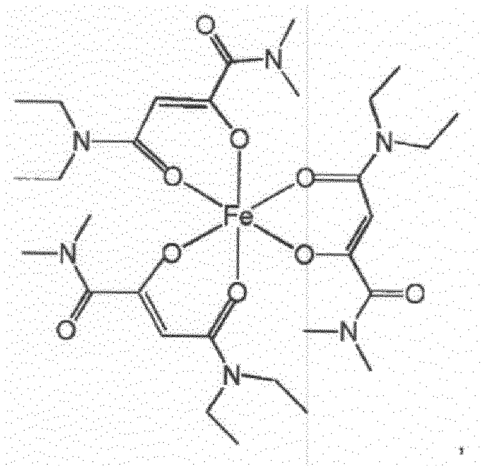

,

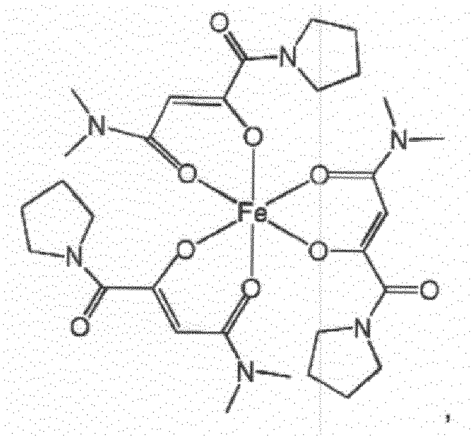

,

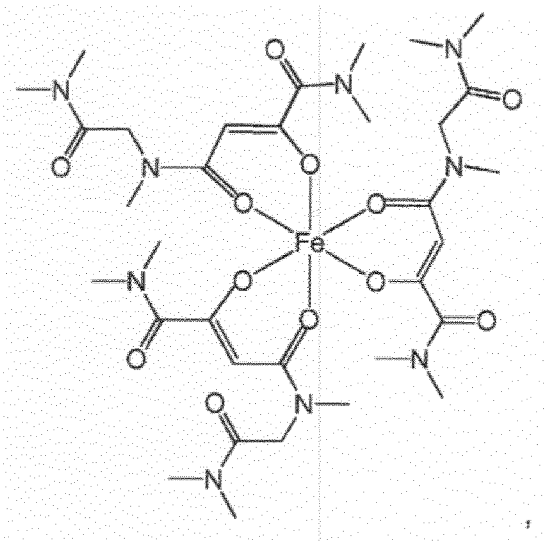

,

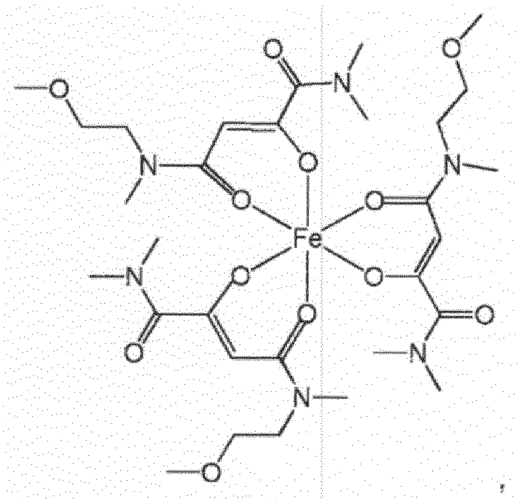

,

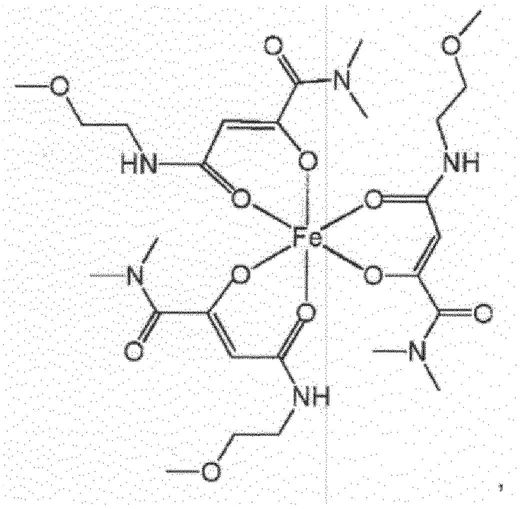

,

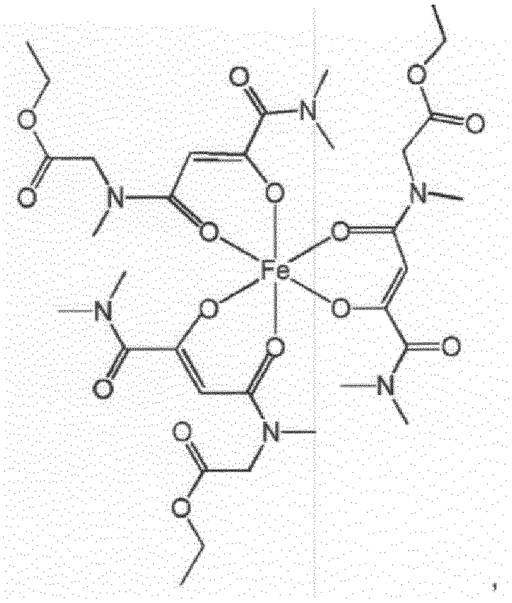

,

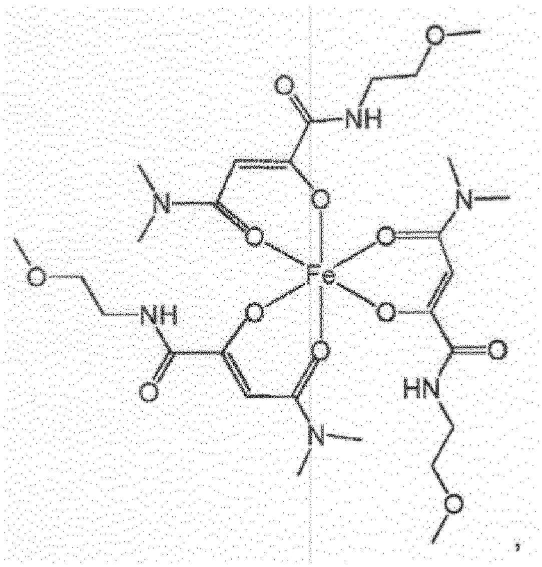

,

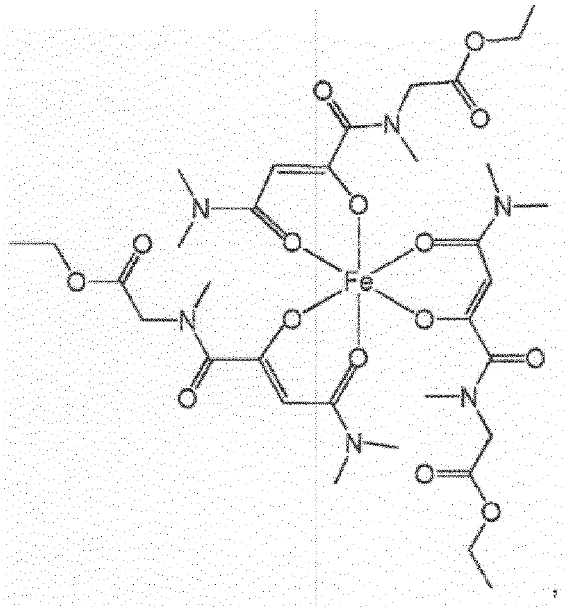

,

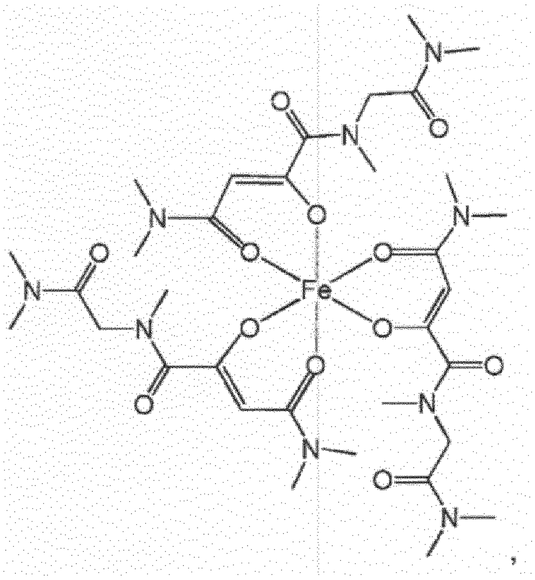

,

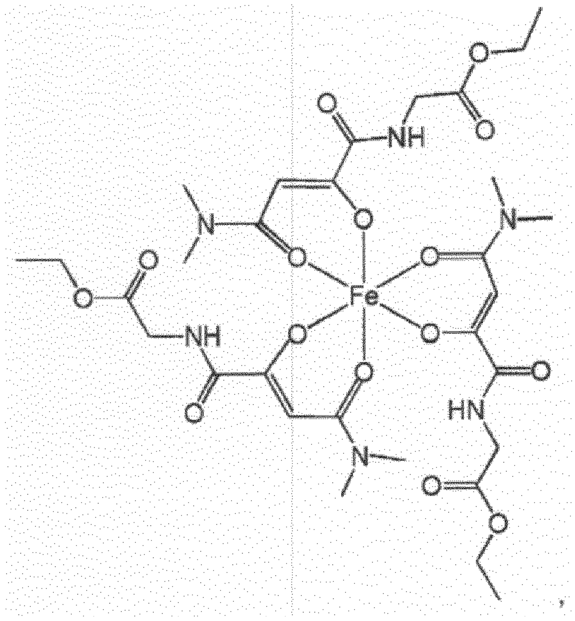

,

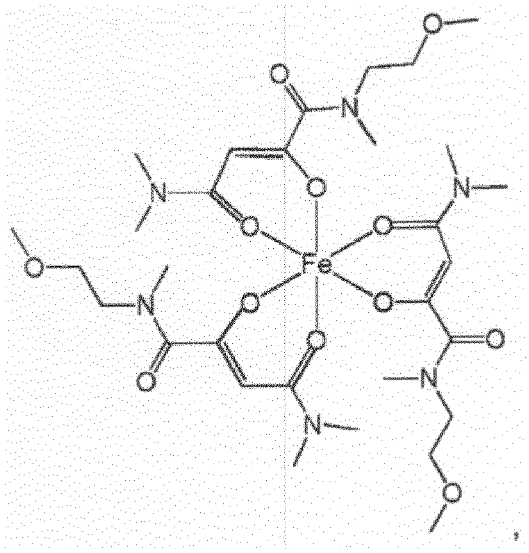

,

und

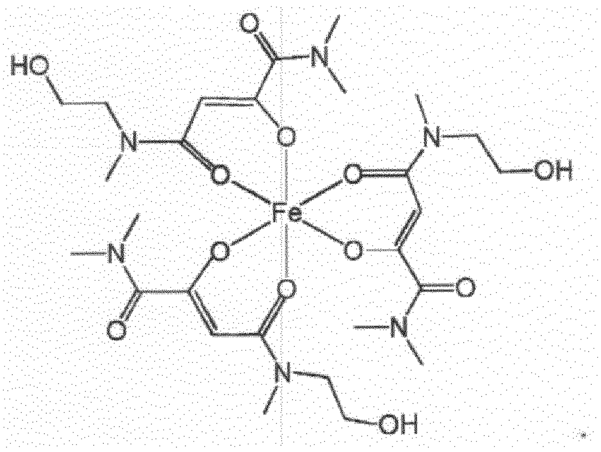

.

**10.** Des composés complexes de fer (III) ou des sels pharmaceutiquement acceptables de ceux-ci pour l'utilisation dans le traitement et la prophylaxie des symptômes de carence en fer et des anémies ferriprives selon l'une des revendications 1 à 9, comprenant une solubilité dans l'eau à 25°C et à pH 6,5 d'au moins 0,3 mg/ml basé sur le fer.

**11.** Des composés complexes de fer (III) pour l'utilisation dans le traitement et la prophylaxie des symptômes de carence en fer et des anémies ferriprives selon l'une des revendications 1 à 10 pour le traitement et la prophylaxie des symptômes de carence en fer et des anémies ferriprives,
les symptômes comprenant notamment : la fatigue, l'apathie, le manque de concentration, l'efficacité cognitive faible, des difficultés de trouver les bons mots, des troubles de mémoire, une pâleur non-naturelle, l'irritabilité, l'augmentation du rythme cardiaque (tachycardie), la langue endolorie ou gonflée, une rate gonflée, des envies de femmes enceintes (pica), des maux de tête, le manque d'appétit, la susceptibilité accrue aux infections, des états dépressifs.

**12.** Des composés complexes de fer (III) pour l'utilisation dans le traitement et la prophylaxie des symptômes de carence en fer et des anémies ferriprives selon l'une des revendications 1 à 11 pour le traitement et la prophylaxie de l'anémie ferriprive chez les femmes enceintes, de l'anémie ferriprive latente chez les enfants et les adolescents, de l'anémie ferriprive causée par des anormalités gastro-intestinales, de l'anémie ferriprive causée par des pertes de sang, telles que des saignements gastro-intestinaux causés par des ulcères, des carcinomes, des hémorroïdes, par des troubles inflammatoires ainsi que par la prise d'acide acétylsalicylique, de l'anémie ferriprive causée par la menstruation, de l'anémie ferriprive causée par des blessures, de l'anémie ferriprive causée par le psilosis (maladie coeliaque), de l'anémie ferriprive causée par un apport réduit en fer dans l'alimentation, notamment chez des enfants et des adolescents, qui mangent sélectivement, de l'immunodéficience causée par l'anémie ferriprive, de la déficience de la capacité cérébrale causée par les anémies ferriprives, du syndrome de jambes sans repos causé par les anémies ferriprives, des anémies ferriprives en cas de cancer, des anémies ferriprives causées par des chimiothérapies, des anémies ferriprives causées par une inflammation (AI), des anémies ferriprives en cas de défaillance cardiaque congestive (CHF, congestive heart failure), des anémies ferriprives en cas d'insuffisance rénale chronique stade 3-5 ; chronic kidney diseases stage 3-5), des anémies ferriprives causées par une inflammation chronique (ACD), des anémies ferriprives en cas d'arthrite rhumatoïde (RA ; rheumatoid arthritis), des anémies ferriprives en cas de Lupus érythémateux disséminé (SLE ; systemic lupus erythematosus), des anémies ferriprives en cas de maladie inflammatoire de l'intestin (IBD ; inflammatory bowel diseases), de l'anémie ferriprive en cas de taux d'hémoglobine dans la plage normale.

**13.** Des composés complexes de fer (III) pour l'utilisation dans le traitement et la prophylaxie des symptômes de carence en fer et des anémies ferriprives selon l'une des revendications 1 à 12 pour l'administration orale, notamment pour l'administration sous forme d'un comprimé ou d'une capsule.

**14.** Produit pharmaceutique pour l'utilisation dans le traitement et la prophylaxie des symptômes de carence en fer et des anémies ferriprives, contenant des composés complexes de fer (III) tels que définis selon l'une des revendications 1 à 10.

**15.** Produit pharmaceutique pour l'utilisation dans le traitement et la prophylaxie des symptômes de carence en fer et des anémies ferriprives, contenant des composés complexes de fer (III) tels que définis selon l'une des revendications 1 à 10 ainsi qu'au moins un porteur ou excipient physiologiquement acceptable et/ou au moins un autre produit pharmaceutique, qui agit sur le métabolisme du fer.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 20081455586 A
- WO 2007062546 A
- WO 20040437865 A
- US 2003236224 A
- EP 150085 A
- CN 101481404
- EP 939083 A
- JP 02083400 A
- US 474670 A
- CN 1687089
- US 2009035385 A
- EP 308362 A
- ES 2044777
- FR 19671016
- US 20050192315 A
- EP 159194 A
- EP 138420 A
- EP 107458 A
- WO 2006037449 A
- WO 2012130882 A
- WO 2012163938 A
- WO 2011117225 A
- WO 11117225 A1


### In der Beschreibung aufgeführte Nicht-Patentliteratur


- **M.W. HENTZE.** Balancing acts: molecular control of mammalian iron metabolism. *Cell,* 2004, vol. 117, 285-297
- *Biometals,* 2009, vol. 22, 701-710
- **R. J. GOBEIL et al.** *Journal of the American Chemical Society,* 1945, vol. 67, 511
- **A. ICHIBA et al.** *Journal of the Scientific Research Institute,* 1948, vol. 23, 23-29
- **J. PERRONNET et al.** *Journal of Heterocyclic Chemistry,* 1980, vol. 17, 727-731
- **W. KANTLEHNER et al.** *Liebigs Annalen der Chemie,* 1980, vol. 9, 1448-1454
- **J. BANVILLE et al.** *Tetrahedron Letters,* 2010, vol. 51, 3170-3173
- **A. ICHIBA et al.** *Journal ofthe Scientific Research,* 1948, vol. 23, 23-29
- **E. FISCHER.** *Chemische Berichte,* 1906, vol. 39, 541